# EUROPEAN PATENT APPLICATION

(11) **EP 3 348 647 A1**
(43) Date of publication of application: **18.07.2018**
(21) Application number: 17305044.4
(22) Date of filing: 13.01.2017
(51) Int. Cl.: C12P 7/64, C12N 1/19

(54) **MUTANT YEAST STRAIN CAPABLE OF PRODUCING MEDIUM CHAIN FATTY ACIDS**

(71) Applicant: INSTITUT NATIONAL DE LA RECHERCHE AGRONOMIQUE, 75007 Paris (FR); INSTITUT NATIONAL DES SCIENCES APPLIQUEES DE TOULOUSE, 31077 Toulouse Cedex 4 (FR); CENTRE NATIONAL DE LA RECHERCHE SCIENTIFIQUE, 75016 Paris (FR)
(72) Inventor: BORDES, Florence, 31200 Toulouse (FR); RIGOUIN, Coraline, 31400 Toulouse (FR); MARTY, Alain, 31000 Toulouse (FR); GUÉROULT, Marc, 51100 Reims (FR); ANDRÉ, Isabelle, 31500 Toulouse (FR); BARBE, Sophie, 31120 Goyrans (FR); PERCHERON, Benjamin, 31300 Toulouse (FR); CROUX, Christian, 31320 Auzeville-Tolosane (FR); DABOUSSI, Fayza, 31750 Escalquens (FR)
(74) Representative: Gevers & Orès

(57) **Abstract**

The present invention relates to mutant yeast strains, in particular mutant *Yarrowia* strains, capable of producing medium chain fatty acids compared to the parent oleaginous yeast strain from which said mutant oleaginous yeast strain derives. This invention also relates to means and methods for obtaining these mutant yeast strains.

## Description

The present invention relates to mutant yeast strains, in particular mutant *Yarrowia* strains, capable of producing medium chain fatty acids compared to wild-type (WT) strain. The present invention also relates to means and methods for obtaining these mutant yeast strains.

Use of alternative sources to oil based kerosene is crucial for the aeronautic industry competitiveness, economic growth and sustainable development. Kerosene is composed of carbon chains that typically contain between 6 and 16 carbon atoms per molecule. Since 2005, aviation industry strongly intensifies researches for sustainable aviation fuel production with very stringent requirements: safety, "drop-in" to allow blends with traditional Jet Fuel, high-performances with international specifications, benefit on full carbon life cycle, no competition with fresh water requirements and food production, no impact on biodiversity. In this regard, lipids from microbial production constitute a very promising route involving oleaginous microorganisms as valuable contributor to the sustainable fuel development.

Fatty acid synthases (FAS) are protein systems that integrate all enzymatic steps of fatty acid synthesis. Two different FAS systems are found in nature. Type I FAS, found in fungi and animals, are giant multifunctional proteins. Animal FAS consist of a homodimer of a single multifunctional polypeptide chain. Fungal FAS is a dodecamer made of 2 different polypeptide chains (six alpha subunits and six beta subunits). Type II FAS found in prokaryotes and plants are made of 9 individual proteins each encoded by distinct genes. However, the underlying enzymatic reactions are conserved between these two systems (Leibundgut *et al*., 2008).

Fatty acid synthesis is initiated when an acetyl moiety from acetyl coenzyme A (CoA) is transferred to the thiol group of the phosphopantetheine arm of the acyl carrier protein (ACP) by the acetyl transferase (AT) and shuttled to the catalytic site of ketoacyl synthase (KS). Subsequently, after the transfer of a malonyl moiety from malonyl-CoA to ACP by malonyl transferase (MT), the KS catalyzes decarboxylative condensation of the ACP-attached malonyl portion with the acetyl starter group. The β-ketoacyl-ACP product is then modified at its β-carbon position by a sequence of three reactions. First, the ketoreductase (KR) reduces it to a β-hydroxyl intermediate; second, a dehydratase (DH) releases a water molecule yielding a β-enoyl moiety, which, in the third step, is reduced by the enoyl reductase (ER) to yield a saturated acyl chain elongated by a two-carbon unit. This acyl product then serves as primer substrate for condensation by the KS with another malonyl-ACP in the next round of elongation. Each cycle results in a 2 carbon elongation of the acyl chain. The reaction cycle is repeated until a chain length of C16 or C18 is reached and the end products palmitate or stearate are released. Fungal FAS utilizes a mono-functional acetyl transferase (AT) to transfer the acetyl-starter to ACP and a bi-functional malonyl/palmitoyl transferase (MPT), which charges ACP with malonyl groups and back-transfers the products to coenzyme A for release as CoA-esters.

Some organisms naturally produce short to medium chain length fatty acids. Octanoic (8:0), decanoic (10:0) and dodecanoic (12:0) fatty acids are found in esterified forms in most milk fats, including those of non-ruminants. They are also major components of some particular seed oils such as coconut oil, palm kernel oil and in *Cuphea* species.

Depending on the FAS system harbored by the organism, the substrate chain length determination does not seem to be driven by the same mechanism. In FASII system, in which the elongation cycle terminates with the hydrolysis of the acyl-ACP, the thioesterase is the enzyme involved in chain length specificity (Jing *et al*., 2011; Schütt *et al*., 1998). In fungal FASI system, thioesterase are not present and the elongation cycle terminates with the transfer of the acyl-ACP to a coenzymeA. The MPT enzyme responsible of this activity, does not seem to be the molecular ruler to determine chain length (Leibundgut *et al*., 2008).

Among the lipid-producer microorganisms, *Yarrowia lipolytica* is the most studied, with an extensive toolbox for metabolic engineering. *Y. lipolytica* is an oleaginous yeast, capable of producing and accumulating large amount of lipids, accounting to more than 50% of its dry weight (Beopoulos *et al*., 2009). *Y. lipolytica,* but more generally *Yarrowia* species, naturally produce lipids with Long Chain Fatty Acid containing 16 and 18 carbons. *Y. lipolytica* has been shown to be suitable for large-scale fermentations (Ledesma-Amaro and Nicaud, 2016). Because of these capacities, and being recognized as a GRAS organism (*Generally Regarded As Safe*), this yeast is a promising organism for the development of various applications including production of recombinant proteins, metabolites and oils with high added value (Madzak, 2015). In regards to its properties to synthesize and accumulate lipids, *Y*. *lipolytica* is an organism of choice for biofuel production as an alternative of vegetable oil extracted from plants or fossil fuels.

The inventors have developed, by genome and enzyme engineering, strains of *Yarrowia lipolytica* capable of producing Medium Chain Fatty Acids (MCFA), which can be useful for their downstream use as biokerosene. Genetically modified strains of *Y. lipolytica* wherein the endogenous αFAS gene was deleted and, in which mutant I1220F or I1220M αFAS was expressed, were capable of modulating Fatty Acid (FA) profile towards Medium Chain Fatty Acids (MCFA), in particular of synthesizing the medium chain length comprising 12 carbons such as lauric acid, also known as dodecanoic acid or 14 carbons such as myristic acid, also known as tetradecanoic acid.

In the present invention:
- Short Chain Fatty Acids relate to Fatty Acids with a hydroxycarbon chain length less than 8 carbons,
- Medium Chain Fatty Acids relate to Fatty Acids with a hydroxycarbon chain length comprised between 8 and 15 carbons,
- Long Chain Fatty Acids relate to Fatty Acids with a hydroxycarbon chain length of and beyond 16 carbons.

Yeast cytosolic FAS (EC 2.3.1.86) catalyzes the synthesis of fatty acid from acetyl-CoA and malonyl-CoA. It is composed of two subunits, Fas1 (βFAS) and Fas2 (αFAS) which are organized as a hexameric α6β6 complex. βFAS carries acetyl transferase, enoyl reductase, dehydratase, malonyl-palmitoyl transferase activities and αFAS carries acyl-carrier protein, 3-ketoreductase, 3-ketosynthase and the phosphopantheteine transferase activities. The spectrum of fatty acid in yeast consists mostly of C16 and C18 fatty acids.

Methods for determining whether an enzyme is a fatty acid synthase (FAS) are known in the art. By way of example, one can use the method described in Stoops *et al.* 1978.

In the present invention, the amino acid numbering of a yeast αFAS protein is made with reference to the *Yarrowia lipolytica* αFAS protein available in the GenBank database under the accession number YALI0_B19382g referred to as SEQ ID NO: 1 (YALI_αFAS). The numbering of amino acid residues of a known αFAS protein can be made by aligning the amino acid sequence of said known αFAS protein with the amino acid sequence of SEQ ID NO: 1 (YALI_αFAS). Alignment of two amino acid sequences can be performed using the programs MUSCLE (Edgar, 2004).

The amino acid residues at positions 1220 and 1305 of *Yarrowia lipolytica* αFAS protein (SEQ ID NO: 1) are respectively isoleucine (I) and serine (S).

The αFAS proteins of yeasts and the amino acid residues at positions corresponding respectively to the positions 1220 and 1305 of *Yarrowia lipolytica* αFAS are mentioned in the Table 1 below:

**Table 1.**

| Yeast | GenBank accession number | SEQ ID NO: 1 | % sequence identity | Amino acid residue corresponding to the amino acid residue at position 1220 in αFAS of *Yarrowi a lipolytica* | Amino acid residue corresponding to the amino acid residue at position 1305 in αFAS of *Yarrowia lipolytica* |
|---|---|---|---|---|---|
| *Yarrowia lipolytica* * | YALI0_B19382g | 1 | 100 | I | s |
| *Rhodotorula sp. JG-1b.* | KWU43709.1 | 2 | 52 | I | G |
| *Rhodotorula toruloides** | XP_016272387.1 | 3 | 51 | I | G |
| *Candida albicans* | KHC55685.1 | 4 | 65 | V | G |
| *Candida tropicalis** | XP_002548204.1 | 5 | 65 | V | G |
| *Trichosporon oleaginosus** | KLT39273.1 | 6 | 52 | M | G |
| *Cryptococcus neoformans* | XP_012049943.1 | 7 | 52 | M | G |
| *Cryptococcus gattii* | KIR51369.1 | 8 | 51 | M | G |
| *Saccharomyces cerevisiae* | AJW19346.1 | 9 | 63 | V | G |

| | | | | | |
|---|---|---|---|---|---|
| *Oleaginous yeast | | | | | |

Accordingly, the present invention provides a method for increasing the ratio of fatty acids having a hydroxycarbon chain length consisting of 16 carbons (C16 fatty acids) to fatty acids having a hydroxycarbon chain consisting of 18 carbons (C18 fatty acids) and/or for increasing the amount of medium chain length fatty acids (C8-C15 fatty acids), produced by a yeast strain, preferably an oleaginous yeast strain, more preferably a *Yarrowia strain*, more preferably a *Yarrowia lipolytica* strain, compared to the parent yeast strain from which said yeast strain derives, comprising expressing in said yeast strain a mutated fatty acid synthase subunit alpha (αFAS), wherein the amino acid residue of said αFAS corresponding to the amino acid residue at position 1220 in SEQ ID NO: 1 is substituted with a larger steric hindrance amino acid residue, and, optionally, wherein the amino acid residue of said αFAS corresponding to the amino acid residue at position 1305 in SEQ ID NO: 1 is substituted with any other amino acid, more preferably threonine (T).

Accordingly, an amino acid residue selected from the group consisting of phenylalanine (F), histidine (H), methionine (M), tryptophan (W) and tyrosine (Y) has a higher steric hindrance than the isoleucine (I), when the non-mutated αFAS is from *Yarrowia lipolytica.*

According to a particular embodiment of the invention, mutated αFAS is as follows:
- when the amino acid residue corresponding to the amino acid residue at position 1220 in SEQ ID NO: 1 of the non-mutated αFAS is isoleucine (I) then it is substituted with an amino acid residue selected from the group consisting of phenylalanine (F), histidine (H), methionine (M), tryptophan (W) and tyrosine (Y), preferably selected from the group consisting of phenylalanine (F) and tryptophan (W),
- when the amino acid residue corresponding to the amino acid residue at position 1220 in SEQ ID NO: 1 of the non-mutated αFAS is valine (V) then it is substituted with an amino acid residue selected from the group consisting of isoleucine (I), phenylalanine (F), histidine (H), methionine (M), tryptophan (W) and tyrosine (Y), preferably selected from the group consisting of phenylalanine (F) and tryptophan (W),
- when the amino acid residue corresponding to the amino acid residue at position 1220 in SEQ ID NO: 1 of the non-mutated αFAS is methionine (M) then it is substituted with an amino acid residue selected from the group consisting of phenylalanine (F), histidine (H), tryptophan (W) and tyrosine (Y), preferably selected from the group consisting of phenylalanine (F) and tryptophan (W).

According to another particular embodiment of the invention, the mutated αFAS is derived from a wild-type αFAS from a yeast, preferably from an oleaginous yeast, more preferably from a yeast belonging to the genus selected from the group consisting of *Candida, Cryptococcus, Lipomyces, Rhodosporidium, Rhodotorula, Trichosporon, Saccharomyces* and *Yarrowia,* most preferably from *Yarrowia,* in particular from *Yarrowia lipolytica.*

According to a more particular embodiment of the invention, the oleaginous yeast strain is selected from the group consisting of a *Y. lipolytica, Y. galli, Y. yakushimensis, Y. alimentaria* and *Y. phangngensis* strain, preferably a *Y. lipolytica* strain.

The amino acid sequence of the non-mutated αFAS has at least 50% identity, or by order of increasing preference at least 51%, 55%, 60%, 65%, 70%, 75%, 82%, 85%, 90%, 92%, 95%, 96%, 97%, 98%, 99% or 100% identity, with the amino acid sequence of *Yarrowia lipolytica* αFAS of SEQ ID NO: 1 (*Y*. *lipolytica* αFAS YALI0_B19382p).

Unless otherwise specified, the percent of identity between two protein sequences which are mentioned herein is calculated from the BLAST results performed either at the NCBI (http://blast.ncbi.nlm.nih.gov/Blast.cgi) or at the GRYC (http://gryc.inra.fr/) websites using the BlastP program with the default BLOSUM62 parameters as described in Altschul *et al.* (1997).

Advantageously, the non-mutated αFAS having at least 50% identity with the polypeptide of sequence SEQ ID NO: 1 (*Y. lipolytica* αFAS YALI0_B19382p) is derived from a wild-type αFAS from a yeast, preferably from an oleaginous yeast, more preferably from a yeast belonging to the genus selected from the group consisting of *Yarrowia, Rhodotorula, Candida, Trichosporon, Cryptococcus* and *Saccharomyces,* most preferably from *Yarrowia*, in particular from *Yarrowia lipolytica.*

The nucleotide sequence corresponding to the *Yarrowia lipolytica* αFAS protein is the sequence SEQ ID NO: 10.

In a preferred embodiment, the mutated αFAS is derived from the consensus amino acid sequence SEQ ID NO: 11, wherein the amino acid residue at position 1230 in SEQ ID NO: 11 and corresponding to the amino acid residue at position 1220 in SEQ ID NO: 1 is substituted with a larger steric hindrance amino acid residue, and, optionally, wherein the amino acid residue at position 1315 in SEQ ID NO: 11 and corresponding to the amino acid residue at position 1305 in SEQ ID NO: 1 is substituted with any other amino acid.

More preferably, the mutated αFAS is derived from αFAS selected from the group consisting of SEQ ID NO: 1, 2, 3, 4, 5, 6, 7, 8 and 9.
In a particular embodiment of the invention, the mutated αFAS is a mutated autologous αFAS.

An autologous αFAS is defined as a αFAS from said yeast strain in which the mutated αFAS is expressed.

In a preferred embodiment of the invention, the method of the invention comprises the expression in a yeast strain defined above, a mutated fatty acid synthase subunit alpha (αFAS), wherein the amino acid residue of said αFAS corresponding to the amino acid residue at position 1220 in SEQ ID NO: 1 is substituted with a larger steric hindrance amino acid residue, and wherein the amino acid residue of said αFAS corresponding to the amino acid residue at position 1305 in SEQ ID NO: 1 is substituted with threonine (T).

In a preferred embodiment of the invention, the ratio of fatty acids having a hydroxycarbon chain length consisting of 12 or 14 carbons (C12 or C14 fatty acids), preferably C14 fatty acids, to fatty acids having a hydroxycarbon chain consisting of 18 carbons (C18 fatty acids) is increased and/or the amount of Medium chain fatty acids having a hydroxycarbon chain length consisting of 12 carbons or 14 carbons (C12 or C14 fatty acids) is increased.

In a more preferred embodiment of the invention, the fatty acid having a hydroxycarbon chain length consisting of 14 carbons is myristic acid (tetradecanoic acid) and the fatty acid having a hydroxycarbon chain length consisting of 12 carbons is lauric acid (Dodecanoic acid).

Advantageously, the substitution of the amino acid residues corresponding to the amino acid residues at positions 1220 and/or 1305 of said αFAS is obtained by site-directed mutagenesis of the αFAS gene targeting the codon encoding the amino acid residues corresponding to the amino acid residues at said positions 1220 and/or 1305.

Expression of a mutated fatty acid synthase subunit alpha (αFAS) as defined above in a yeast strain, in particular in a *Yarrowia* strain in which αFAS gene is inhibited, according to the present invention can be obtained in various ways by methods known *per se*.

Expression of said mutated fatty acid synthase subunit alpha (αFAS) can be performed by placing one or more (preferably two or three) copies of the coding sequence (CDS) of the sequence encoding said mutated αFAS under the control of appropriate regulatory sequences. Said regulatory sequences include promoter sequences, located upstream (at 5' position) of the ORF of the sequence encoding said mutated αFAS, and terminator sequences, located downstream (at 3' position) of the ORF of the sequence encoding said enzyme.

Promoter sequences that can be used in yeast are well known to those skilled in the art and may correspond in particular to inducible or constitutive promoters. Examples of promoters which can be used according to the present invention, include the promoter of a *Y*. *lipolytica* gene which is strongly repressed by glucose and is inducible by the fatty acids or triglycerides such as the promoter of the *POX2* gene encoding the acyl-CoA oxidase 2 (AOX2) of *Y. lipolytica* and the promoter of the *LIP2* gene described in International Application WO 01/83773. One can also use the promoter of the *FBA1* gene encoding the fructose-bisphosphate aldolase (see Application US 2005/0130280), the promoter of the *GPM* gene encoding the phosphoglycerate mutase (see International Application WO 2006/0019297), the promoter of the *YAT1* gene encoding the transporter ammonium (see Application US 2006/0094102), the promoter of the *GPAT* gene encoding the O-acyltransferase glycerol-3-phosphate (see Application US 2006/0057690), the promoter of the *TEF gene* (Müller *et al*., 1998; Application US 2001/6265185), the hybrid promoter hp4d (described in International Application WO 96/41889), the hybrid promoter XPR2 described in Mazdak *et al.* (2000) or the hybrid promoters UAS1-TEF or UAStef-TEF described in Blazeck *et al.* (2011, 2013, 2014).

Advantageously, the promoter is the promoter of the *TEF* gene.

Terminator sequences that can be used in yeast are also well known to those skilled in the art. Examples of terminator sequences which can be used according to the present invention include the terminator sequence of the *PGK1* gene and the terminator sequence of the *LIP2* gene described in International Application WO 01/83773.

Said copies of the gene encoding said mutated αFAS under the control of regulatory sequences such as those described above may be carried by an episomal vector, that is to say capable of replicating in the yeast strain or may be introduced in the yeast genome as a linear expression cassette by homologous or non homologous recombination.

For the introduction by homologous recombination, the sequence of the (wild-type) αFAS is replaced by the sequence of the mutated αFAS as those described above. The skilled person can replace the copy of the gene encoding the αFAS in the genome as well as its own regulatory sequences, by genetically transforming the yeast strain with a linear polynucleotide comprising the ORF of the sequence coding for said mutated αFAS, optionally under the control of regulatory sequences such as those described above. Advantageously, said polynucleotide is flanked by sequences which are homologous to sequences located on each side of said chromosomal gene encoding said αFAS. Selection markers can be inserted between the sequences ensuring recombination to allow, after transformation, to isolate the cells in which integration of the fragment occurred by identifying the corresponding markers. Advantageously also, the promoter and terminator sequences belong to a gene different from the gene encoding the αFAS to be expressed in order to minimize the risk of unwanted recombination into the genome of the yeast strain.

Expression of said mutated fatty acid synthase subunit alpha (αFAS) can also be obtained by introducing into the yeast strain copies of the gene encoding said mutated αFAS under the control of regulatory sequences such as those described above. Said copies encoding said mutated αFAS may be carried by an episomal vector, that is to say capable of replicating in the yeast strain.

For the introduction by homologous recombination, other locus of the yeast genome, *e.g*., *Yarrowia* genome could be targeted (Madzak *et al*., 2004). In this case, the polynucleotide comprising the gene encoding said mutated αFAS under the control of regulatory regions is integrated by targeted integration. Said additional copies can also be carried by PCR fragments whose ends are homologous to a given locus of the yeast strain, allowing integrating said copies into the yeast genome by homologous recombination. Said additional copies can also be carried by auto-cloning vectors or PCR fragments, wherein the ends have a zeta region absent from the genome of the yeast, allowing the integration of said copies into the yeast genome, *e.g*., *Yarrowia* genome, by random insertion as described in Application US 2012/0034652.

Targeted integration of a gene into the genome of a yeast cell is a molecular biology technique well known to those skilled in the art: a DNA fragment is cloned into an integrating vector, introduced into the cell to be transformed, wherein said DNA fragment integrates by homologous recombination in a targeted region of the recipient genome (*Orr*-Weaver *et al*., 1981).

An advantageous method according to the present invention consists in genetically transforming said yeast strain with a cassette of said endogenous αFAS gene. A suitable integration cassette for expression of αFAS gene contains specific sequences for random genomic transformation, and a selection marker.

The integration cassette can carry the αFAS gene wild type of mutated. The invention shows that random integration of the wild type copy of the αFAS gene in the ΔαFAS genome restore the growth of the strain without fatty acid complementation, showing that the αFAS gene, randomly incorporated in the genome and under the control of the constitutive pTEF promoter is correctly expressed and processed up to the formation of the active FAS complex.

According to an advantageous embodiment of the invention, the method further comprises inhibiting in said yeast strain, in particular said oleaginous strain, the expression and/or the activity of the endogenous elongase proteins, in particular elongase 1 (ELO1; EC 2.3.1.199) having at least 50% identity or by order of increasing preference at least 55%, 60%, 65%, 70%, 75%, 80%, 85%, 90%, 92%, 95%, 96%, 97%, 98% or 99% identity, with the polypeptide of sequence SEQ ID NO: 12 (YALI_ELO1) and/or of the endogenous elongase 2 (ELO2; EC 2.3.1.199) having at least 50% identity or by order of increasing preference at least 55%, 60%, 65%, 70%, 75%, 80%, 85%, 90%, 92%, 95%, 96%, 97%, 98% or 99% identity, with the polypeptide of sequence SEQ ID NO: 13 (YALI_ELO2).

The elongase 1 from the strain *Y. lipolytica* (YALI_ELO1) of SEQ ID NO: 14 is encoded in *Y. lipolytica* by the gene YALI0B20196p.

The elongase 2 from the strain *Y. lipolytica* (YALI_ELO2) of SEQ ID NO: 15 is encoded in *Y. lipolytica* by the gene YALI0F06754p.

According to an advantageous embodiment of the invention, the method further comprises inhibiting in said yeast strain the expression and/or the activity of the endogenous fatty acid synthase subunit alpha (EC 2.3.1.86).

The inhibition of the expression and/or activity of the endogenous αFAS and/or elongase proteins, in particular elongase 1 (ELO1) and/or elongase 2 (ELO2) can be total or partial and involve the entire protein or a particular domain of the protein. For example, the inhibition of the expression and/or activity of the endogenous αFAS can be partial and concerned the ketoacyl synthase (KS) domain of the αFAS. The ketoacyl synthase (KS) (EC 3.2.1.41) belongs to the subunit alpha of the αFAS. It is a beta-ketoacyl-[acyl-carrier-protein] synthase and catalyses the reaction acyl-[acyl-carrier protein] + malonyl-[acyl-carrier protein] -> 3-oxoacyl-[acyl-carrier protein] + CO2 + [acyl-carrier protein].

The inhibition of the expression and/or activity of the endogenous αFAS and/or elongase proteins, in particular elongase 1 (ELO1) or elongase 2 (ELO2) may be obtained in various ways by methods known in themselves to those skilled in the art. The term inhibiting the expression of an endogenous αFAS and/or elongase proteins in a yeast strain refers to decreasing the quantity of said enzyme produced in a yeast strain compared to a reference (control) yeast strain wherein the expression of said endogenous αFAS and/or elongase proteins is not inhibited and from which the mutant strain derives. The term inhibiting the activity of an endogenous αFAS and/or elongase proteins in a yeast strain refers to decreasing the enzymatic activity of said enzyme compared to a reference (control) yeast strain wherein the activity of said endogenous αFAS and/or elongase proteins is not inhibited and from which the mutant strain derives.

This inhibition may be obtained by mutagenesis of the endogenous gene encoding said αFAS (*αFAS* gene), partially or totally or of the endogenous gene encoding said elongase proteins using recombinant DNA technology or random mutagenesis. This may be obtained by various techniques, performed at the level of DNA, mRNA or protein, to inhibit the expression and/or the activity of the αFAS and/or elongase proteins.

The mutagenesis of the endogenous *αFAS* gene can also be carried out using physical agents (for example radiation) or chemical agents. This mutagenesis also makes it possible to introduce one or more point mutations into the *αFAS* gene.

At the level of DNA, mRNA, this inhibition, which may be resulted in a modification of the activity or in the specificity of the protein, may be accomplished by deletion, insertion and/or substitution of one or more nucleotides, site-specific mutagenesis, random mutagenesis, targeting induced local lesions in genomes (TILLING), knock-out techniques, Molecular scissors (Nucleases) (TALEN, CRISPR/ Cas9...) or gene silencing using, e.g., RNA interference, antisense, aptamers, and the like.

This inhibition may also be obtained by insertion of a foreign sequence in the *αFAS* gene and/or in the elongase genes, e.g., through transposon mutagenesis using mobile genetic elements called transposons, which may be of natural or artificial origin.

The mutagenesis of the endogenous gene encoding said αFAS and/or elongase proteins can be performed at the level of the coding sequence or of the sequences for regulating the expression of this gene, in particular at the level of the promoter, resulting in an inhibition of transcription or of translation of said αFAS and/or elongase proteins.

This inhibition may also be obtained by the use of specific inhibitors to decrease enzymatic activity.

The mutagenesis of the endogenous *αFAS* gene and/or elongase genes can be carried out by genetic engineering. It is, for example, possible to delete all or part of said gene or domain and/or to insert an exogenous sequence. Methods for deleting or inserting a given genetic sequence in yeast, in particular in *Y. lipolytica*, are well known to those skilled in the art (for review, see Barth and Gaillardin, 1996; Madzak *et al*., 2004). By way of example, one can use the method referred to as POP IN/POP OUT which has been used in yeasts, in particular in *Y. lipolytica*, for deleting the *LEU2* and *XPR2* genes (Barth and Gaillardin, 1996). One can also use the SEP method (Maftahi *et al.,* 1996) which has been adapted in *Y. lipolytica* for deleting the *POX* genes (Wang *et al*., 1999). One can also use the SEP/Cre method developed by Fickers *et al.* (2003) and described in International application WO 2006/064131. In addition, methods for inhibiting the expression and/or the activity of an enzyme in yeasts are described in International application WO 2012/001144.

An advantageous method according to the present invention consists in replacing the coding sequence of the endogenous *αFAS* gene partially or totally and/or elongase genes with an expression cassette containing the sequence of a gene encoding a selectable marker, as described in Fickers *et al.* (2003). It is also possible to introduce one or more point mutations into the endogenous *αFAS* gene and/or elongase genes, resulting in a shift in the reading frame, and/or to introduce a stop codon into the sequence and/or to inhibit the transcription or the translation of the endogenous *αFAS* gene and/or elongase genes.

Another advantageous method according to the present invention consists in genetically transforming said yeast strain with a disruption cassette of said endogenous *αFAS* gene and of the endogenous gene encoding elongase 1 and/or of the endogenous gene encoding elongase 2. A suitable disruption cassette for disrupting the endogenous *αFAS* gene contains specific sequences for homologous recombination and site-directed insertion, and a selection marker.

The mutagenesis of the endogenous *αFAS* gene and/or elongase genes can also be carried out using physical agents (for example radiation) or chemical agents. This mutagenesis also makes it possible to introduce one or more point mutations into the *αFAS* gene and/or elongase genes.

The mutated *αFAS* gene and/or the mutated elongase genes can be amplified for example by PCR using primers specific for said gene.

It is possible to use any selection method known to those skilled in the art which is compatible with the marker gene (or genes) used. The selectable markers which enable the complementation of an auxotrophy, also commonly referred to as auxotrophic markers, are well known to those skilled in the art in the field of yeast transformation. The URA3 selectable marker is well known to those skilled in the art. More specifically, a yeast strain in which the *URA3* gene (sequence available in the Genolevures database (http://genolevures.org/) under the name YALI0E26741g or the UniProt database under accession number Q12724), encoding orotidine-5'-phosphate decarboxylase, is inactivated (for example by deletion), will not be capable of growing on a medium not supplemented with uracil. The integration of the URA3 selectable marker into this yeast strain will then make it possible to restore the growth of this strain on a uracil-free medium. The LEU2 selectable marker described in particular in patent US 4 937 189 is also well known to those skilled in the art. More specifically, a yeast strain in which the *LEU2* gene (*e.g*., YALI0C00407g in *Y. lipolytica*), encoding β-isopropylmalate dehydrogenase, is inactivated (for example by deletion), will not be capable of growing on a medium not supplemented with leucine. As previously, the integration of the LEU2 selectable marker into this yeast strain will then make it possible to restore the growth of this strain on a medium not supplemented with leucine. The ADE2 selectable marker is also well known to those skilled in the art. A yeast strain in which the *ADE2* gene (*e.g*., YALI0B23188g in *Y. lipolytica*), encoding phosphoribosylaminoimidazole carboxylase, is inactivated (for example by deletion), will not be capable of growing on a medium not supplemented with adenine. Here again, the integration of the ADE2 selectable marker into this yeast strain will then make it possible to restore the growth of this strain on a medium not supplemented with adenine. Leu⁻ Ura⁻ auxotrophic *Y. lipolytica* strains have been described by Barth and Gaillardin, 1996.

Oleaginous yeast strains are well known in the art (Ratledge *et al*., 1994 and 2005). They naturally accumulate lipids to more than 20% of their dry cell weight. They include the genus *Candida, Cryptoccocus, Lipomyces, Rhodosporidium* (*e.g*., *Rhodosporidium toruloides*), *Rhodotorula* (*e.g*., *Rhodotorula glutinis*), *Trichosporon* and *Yarrowia.* According to this embodiment, the *Yarrowia* strain is preferably selected from the group consisting of a *Y. lipolytica, Y. galli, Y. yakushimensis, Y. alimentaria* and *Y. phangngensis* strain, more preferably a *Y. lipolytica* strain.

Said yeast can be genetically modified to accumulate lipids. This can be done by:
- preventing lipids (TAG) degradation/remobilization. For example, by inhibiting the expression and/or the activity of at least one endogenous lipase, preferably a triglyceride (TGL) lipase, more preferably the TGL4 lipase (*e.g*., in *Y. lipolytica*: YALI0F10010g) and/or TGL3 lipase (*e.g*., in *Y. lipolytica*: YALI0D17534g). A method of inhibiting the expression of the TGL3 and TGL4 in a *Y. lipolytica* strain is described in Dulermo *et al*., 2013;
- preventing fatty acid degradation by inhibiting beta oxidation. Enzymes involved in beta oxidation in yeast are for example the endogenous isoforms of acyl-coenzymeA oxidases (AOX, EC 6.2.1.3) or MFE enzyme (YALI0E15378g). In *Y. lipolytica,* 6 genes (*POX1*, *POX2, POX3, POX4, POX5* and *POX6* respectively YALI0E32835g, YALI0F10857g, YALI0D24750g, YALI0E27654g, YALI0C23859g and YALI0E06567g) encode these isoforms. Said inhibition can be total or partial. A method of inhibiting the expression of the 6 endogenous AOX in a *Y. lipolytica* strain is described in Beopoulos *et al*., 2008 and International Applications WO 2006/064131, WO 2010/004141 and WO 2012/001144;
- preventing peroxisome biogenesis. Because beta-oxidation takes place in the peroxisome, several strategies to block peroxisome biogenesis can be envisaged through the deletion of *PEX3*, *PEX10* and *PEX11* genes for example (US 20090117253 A1 and Dulermo et al.,2015)
- improving lipids accumulation by enhancing fatty acid incorporation into TAG. Said yeast strain having improved properties for lipid accumulation can be a mutant yeast strain, preferably a *Y. lipolytica* mutant strain, wherein at least one protein, preferably at least one endogenous or autologous protein, selected from the group consisting of an acyl-CoA:diacylglycerol acyltransferase 2 (encoded by *DGA1*), an acyl-CoA:diacylglycerol acyltransferase 1 (encoded by *DGA2*), a glycerol-3-phosphate dehydrogenase NAD+ (encoded by *GPD1*), an acetyl-CoA carboxylase (encoded by *ACC1*) is overexpressed.

A method of overexpressing the endogenous genes *DGA1*, *DGA2*, *GPD1* and *ACC1* and inhibiting the expression and/or activity of the endogenous genes *GUT2*, *TGL4* and *PEX10* in a *Y. lipolytica* strain is described in International Application WO 2014/136028.

A method of overexpressing the endogenous genes *DGA2*, *GPD1* and *HXK1*, and inhibiting the expression and/or activity of the endogenous gene *TGL4* in a *Y. lipolytica* strain is described in Lazar *et al.,* 2014.

Methods for transforming yeast are also well known to those skilled in the art and are described, *inter alia*, by Ito *et al.* (1983), Klebe *et al.* (1983) and Gysler *et al.* (1990).

Any gene transfer method known in the art can be used to introduce a gene encoding an enzyme. Preferably, one can use the method with lithium acetate and polyethylene glycol described by Gaillardin *et al*., (1987) and Le Dall *et al*., (1994).

A preferred method for expressing said mutated *α*FAS in a yeast strain comprises introducing into the genome of said yeast strain a DNA construct comprising a nucleotide sequence encoding said mutated αFAS, placed under the control of a promoter.

Method for expressing genes in *Yarrowia lipolytica* is well known as described in example in Nicaud *et al.* (2002; 2012).

The present invention also provides means for expressing in an oleaginous yeast strain, preferably a *Yarrowia* strain, more preferably a *Y. lipolytica,* a mutated *α*FAS as defined above.

This includes, in particular, recombinant DNA constructs for expressing a mutated *αFAS* as defined above in a yeast cell, in particular in a *Yarrowia* cell. These DNA constructs can be obtained and introduced in said yeast strain by the well-known techniques of recombinant DNA and genetic engineering.

Recombinant DNA constructs of the invention include in particular recombinant expression cassettes, comprising a polynucleotide encoding a mutated *α*FAS as defined above under the control of a suitable promoter such as promoter functional in a yeast cell as defined above.

The expression cassettes generally also include a transcriptional terminator, such as those describes above. They may also include other regulatory sequences, such as transcription enhancer sequences.

Recombinant DNA constructs of the invention also include recombinant vectors containing expression cassettes comprising a polynucleotide encoding a mutated *α*FAS as defined above under transcriptional control of a suitable promoter, such as a yeast (*e.g*., *Yarrowia*) promoter.

Recombinant vectors of the invention may also include other sequences of interest, such as, for instance, one or more marker genes, which allow for selection of transformed yeast cells.

The invention also comprises host cells containing a recombinant DNA construct of the invention. These host cells can be prokaryotic cells (such as bacteria cells) or eukaryotic cells, preferably yeast cells.

The present invention also provides a mutant yeast strain, preferably a mutant oleaginous yeast strain, more preferably a *Yarrowia* strain, even more preferably a *Y. lipolytica* strain, able to produce an increased ratio of fatty acids having a hydroxycarbon chain length consisting of 16 carbons (C16 fatty acids) to fatty acids having a hydroxycarbon chain consisting of 18 carbons (C18 fatty acids) and/or an increased amount of medium chain length fatty acids (C8-CI5 fatty acids), compared to the parent oleaginous yeast strain from which said mutant oleaginous yeast strain derives, wherein said mutant yeast strain expresses a mutated *αFAS* as defined above and optionally wherein the expression and/or the activity of the endogenous elongases 1 and/or 2 as defined above is inhibited and/or the expression and/or the activity of the endogenous *αFAS* is inhibited in said mutant yeast strain.

Said mutant yeast strain can be obtainable by the method for obtaining an oleaginous yeast strain capable of producing myristic acid (tetradecanoic acid) according to the invention as described above.

The mutant yeast strain of the invention includes not only the yeast cell resulting from the initial mutagenesis or transgenesis, but also their descendants, as far as the mutated αFAS as defined above is expressed and optionally as far as the expression and/or the activity of the endogenous αFAS and/or of the endogenous elongases 1 and/or 2 is inhibited.

The present invention also provides a mutant yeast strain, preferably an oleaginous yeast strain, more preferably *Yarrowia* strain, even more preferably a *Y. lipolytica* strain, wherein the ratio of fatty acids having a hydroxycarbon chain length consisting of 16 carbons (C16 fatty acids) to fatty acids having a hydroxycarbon chain consisting of 18 carbons (C18 fatty acids) and/or an increased amount of medium chain length fatty acids (C8-C15 fatty acids), compared to the parent oleaginous yeast strain from which said mutant oleaginous yeast strain derives, comprising, stably integrated in its genome, at least one recombinant DNA constructs for expressing mutated αFAS as defined above and optionally wherein the expression and/or the activity of the endogenous αFAS and/or of the endogenous elongases 1 and/or 2 is inhibited.

According to another particular embodiment of the invention, the yeast strain of the mutant yeast strain is derived from preferably from an oleaginous yeast, more preferably from a yeast belonging to the genus selected from the group consisting of *Candida, Cryptococcus, Lipomyces, Rhodosporidium, Rhodotorula, Trichosporon, Saccharomyces* and *Yarrowia,* most preferably from *Yarrowia,* in particular from *Yarrowia lipolytica.*

According to a more particular embodiment of the invention, the oleaginous yeast strain is selected from the group consisting of a *Y. lipolytica, Y. galli, Y. yakushimensis, Y. alimentaria* and *Y. phangngensis* strain, preferably a *Y. lipolytica* strain.

Methods for growing oleaginous yeast strains are well known in the art, e.g, one can use an oleaginous yeast fermentation technology (Aggelis et al, 1999, Papanikolaou et al. 2006).

Mutant yeast strains according to the invention can be grown with fatty acid complementation.

The mutant yeast strain of the invention can be cultured in repeated batch, fed-batch on continuous cultures as planktonic cell or biofilm (i.e., cell growing on the surface or inside a solid support).

The present invention will be understood more clearly from the further description which follows, which refers to non-limitative examples illustrating the expression of a mutated fatty acid synthase in *Y. lipoytica.*
**Figure 1** shows the fatty acid profile in mg/l for the mutated strains ΔαFAS + αFASI1220F clone A, B and C after 5 days of culture in minimum medium.
**Figure 2** shows the fatty acid profile in mg/mL for the strain ΔαFAS transformed with αFASwt or with mutated αFAS after five days of culture in minimum medium complemented or not with Oleic acid (AO). /: Minimum medium non-inoculated complemented with oleic acid; IW: strain ΔαFAS + αFASI1220W; IWST: strain ΔαFAS + αFAS I1220W/S1305T; MM AO: minimum medium complemented with oleic acid; wt: strain ΔαFAS + αFASwt; MM: minimum medium with no complementation.
**Figure 3** shows the fatty acid profile in mg/mL for ΔαFAS strain transformed with αFASwt or with mutated αFAS after five days of culture in minimum medium. Wt: strain ΔαFAS + αFASwt; I1220F: strain ΔαFAS + αFASI1220F; I1220M: strain ΔαFAS + αFASI1220M; I1220F/S1305T: strain ΔαFAS + αFAS I1220F/S1305T.
**Figure 4** shows the plasmid map of the shuttle plasmid JMP62Leu2expTEF including the *αFAS* gene of *Y. lipolytica.*

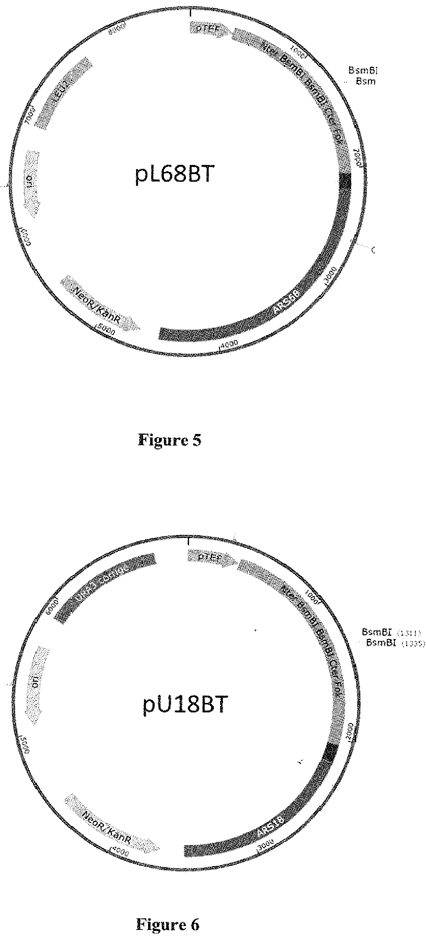

**Figure 5** shows the plasmid map of the shuttle plasmid pL68BT.
**Figure 6** shows the plasmid map of the shuttle plasmid pU18BT.
**Figure 7** shows the plasmid map of the shuttle plasmid pL68TAL_KSl.
**Figure 8** shows the plasmid map of the shuttle plasmid pU18TAL_KSr.

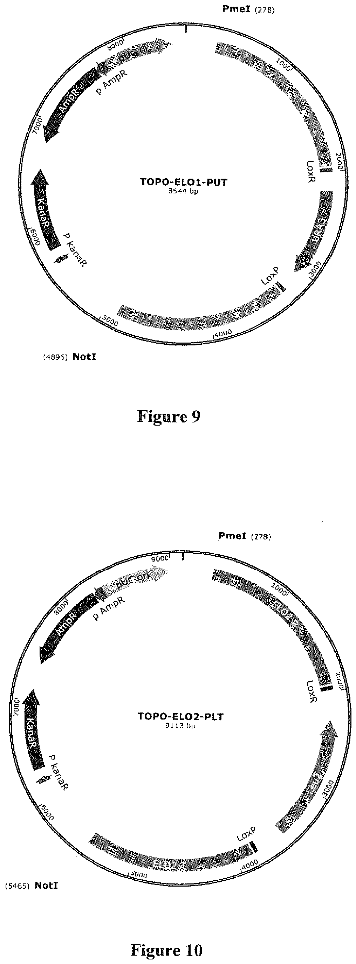

**Figure 9** shows the plasmid map of the shuttle plasmid TOPO-ELO1-PUT.
**Figure 10** shows the plasmid map of the shuttle plasmid TOPO-ELO2-PLT.
**Figure 11** shows the plasmid map of the shuttle plasmid pUB4-CRE.
**Figure 12** shows the FA profile in mg/mL for each mutant (the letter stands for the amino acid replacing the residue I in position 1220. For example, E: mutant I1220E). Fatty acids were extracted from the cells and medium.
**Figure 13****:** shows the FA profile in mg/mL for each mutant (the letter stands for the amino acid replacing the residue I in position 1220. For example, Y: mutant I1220Y). Fatty acids were extracted from the cells and medium.
**Figure 14****:** shows FA profile in mg/mL for the strain JMY1233_FAS- after 5 days of culture in minimum medium supplemented with either methylC14 (MM mC14) or Oleic acid (MM AO). Fatty acids were extracted from the cells.
**Figure 15****:** FA profile in mg/mL for the strain JMY1233_FASI1220F (IF) and the strain JMY1233_ΔElo1ΔElo2_FASI1220F after 5 days of culture in minimum medium. Fatty acids were extracted from the cells and medium.

### EXAMPLE 1: YARROWIA LIPOLYTICA MUTANTS WITH ENGINEERED FATTY ACID SYNTHASE TO PRODUCE MEDIUM CHAIN FATTY ACIDS

### 1. Materials and Methods

### a. Media

Rich medium YPD (Yeast extract 10g/L, bactopeptone 10g/L, glucose 10g/L) was used for growing cells prior genomic extraction and for start cultures. Minimal medium YNB (glucose 10 g/L, YNB w/o AA 1,7 g/l, NH₄Cl 5g/L, Phosphate buffer pH 6,8 50 mM, agarose 15 g/l) was used to select colonies after transformation. When necessary, Oleic Acid prepared in Tween^{®} 40 was added at 0.1 % final. To grow cells for analysis of lipid content, the specific minimal medium for lipid accumulation was used (glucose (80 g/L), ammonium sulfate (1,5 g/L), Phosphate buffer (100 mM), oligo elements: CoCl₂ 0,5 mg/L, CuSO₄ 0.9 mg/L, Na₂MoO₄ 0.06 mg/L, CaCl₂ 23 mg/L, H₃BO₃ 3 mg/L, MnSO₄ 3,8 mg/L, MgSO₄ 10 mg/L, ZnSO₄ 40 mg/L, FeSO₄ 40 mg/L, vitamins (D-biotin 0,05 mg/L, Panthoténate 1 mg/L, nicotinic acid 1 mg/L, Myo inositol 25 mg/L, Thiamine hydrochloride 1 mg/L, Pyridoxol hydrochloride 1 mg/L, p-aminobenzoic acid 0,2 mg/L). For mutants that required oleic acid to grow, 0.1% was added.

### b. Strains

The strain JMY3776 (Genotype MATa URA3-302 Leu2-270 xpr2-322 Δphd1 Δmfe Δtgl4 +TEF ylDGA2 + TEF GPD1) disclosed in International Application WO 2014136028 A1 was deleted of its αFAS gene (SEQ ID NO: 10) by homologous recombination leading to the ΔαFAS strain JMY4368 (Genotype MATa URA3-302 Leu2-270 xpr2-322 Δphd1 Δmfe Δtgl4 +TEF ylDGA2 + TEF GPD1 + PTFAS2). The deletion cassette of sequence SEQ ID NO: 16 were typically generated by PCR amplification according to Fickers *et al.* (2003).

### c. Plasmids

Gene were cloned into the shuttle plasmid JMP62Leu2expTEF (Beopoulos *et al.,* 2014) harboring a replicative origin and a kanamycine resistance gene for plasmid proliferation and selection in *E. coli* and the gene encoding for LEU2 protein for selection *in Y. lipolytica.*

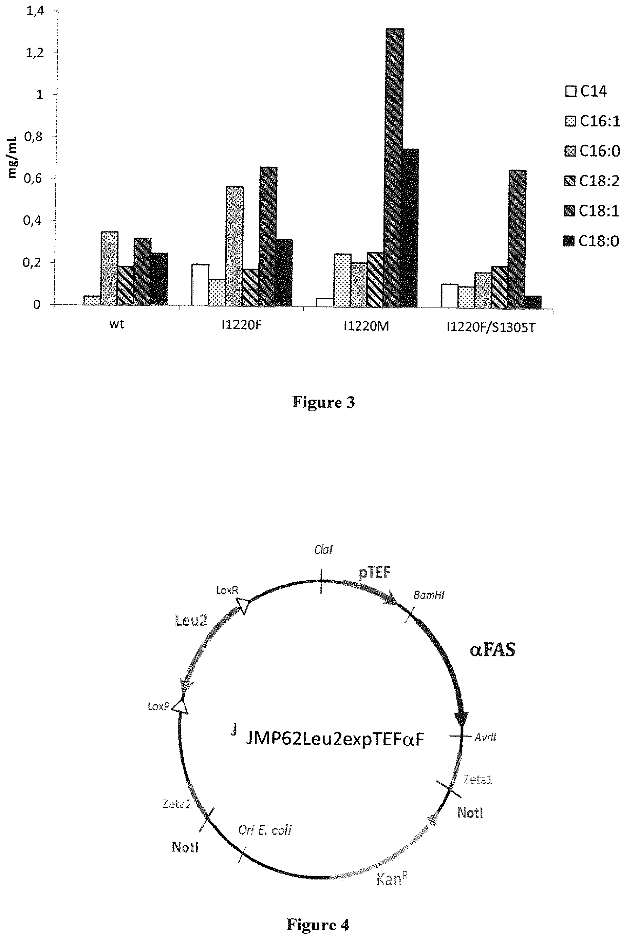

The plasmid map of the shuttle plasmid JMP62Leu2expTEF including the *FAS* gene of *Y. lipolytica* (JMP62Leu2expTEFαFAS) in shown in Figure 4.

### d. Expression cassette and transformation

In parallel, the whole αFAS gene (SEQ ID NO: 10; amplified from genomic DNA of the PO1d strain; Barth and Gaillardin, 1996 with the αFAS F and αFAS R primers) was cloned in the JMP62Leu2expTEF plasmid using in-Fusion® technic leading to JMP62Leu2expTEFαFAS plasmid. To generate the cassette for random genomic integration of the αFAS gene in the strain, the plasmid JMP62Leu2expTEFαFAS was digested with *Not*I and used for transformation into the ΔαFAS strain. Transformations were plated on YNB medium for a selection by integration of the Leucine marker. Transformation was performed using the Frozen-EZ Yeast Transformation II Kit™ (Zymoresearch) following manufacturer instructions.

**Table 2: Primers used in this study**

| Primer | Sequence (5' - 3') | SEQ ID NO: |
|---|---|---|
| alphaFAS F | ACCCGAAGGATCCCACAATGCACCCCGAAGTCGAACAAGAAC | 20 |
| alphaFAS R | ACCACAGACACCCTAGGCTACTCGGCAACAGCAACAGCCA | 21 |
| FAS_S1305T F | atgatttccaggaggagacttctcaggagtttgca | 22 |
| FAS_S1305T R | tgcaaactcctgagaagtctcctcctggaaatcat | 23 |
| FAS_M1217F_F | ctgttccggttccggtttcggtggtatcaccg | 24 |
| FAS_M1217F_R | cggtgataccaccgaaaccggaaccggaacag | 25 |
| FAS_M1217Y_F | aactgttccggttccggttacggtggtatcaccgcc | 26 |
| FAS_M1217Y_R | ggcggtgataccaccgtaaccggaaccggaacagtt | 27 |
| FAS_I1220M F | tccggtatgggtggtatgaccgccctg | 28 |
| FAS_I1220M R | cagggcggtcataccacccataccgga | 29 |
| FAS_I1220F F | ccggtatgggtggtttcaccgccctgc | 30 |
| FAS_I1220F R | gcagggcggtgaaaccacccataccgg | 31 |
| FAS_I1220W F | tccggttccggtatgggtggttggaccgccctgcg | 32 |
| FAS_I1220W R | cgcagggcggtceaaccacccataccggaaccgga | 33 |
| KS-T7_F | TGGATGGGATGCCCGACGATA | 34 |
| KS-T7_R | GAGTGATCTCGGTCTCAGCGTT | 35 |
| HRscreening_R | GGTCCTTaAACATaCCtCtg | 36 |
| WTscreening_R | GGTCCTTGAACATGCCTCGC | 37 |
| KS_P1 | GTCGTTGAGAACAAGGCTGTTTCTG | 38 |
| KS_T2 | GAATTTGGATAGTAGGGGCCACAGT | 39 |
| Matrix KS-TALEN 1F | AACGGTGGCTTCCAGTACATTCC | 40 |
| Matrix KS-TALEN 4R | TTGGATGGCTCCGTTGAGCATCCA | 41 |
| Matrix KSI1220L 3F | ATGGGTGGTCTGACCGCCCTcaGaGGtATGTTtAAGGACCGGTTCATGGAC | 42 |
| Matrix KSI1220L 2R | AGGGCGGTCAGACCACCCATgCCGGAtCCaGAgCAGTTACCGACCTCGGA | 43 |
| Matrix KSI1220V 3F | ATGGGTGGTgtcACCGCCCTcaGaGGtATGTTtAAGGACCGGTTCATGGAC | 44 |
| Matrix KSI1220V 2R | AGGGCGGTgAcACCACCCATgCCGGAtCCaGAgCAGTTACCGACCTCGGA | 45 |
| Matrix KSI12205 3F | ATGGGTGGTtccACCGCCCTcaGaGGtATGTTtAAGGACCGGTTCATGGAC | 46 |
| Matrix KSI12205 2R | AGGGCGGTGGAACCACCCATgCCGGAtCCaGAgCAGTTACCGACCTCGGA | 47 |
| Matrix KSI1220P 3F | ATGGGTGGTcccACCGCCCTcaGaGGtATGTTtAAGGACCGGTTCATGGAC | 48 |
| Matrix KSI1220P 2R | AGGGCGGTGGgACCACCCATgCCGGAtCCaGAgCAGTTACCGACCTCGGA | 49 |
| Matrix KSI1220T 3F | ATGGGTGGTaccACCGCCCTcaGaGGtATGTTtAAGGACCGGTTCATGGAC | 50 |
| Matrix KSI1220T 2R | AGGGCGGTGGtACCACCCATgCCGGAtCCaGAgCAGTTACCGACCTCGGA | 51 |
| Matrix KSI1220A 3F | ATGGGTGGTgccACCGCCCTcaGaGGtATGTTtAAGGACCGGTTCATGGAC | 52 |
| Matrix KSI1220A 2R | AGGGCGGTGGcACCACCCATgCCGGAtCCaGAgCAGTTACCGACCTCGGA | 53 |
| Matrix KSI1220Y 3F | ATGGGTGGTtacACCGCCCTcaGaGGtATGTTtAAGGACCGGTTCATGGAC | 54 |
| Matrix KSI1220Y 2R | AGGGCGGTGtAACCACCCATgCCGGAtCCaGAgCAGTTACCGACCTCGGA | 55 |
| Matrix KSI1220H 3F | ATGGGTGGTcacACCGCCCTcaGaGGtATGTTtAAGGACCGGTTCATGGAC | 56 |
| Matrix KSI1220H 2R | AGGGCGGTGtgACCACCCATgCCGGAtCCaGAgCAGTTACCGACCTCGGA | 57 |
| Matrix KSI1220Q 3F | ATGGGTGGTcagACCGCCCTcaGaGGtATGTTtAAGGACCGGTTCATGGAC | 58 |
| Matrix KSI1220Q 2R | AGGGCGGTctgACCACCCATgCCGGAtCCaGAgCAGTTACCGACCTCGGA | 59 |
| Matrix KSI1220N 3F | ATGGGTGGTaacACCGCCCTcaGaGGtATGTTtAAGGACCGGTTCATGGAC | 60 |
| Matrix KSI1220N 2R | AGGGCGGTGttACCACCCATgCCGGAtCCaGAgCAGTTACCGACCTCGGA | 61 |
| Matrix KSI1220K 3F | ATGGGTGGTaagACCGCCCTcaGaGGtATGTTtAAGGACCGGTTCATGGAC | 62 |
| Matrix KSI1220K 2R | AGGGCGGTcttACCACCCATgCCGGAtCCaGAgCAGTTACCGACCTCGGA | 63 |
| Matrix KSI1220D 3F | ATGGGTGGTgacACCGCCCTcaGaGGtATGTTtAAGGACCGGTTCATGGAC | 64 |
| Matrix KSU1220D 2R | AGGGCGGTGtcACCACCCATgCCGGAtCCaGAgCAGTTACCGACCTCGGA | 65 |
| Matrix KSI1220E 3F | ATGGGTGGTgagACCGCCCTcaGaGGtATGTTtAAGGACCGGTTCATGGAC | 66 |
| Matrix KSI1220E 2R | AGGGCGGTctcACCACCCATgCCGGAtCCaGAgCAGTTACCGACCTCGGA | 67 |
| Matrix KSI1220C 3F | ATGGGTGGTtgcACCGCCCTcaGaGGtATGTTtAAGGACCGGTTCATGGAC | 68 |
| Matrix KSI1220C 2R | AGGGCGGTGcaACCACCCATgCCGGAtCCaGAgCAGTTACCGACCTCGGA | 69 |
| Matrix KSI1220M 3F | ATGGGTGGTATGACCGCCCTcaGaGGtATGTTtAAGGACCGGTTCATGGAC | 70 |
| Matrix KSI1220M 2R | AGGGCGGTCATACCACCCATgCCGGAtCCaGAgCAGTTACCGACCTCGGA | 71 |
| Matrix KSI1220W 3F | ATGGGTGGTtggACCGCCCTcaGaGGtATGTTtAAGGACCGGTTCATGGAC | 72 |
| Matrix KSI1220W 2R | AGGGCGGTccaACCACCCATgCCGGAtCCaGAgCAGTTACCGACCTCGGA | 73 |
| matrix KSI1220F 3F | ATGGGTGGTTTCACCGCCCTcaGaGGtATGTTtAAGGACCGGTTCATGGAC | 74 |
| matrix KSI1220F 2R | AGGGCGGTGAaACCACCCATgCCGGAtCCaGAgCAGTTACCGACCTCGGA | 75 |
| Matrix KSI1220R 3F | ATGGGTGGTcgaACCGCCCTcaGaGGtATGTTtAAGGACCGGTTCATGGAC | 76 |
| Matrix KSI1220R 2R | AGGGCGGTtcgACCACCCATgCCGGAtCCaGAgCAGTTACCGACCTCGGA | 77 |
| Matrix KSI1220G 3F | ATGGGTGGTggcACCGCCCTcaGaGGtATGTTtAAGGACCGGTTCATGGAC | 78 |
| Matrix KSI1220G 2R | AGGGCGGTgccACCACCCATgCCGGAtCCaGAgCAGTTACCGACCTCGGA | 79 |
| FAS_S1305V F | cgatgatttccaggaggaggtttctcaggagtttgcaaac | 80 |
| FAS_S1305V R | gtttgcaaactcctgagaaacctcctcctggaaatcatcg | 81 |

### e. Construction of the mutated strain

Site directed mutagenesis was performed on position I1220 and M1217 *in vitro* using the QuickChange Site-Directed Mutagenesis kit from Agilent, using the plasmid JMP62LEU2expTEFαFAS as DNA template and the primers listed in Table 2. After transformation in *E. coli,* plasmid extraction and sequencing, the plasmid was digested with *Not*I and used for transformation in *Y. lipolytica.* Clones were selected for Leucine integration on minimum medium. Due to the Fatty acid auxotrophy of the ΔαFAS and the length of the integration cassette (8,6 kb), efficiency of transformation was usually low (around 20 cfu/µg plasmid). Gene integration was verified by amplification of the αFAS gene from genomic DNA of the selected clones followed by sequencing. Validated mutants were then evaluated for their ability to grow on medium without fatty acid (Oleic acid) and for their fatty acid profile after cultivation.

### f. DNA extraction

Plasmids were extracted from *E. coli* cells and genomic DNA from *Y. lipolytica* using QIAprep spin miniprep™ kit (QIAGEN^{®}) after the cells were grown overnight in LB Kanamycin or YPD respectively.

### g. Sequencing

Sequencing was performed by Sanger method (GATC-Biotech^{®}, LIGHTRUN™).

### h. Growth and Fatty acid analysis

Mutants were grown in 50 mL of minimum medium required for lipid accumulation at 28°C for 5 days. 2 mL samples were collected after 5 days for a measure of the growth and were lyophilized for further analysis. FA profile was analyzed after transmethylation. 2 mL of a solution of Methanol with 2.5% sulfuric acid is added to the dried sample in addition to 1 mL of toluene. Samples are heated at 80°C for 3h. Once the samples are cooled down, biphasic liquid extraction takes place using 1.5 mL NaCl and 1.5 mL hexane. Samples are mixed and centrifuged to separate organic to water phase. Analyses are performed on organic phase with a gas chromatography coupled with Mass Spectrometry (GC-MS) *TRACE*™ 1310. For all the mutants tested, fatty acids were extracted both from the supernatant and the cells. However, the amount detected in the supernatant represented less than 1% of total FA extracted.

### 2. Results

### Analysis per mutant

Table 3 below summarizes the mutants constructed and their fatty acid profile. The position chosen for mutagenesis, the number of clones validated for each construct, the requirement in fatty acid for growth and their ability to produce medium chain length fatty acid are also indicated.

Table 3: Mutations tested in Y1_FAS,. For each mutant, requirement in fatty acid for growth and their ability to increase the ratio of fatty acids having a hydroxycarbon chain length consisting of 16 carbons (C16 fatty acids) to fatty acids having a hydroxycarbon chain consisting of 18 carbons (C18 fatty acids) and/or to increase the amount of medium chain length fatty acids (C8-C15 fatty acids).

| Mutations | Fatty acid auxotrophy | Increase of ratio C16/C18 FA and/or increase of the amount of Medium chain length FA |
|---|---|---|
| WT | no | no |
| M1217F | no | no |
| M1217Y | yes | no |
| I1220F | no | yes |
| I1220M | no | yes |
| I1220W | yes | yes |
| I1220F S1305V | yes | no |
| I1220W S1305T | yes | yes |
| I1220F S1305T | no | yes |

For each position to assess, several variants were obtained (Table 3) and were first tested separately. It was found a good reproducibility in term of FA profile between the 2 or 3 variants carrying the same mutation. A difference in quantity of FA accumulated could nonetheless be found among the variants.

### Example for the mutant ΔαFAS + αFASI1220F

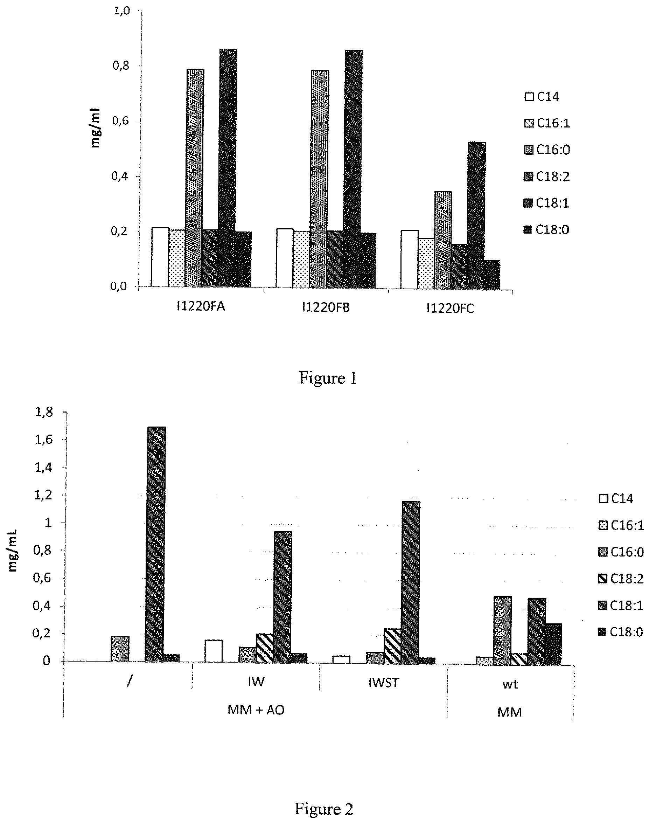

The results obtained for the mutant ΔαFAS + αFASI1220F are shown in Figure 1. Three different variants (A, B and C) were validated for the αFASI1220F integration after transformation in the ΔαFAS strain. Oleic acid complementation was not necessary for their growth meaning that the αFAS mutated at position I1220 was retaining enough activity to allow the normal growth of the strain. The 3 variants were cultivated and the FA content was analyzed in term of quantity (Figure 1). As mentioned previously, it was found a good reproducibility in term of FA species repartition: 50% of total FA was C18 species with a majority of C18:1, 40% was C16 species and interestingly around 10% was the medium chain length Fatty acid C14. The amount of C14 reached 0.2 mg/mL for the three variants. Because variant C is capable of producing the highest percentage of C14 it was chosen for further experiment.

In order to compare the mutants, it was decided to choose for each mutated position the clone that showed the highest percentage of medium chain length Fatty acids.

### Results for mutants auxotrophic for fatty acid

The clones harboring the mutations I1220W and M1217Y were not capable of growing without fatty acid complementation. This result suggests that whether the mutated αFAS is completely inactive and cannot produce any fatty acid or it is active but it produces fatty acid species that cannot support the growth of the strain (short or medium chain length FA). To answer this question, the variants were cultivated in the presence of oleic acid to support their growth. For lipid content analysis, cells were washed out of the medium containing the added oleic acid before transmethylation. After separation on GC-MS, it could not be detected any short or medium chain length FAs for the mutant M1217Y. However, for the mutants ΔαFAS + αFAS I1220W and ΔαFAS + αFAS I1220W, S1305T, C14 was produced and seemed to be the only FA specie neosynthesized (Figure 2).

### Result for the mutant that behaves like the control strain

The mutant ΔαFAS + αFASM1217F was grown without any fatty acid complementation and analyzed as indicated above. It could not be found any difference of FA content between these clones and the control strain ΔαFAS + αFASwt, indicating that this particular mutation does not seem to have an impact on the FAS activity.

### Comparison between mutants producing MCFA

For each mutant producing C14, one representative mutant was tested and compared for their FA profile (Figure 3)

The mutants were cultivated without oleic acid complementation along with the control strain ΔαFAS + αFASwt expressing a wild type copy of the αFAS gene. It was found that the mutant ΔαFAS + αFASI1220F produced the highest percentage and quantity of C14 (9% of total FA and 0.2 mg/mL). The percentage of C18 species was greatly reduced compared to the wild type (68% to 57%) whereas the percentage of C16 species did not change significantly. This suggests that C14 species are produced at the expense of C18 FAs. The mutant ΔαFAS + αFASI1220M also produced C14 but to a level of 2% of total FAs and at the amount of 0.05 mg/mL. However, the relative amount of C16 FAs produced was enhanced to 48% of total FA extracted compared to the control strain that reached 32%. No significant difference was found for the double mutated strain ΔαFAS + αFASI1220F-S1305T compared to the single mutated strain ΔαFAS + αFASI1220F. The single mutant ΔαFAS + αFASI1220W and the double mutated strain ΔαFAS + αFASI1220W-S1305T could not grow without FA complementation. Cultivated in medium supplemented with Oleic acid, they were capable of producing C14 FA at a level of 0.15 mg/mL and 0.05 mg/mL respectively after 5 days (Figure 2).

### EXAMPLE 2 : MUTANT STRAINS OF YARROWIA LIPOLYTICA WITH MODIFIED LIPID PROFILE AND PRODUCING MEDIUM CHAIN LENGTH FATTY ACID.

### 1. Materials and Methods

### TALENs design and plasmids.

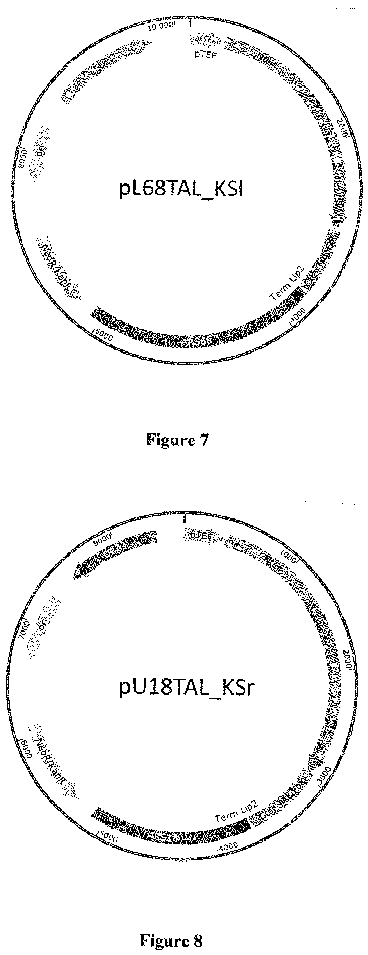

The replacement of the wild type FAS by a mutated FAS has been done via homologous recombination-mediated by engineered nuclease. A TALE-Nuclease (described and used to simulate targeted gene modifications (Christian *et al*., 2010 and Cermak *et al*., 2011)) has been designed to generate a double-strand break centered on the I1220 Codon position. The TALE-Nuclease_KS encoded by the TAL_KSr (SEQ ID NO: 17) and the TAL_KSl (SEQ ID NO: 18) plasmids was designed to cleave the DNA sequence (5'-TGTTCCGGTTCCGGTATgggtggtatcaccgcCCTGCGAGGCATGTTCA -3'. The sequences of the corresponding TAL_KS l and TAL_KS r were synthesized following the Golden Gate TALEN kit (Addgene) and cloned into a shuttle plasmid designed and constructed for usage in *Yarrowia lipolytica.* The empty plasmids pL68 and pU18 harbor a yeast origin of replication (ARS68 and ARS18 respectively), a selection marker (LEU2 or URA3 respectively) in addition to an origin of replication in *E. coli* and the Kanamycin resistance encoding gene. Shuttle plasmids pL68BT and pU18BT were built from the empty plasmids pL18 and pU18 by insertion of the N-terminal and C-terminal sequences for optimal TALEN scaffolding in between the constitutive promoter pTEF and the Lip2 terminator. Subsequently, TAL_KSr and TAL_KSl were cloned into pU18BT and pL68BT plasmids giving respectively pU18TAL-KSr and pL68TAL_KSl (Figures 7 and 8). Plasmids were amplified in *E. coli*, extracted and sequences checked before further utilization in *Yarrowia lipolytica.*

### Matrix design

19 different matrixes were synthesized by PCR fusion of two overlapping PCR products synthesized using primers carrying the desired mutations substituting the I1220 codon by the 19 other amino acids codons (listed in table 2). In addition, four silent mutations were introduced into each TALEN target site to prevent the TALEN to bind to the matrix. In addition, these silent mutations allowed the design of a matrix specific primer, used consequently for the screening of desired clones, i.e. where the Homologous Recombination (HR) between the matrix and the chromosome occurred. As an example, the matrix DNA sequence corresponding to the mutation I1220F is given in SEQ ID NO: 19.

### Strain and transformation

The strain *Yarrowia lipolytica* JMY1233 (*MATa, leu2-270, ura3-302, xpr2-322, Δpox1-6*) was used in this study. It has been deleted of the β-oxidation pathway (Beopoulos *et al.* 2008) and is auxotroph for Uracil and Leucine. This strain is used as a platform for the engineering of strain producing new or original lipids. β-oxidation was removed to prevent degradation of any new types of fatty acid produced by the engineered strain. JMY1233 cells were made competent with the Frozen-EZ Yeast Transformation II Kit ™ (Zymoresearch). Transformations were performed as described by the manufacturer using 50µL of competent cells and 500ng of each of the empty plasmids pU18BT and pL68BT or TALEN plasmids pU18TAL_KSr and pL68TAL_KSl.

For homologous recombination experiments, 500 ng of matrix of sequence SEQ ID NO: 19 was used in addition of the pU18BT and pL68BT plasmids or the pU18TAL_KSr and pL68TAL_KSl plasmids.

Transformants were selected on YNB AO plates for the selection of FAS+ and FAS- colonies. After transformation, colonies were grown on rich medium and streaked on plates to allow the loss of the replicative plasmids. Genomic DNA was extracted from the cultures and screened for HR event by PCR.

### PCR screening on genomic DNA

Primers used for the screening and the sequencing are listed in table 2. Genomic DNA extractions were performed on 2mL overnight cultures in YPD medium using *QIAprep spin miniprep*™ (Qiagen). PCR were carried out with 1 µL of gDNA. For the screening of NHEJ clones, KS-T7_F and KS-T7_R primers were used to amplify a fragment of 500 pb centered on I1220 codon position. KS-T7_F primer was then used to sequence the PCR product. For the screening of the HR experiment clones, the primers KS_P1 and HR screening_R or WT screening_R were used. KS_P1 and KS_T2 primers were then used to amplify a fragment of 4000 bp and KS-T7_F primer used to sequence this PCR product in order to confirm the introduction of the desired mutation at position I1220.

### 2. Results

19 transformations using the desire matrix were carried out and the screening was focused on the small white colonies growing on YNB AO plates that gave the best HR frequency and were present for all the transformations. For 17 mutations, at least 2 clones "HR positive" could be identified rapidly by PCR screening of less than 10 small white colonies. Regarding the transformation with the matrixes I1200R and I1220D, no small white colonies were detected. These particular amino acids at position 1220 could be deleterious for the FAS activity, leading to FAS- phenotype upon HR. Translucent colonies displaying a FAS-phenotype were screened on rich medium supplemented with oleic acid and HR positive clones were found.

### Analysis of the mutants

After the clones were grown overnight in Liquid YPD medium and streaked on YPD plates, 100% of the clones had lost both pU18TAL-KSl and pL68TAL_KSr plasmids. All the clones obtained being URA- and LEU-, were transformed with the empty plasmid pL68 and pU 18 in order to maintain the prototrophy of the strain without maintaining the TALEN. All the clones were grown in minimum medium and lipid content was extracted and analyzed. Results obtained were the following: When Isoleucine in position 1220 was replaced by Arg or Asp, mutants required to be complemented with Oleic acid to grow. This suggests that either the FAS activity is completely lost or the mutated FAS cannot support the synthesis of FA suitable for the growth. Analysis of the FA profile for the cultures grown in the presence of AO to complement the lack of suitable lipids showed that no neo-synthesis of FA occurred. For all other amino acid substitutions at position 1220, the cells were able to grow without addition of exogenous Fatty acid in the medium.

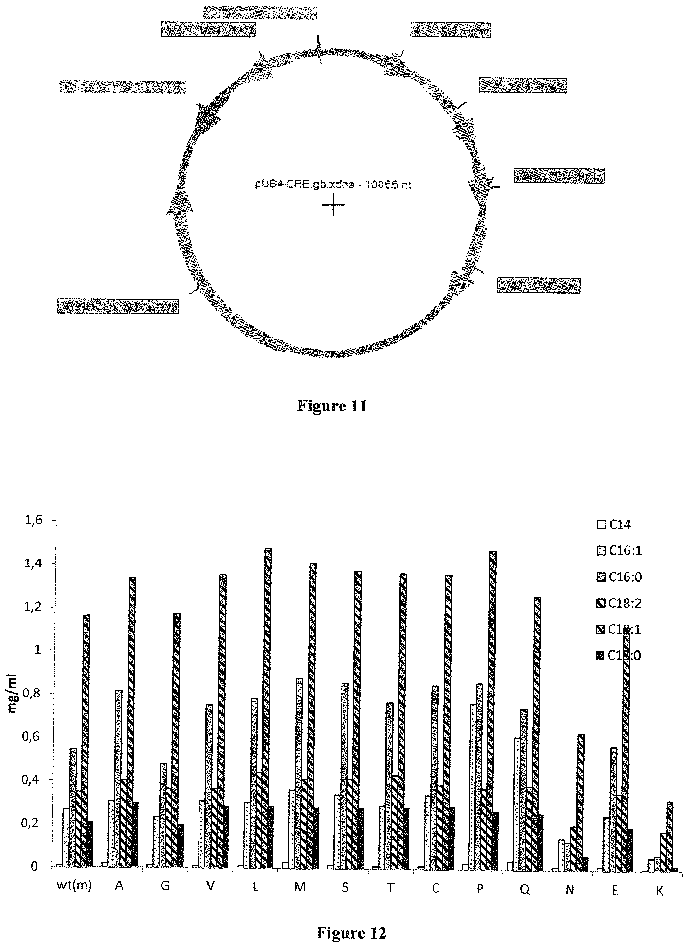

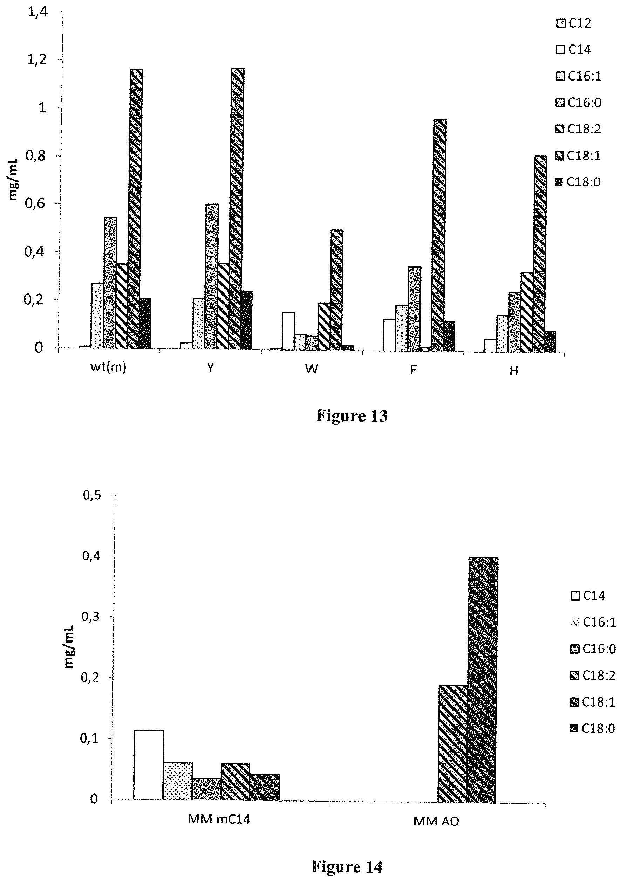

When Isoleucine 1220 was replaced by Ala, Gly, Val, Leu, Met, Ser, Thr, Cys, Pro or Gln residue, the strain displayed a FA profile overall quite similar to that of the wild type at 8 days (Figure 12). These mutations did not seem to impact FAS specificity or activity as accumulation was similar to that of the wild type. Nonetheless, amount of C14 for JMY1233_I1220M reached 0.03 mg/mL compare to the 0.007 mg/mL obtained for the wild type strain. When I220 was replaced by Asn, Glu or Lys, no change of specificity was observed. However, we could notice a lower accumulation of lipids. More interestingly, when I1220 was replaced by aromatic residues Trp, Tyr, Phe or His, FA profile was modified and significant amount of C14 was produced (Figure 13). C14 reached 0.025 mg/mL for the strain JMY1233_I1220Y, 0.052 mg/mL for JMY1233_I1220H, 0.13 mg/mL for JMY1233_I1220F and 0.16 mg/mL for JMY1233_I1220W. This corresponds respectively to a 4, 7, 17.5 and 29 fold increase of C14 accumulation (of DWC) compared to the wild type FAS. Of note, traces of C12 were also detected for JMY1233_I1220W.

**Conclusion:** Using targeted engineering approach leads to the generation of *Yarrowia lipolityca* strains harboring mutated FAS (I1220F or I1220W) which are both able to produce an increase amount of medium fatty acids chains.

### EXAMPLE 3: YARROWIA LIPOLYTICA ELONGASES ELO1 AND ELO2 ARE INVOLVED IN ELONGATION OF C14 AND C16 FATTY ACIDS SPECIES. A STRAIN OF YARROWIA LIPOLYTICA WITH A MUTATED FAS AND IIELETED OF THE ELONGASES ELO1 AND ELO2 SHOWS A LIPID PROFILE SHIFTED TOWARD SHORTER CHAINS.

### 1. Materiels and Methods

The strain JMY1233 (*MATa, leu2-270, ura3-302, xpr2-322, Δpox1-6* (Beopoulos *et al.* (2008)) was used to delete the two Elongase genes ELO1 (YALI0B20 1696p) and ELO2 (YALI0F06754p). The respective disruption cassette ELO1-PUT and ELO2-PLT were designed as described by Fickers et al., using the marker URA3 and LEU2 respectively. JMY1233 was made competent and was transformed by the Lithium acetate method. Disruption cassettes ELO1-PUT and ELO2-PLT were cloned into TOPO vector. Plasmids were digested with the restriction enzyme NotI HF / PmeI and used for transformation.
To excise selection markers, the strain was made competent and transformed with the PUB4-Cre plasmid. This plasmid carries the Cre recombinase allowing the marker excision by recombination of the LoxR and LoxP sites (flanking the URA3 or LEU2 marker).

### Media:

Strains were grown in 50 mL of minimum medium required for lipid accumulation at 28°C for 5 days. When necessary, medium was complemented with 0.2% Oleic acid or 0.2% methyl myristate (mC14) prepared at 20% in Tergitol. Along the culture, 2 mL samples were collected after 5 days for a measure of the growth and were lyophilized for further analysis. FA profile was analyzed after transmethylation. 2 mL of a solution of Methanol with 2.5% sulfuric acid is added to the dried sample in addition to 1 mL of toluene. Samples are heated at 80°C for 3h. Once the samples are cooled down, biphasic liquid extraction takes place using 1.5 mL NaCl and 1.5 mL hexane. Samples are mixed and centrifuged to separate organic to water phase. Analyses are performed on organic phase with a gas chromatography coupled with Mass Spectrometry (GC-MS) *TRACE*™ 1310. Fatty acids were extracted only from the cells.

### 2. Results:

### Strain J MY1233_ΔELO1ΔELO2:

The strain was made from the parental strain JMY1233 (URA- LEU-). ELO1 gene was first deleted using the disruption cassette ELO1-PUT giving the strain JMY1233_ΔELO1 (URA+ LEU-). Subsequently, this strain was used for the deletion of the second gene ELO2 using the disruption cassette ELO2-PLT, giving the strain JMY1233_ΔELO1ΔELO2 (URA+ LEU+). LEU2 and URA3 markers were excised from this strain leading to the strain JMY1233_ΔELO1ΔELO2 (URA- LEU-).

### Strain JMY1233_ΔELO1ΔELO2_FAS-

JMY1233_ΔELO1ΔELO2 (URA-LEU-) were made competent and were transformed with the plasmid PL68TAL-KS1 and PU18TAL-KSr as described in example 2. Transformation was plated on YNB AO to allow the growth of the FAS- clones (αFAS gene disrupted by NHEJ). The phenotype of the FAS- clones is easily noticeable since it shows a growth delay in medium supplemented with AO. One clone was selected, disruption of the FAS gene was verified by PCR and the selected clone was grown in rich medium complemented with AO in order to loose both replicative plasmids carrying the TALEN. The strains JMY1233_FAS-and JMY1233_ΔELO1ΔELO2_FAS- were then cultured in minimum or rich medium with methyl-C14 (mC14) as unique source of Fatty acid. These two strains are deficient in FAS activity hence are not capable of making their own fatty acid. The medium needs to be supplemented with Fatty acid.

When we used Oleic acid to complement the medium in FA, both strains were capable of growing at the same rate. However, when mC14 was used to complement the medium, only the strain JMY1233_FAS- was capable of growing. Lipids extraction and analysis showed that C16 species and C 18 species were produced from the mC14 provided in the medium (figure 14). The strain FAS- deleted of the two Elongase genes could not grow in C14. These results suggest that C14 must be elongated to C16 and C18 to be used by the FAS- cells and the Elongase genes are responsible for this elongation.

### Strain JMY1233_ΔELO1ΔELO2-FASI1220F

JMY1233_ΔELO1ΔELO2 (URA-LEU-) were made competent and were transformed with the plasmid PL68TAL-KS1 and PU18TAL-KSr in addition to the matrix I1220Fm as described in example 2. Small white colonies were screened by PCR to verify the homologous recombination. One clone identified as HR positive was selected, mutation I1220F was verified by sequencing and the clone was cultivated in rich medium in order to loose both replicative plasmids carrying the TALENs.

### Analysis of the strains

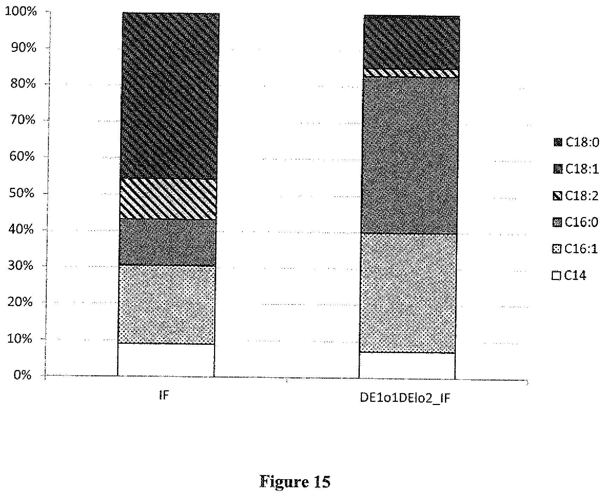

After genome edition with TALEN, the clones were URA- and LEU-. The clones were transformed with the integration cassettes carrying either the URA3 or LEU2 markers. Transformations were plated on YNB. The two strains JMY1233_FASI1220F and JMY1233_ΔELO1ΔELO2_FASI1220F were cultivated in Minimum medium for lipid accumulation as described in Example 1. Total FA were extracted after 5 and 12 days, results are given for day 5 in figure 15. The % of C14 was unchanged between the two strains. However, the amount of C16/C16:1 accumulated increased from 32% to 74% when Elongases are deleted. Consequently, the amount of C18 FA species decreased from 45% to 15% in the ΔELO1ΔElo2_FASI1220F strain. This suggests that Elol and Elo2 are likely involved in elongation of C16 species into C18 species.

### REFERENCES

Altschul, S., et al., 1997. Nucleic Acids Res. 25, 3389-3402.
Aggelis G. et al, 1999, Biotechnol Lett, 21, 747-9.
Barth, G. and Gaillardin, C. 1996. Yarrowia lipolytica. In: Nonconventional Yeasts in Biotechnology. Springer Berlin Heidelberg, Berlin, Heidelberg, pp. 313-388.
Beopoulos, A., et al., 2008. Appl. Environ. Microbiol. 74, 7779-7789.
Beopoulos, A., et al., 2009. Prog. Lipid Res. 48, 375-387.
Beopoulos, A., et al., 2014. Applied Microbiology and Biotechnology, 98, 251-262
Blazeck, J., 2011. Applied and Environmental Microbiology. 77, 7905-7914.
Blazeck, J., 2013. Applied Microbiology and Biotechnology. 97, 3037-3052.
Blazeck, J., et al., 2014. Nature Communications. 5, 3131.
Dulermo R., et al., 2015. Eukayot cell 14(5) :511-25
Edgar, R.C., 2004. BMC Bioinformatics. 5, 113.
Eswar, N., et al., 2006. Comparative protein structure modeling using Modeller. Curr. Protoc. Bioinforma. Ed. Board Andreas Baxevanis Al Chapter 5, Unit 5.6.
Fernandez-Moya, R., et al., 2015. Biotechnol. Bioeng. 112, 2618-2623.
Fickers, P., et al., 2003. Journal of Microbiological Methods. 55, 727-737.
Gaillardin, C., et al., 1987. Current Genetics. 11, 369-375.
Gysler, C., et al., 1990, Biotechnology Techniques. 4, 285-290.
Ito, H., et al., 1983, Journal of Bacteriology. 153, 163-168.
Jing, F., et al., 2011. BMC Biochem. 12, 44.
Klebe, R.J., et al., 1983, Gene. 25, 333-341.
Lazar Z., et al., 2014, Metab. Eng. 26, 89-99.
Leber, C., and Da Silva, N.A., 2014. Biotechnol. Bioeng. 111, 347-358.
Le Dall, M.T., et al., 1994, Current Genetics. 26, 38-44.
Ledesma-Amaro, R., and Nicaud, J.-M., 2016. Prog. Lipid Res. 61, 40-50.
Leibundgut, M., et al., 2008. Curr. Opin. Struct. Biol. 18, 714-725.
Madzak, C., et al., 2000, Journal of Molecular Microbiology and Biotechnology. 2, 207-216.
Madzak, C., et al., 2004, Journal of Biotechnology.109, 63-81.
Madzak, C., 2015. Appl. Microbiol. Biotechnol. 99, 4559-4577.
Maftahi, M., et al., 1996, Yeast. 12, 859-868.
Müller, S., et al., 1998. Yeast. 14, 1267-1283.
Nicaud, J-M, et al., 2002. FEMS Yeast Research 2, 371-379.
Nicaud, J.-M., 2012. Yeast. 29, 409-418.
Orr-Weaver, T.L., et al., 1981, Proc Natl Acad Sci USA, 78, 6354-6358.
Papanikolau S. et al, 2006, Curr Microbiol, 52, 134-42.
Ratledge C., 1994. Yeasts, moulds, algae and bacteria as sources of lipids. Technological advances in improved and alternative sources of lipids. B. S. Kamel, Kakuda, Y. London, Blackie academic and professional, 235-291.
Ratledge, C., 2005. Single cell oils for the 21th century, In: Cohen, R. (Eds.), Single cell oils. AOCS Press, Champaign, pp. 1-20.
Rutter, C.D., et al., 2015. Appl. Microbiol. Biotechnol. 99, 7359-7368.
Sangwallek, J., et al., 2013. Arch. Microbiol. 195, 843-852.
Schütt, B.S., et al., 1998. Planta. 205, 263-268.
Stoops J.K. et al., 1978. JBC. 253, 4464-4475.
Wang, H.J., et al., 1999, Journal of Biotechnology. 181, 5140-5148.
Zang l. et al 2005 JBC,28, 12422-12429.

## Claims

1. A method for increasing the ratio of fatty acids having a hydroxycarbon chain length consisting of 16 carbons (C16 fatty acids) to fatty acids having a hydroxycarbon chain consisting of 18 carbons (C18 fatty acids) and/or for increasing the amount of medium chain length fatty acids (C8-C15 fatty acids), produced by a yeast strain, compared to the parent yeast strain from which said yeast strain derives, comprising expressing in said yeast strain a mutated fatty acid synthase subunit alpha (αFAS), wherein the amino acid residue of said αFAS corresponding to the amino acid residue at position 1220 in SEQ ID NO: 1 is substituted with a larger steric hindrance amino acid residue, and, optionally, wherein the amino acid residue of said αFAS corresponding to the amino acid residue at position 1305 in SEQ ID NO: 1 is substituted with any other amino acid.

2. The method according to claim 1, wherein:
- when the amino acid residue corresponding to the amino acid residue at position 1220 in SEQ ID NO: 1 of the non-mutated αFAS is isoleucine (I) then it is substituted with an amino acid residue selected from the group consisting of phenylalanine (F), histidine (H), methionine (M), tryptophan (W) and tyrosine (Y), preferably selected from the group consisting of phenylalanine (F) and tryptophan (W),
- when the amino acid residue corresponding to the amino acid residue at position 1220 in SEQ ID NO: 1 of the non-mutated αFAS is valine (V) then it is substituted with an amino acid residue selected from the group consisting of isoleucine (I) phenylalanine (F), histidine (H), methionine (M), tryptophan (W) and tyrosine (Y), preferably selected from the group consisting of phenylalanine (F) and tryptophan (W),
- when the amino acid residue corresponding to the amino acid residue at position 1220 in SEQ ID NO: 1 of the non-mutated αFAS is methionine (M) then it is substituted with an amino acid residue selected from the group consisting of phenylalanine (F), histidine (H), tryptophan (W) and tyrosine (Y), preferably selected from the group consisting of phenylalanine (F) and tryptophan (W).

3. The method according to claim 1 or claim 2, wherein the amino acid sequence of the non-mutated αFAS has at least 50% identity, or by order of increasing preference at least 51%, 55%, 60%, 65%, 70%, 75%, 82%, 85%, 90%, 92%, 95%, 96%, 97%, 98%, 99% or 100% identity, with the amino acid sequence of *Yarrowia lipolytica* αFAS of SEQ ID NO: 1.

4. The method according to claim 3, **characterized in that** the mutated αFAS is derived from the consensus amino acid sequence SEQ ID NO: 11, wherein the amino acid residue corresponding to the amino acid residue at position 1220 in SEQ ID NO: 1 is substituted with a larger steric hindrance amino acid residue, and, optionally, wherein the amino acid residue corresponding to the amino acid residue at position 1305 in SEQ ID NO: 1 is substituted with any other amino acid.

5. The method according to any one of claims 1 to 4, wherein the mutated αFAS is derived from αFAS selected from the group consisting of αFAS of SEQ ID NO: 1, 2, 3, 4, 5, 6, 7, 8 and 9.

6. The method according to any one of claims 1 to 5, wherein the amino acid residue at position 1305 of said αFAS is substituted with threonine (T).

7. The method according to any one of claims 1 to 6, wherein the ratio of fatty acids having a hydroxycarbon chain length consisting of 12 or 16 carbons (C12 or C16 fatty acids), to fatty acids having a hydroxycarbon chain consisting of 18 carbons (C18 fatty acids) is increased and/or the amount of Medium chain fatty acids having a hydroxycarbon chain length consisting of 12 carbons or 14 carbons (C12 or C14 fatty acids) is increased.

8. The method according to claim 7, wherein the fatty acid having a hydroxycarbon chain length consisting of 14 carbons is myristic acid (tetradecanoic acid) and the fatty acid having a hydroxycarbon chain length consisting of 12 carbons is lauric acid (Dodecanoic acid).

9. The method according to any one of claims 7 to 8, wherein the substitution of the amino acid residues corresponding to the amino acid residues at positions 1220 and/or 1305 of said αFAS is obtained by site-directed mutagenesis of the *αFAS* gene targeting the codon encoding the amino acid residues corresponding to the amino acid residues at said positions 1220 and/or 1305.

10. The method according to any one of claims 1 to 9, wherein the method further comprises inhibiting in said yeast strain the expression and/or the activity of the endogenous elongase proteins, in particular elongase 1 (ELO1; EC 2.3.1.199) having at least 50% identity or by order of increasing preference at least 55%, 60%, 65%, 70%, 75%, 80%, 85%, 90%, 92%, 95%, 96%, 97%, 98% or 99% identity, with the polypeptide of sequence SEQ ID NO: 12 (YALI_ELO1) and/or of the endogenous elongase 2 (ELO2; EC2.3.1.199) having at least 50% identity or by order of increasing preference at least 55%, 60%, 65%, 70%, 75%, 80%, 85%, 90%, 92%, 95%, 96%, 97%, 98% or 99% identity, with the polypeptide of sequence SEQ ID NO: 13 (YALI_ELO2).

11. The method according to any one of claims 1 to 9, wherein the method further comprises inhibiting in said yeast strain the expression and/or the activity of the endogenous fatty acid synthase subunit alpha (EC 2.3.1.86).

12. The method according to claims 10 or claim 11, wherein said inhibition is obtained by genetically transforming the yeast strain with a disruption cassette of the endogenous gene encoding said αFAS and of the endogenous gene encoding said elongase 1 or of the endogenous gene encoding said elongase 2.

13. A recombinant DNA expression cassette, comprising a polynucleotide encoding a mutated αFAS as defined in any one of claims 1 to 6 under the control of a suitable promoter.

14. A recombinant vector comprising a recombinant DNA expression cassette of claim 13.

15. A host cell comprising a recombinant DNA expression cassette of claim 13 or a recombinant vector of claim 14.

16. A mutant yeast strain able to produce an increased ratio of fatty acids having a hydroxycarbon chain length consisting of 16 carbons (C16 to fatty acids) to fatty acids having a hydroxycarbon chain consisting of 18 carbons (C18 fatty acids) and/or an increased amount of medium chain length fatty acids (C8-C15 fatty acids). compared to the parent oleaginous yeast strain from which said mutant yeast strain derives, wherein said mutant yeast strain expresses a mutated αFAS as defined in any one of claims 1 to 6, and optionally wherein the expression and/or the activity of the endogenous elongases 1 and/or 2 as defined in claims 9 or 10 is inhibited and/or the expression and/or the activity of the endogenous αFAS as defined in claim 11 is inhibited in said mutant yeast strain.

17. A mutant yeast strain according to claim 16, wherein it comprises, stably integrated in its genome, a recombinant DNA expression cassette of claim 13.

18. The method according to any one of claims 1 to 11, or the mutant yeast strain according to claims 16 or 17, wherein the yeast strain belongs to the genus selected from the group consisting of *Candida, Cryptoccocus, Lipomyces, Rhodosporidium, Rhodotorula, Trichosporon, Saccharomyces* and *Yarrowia.*

19. The method or the mutant yeast strain according to claim 18, wherein the oleaginous yeast strain is selected from the group consisting of a *Y. lipolytica, Y. galli, Y. yakushimensis, Y. alimentaria* and *Y. phangngensis* strain, preferably a *Y. lipolytica* strain.


SEQUENCE LISTING

<110> INSTITUT NATIONAL DE LA RECHERCHE AGRONOMIQUE
INSTITUT NATIONAL DES SCIENCES APPLIQUEES DE TOULOUSE
CENTRE NATIONAL DE LA RECHERCHE SCIENTIFIQUE
BORDES, Florence
RIGOUIN, Coraline
MARTY, Alain
GUEROULT, Marc
ANDRE, Isabelle
BARBE, Sophie
PERCHERON, Benjamin
CROUX, Christian
DABOUSSI, Fayza

<120> Mutant yeast strain capable of producing medium chain fatty acids

<130> F539/184EP

<160> 81

<170> PatentIn version 3.5

<210> 1
<211> 1850
<212> PRT
<213> Yarrowia lipolytica

<400> 1

Met His Pro Glu Val Glu Gln Glu Leu Ala His Val Leu Leu Thr Glu
1               5                   10                  15

Leu Leu Ala Tyr Gln Phe Ala Ser Pro Val Arg Trp Ile Glu Thr Gln
            20                  25                  30

Asp Val Leu Phe Lys Gln Phe Asn Val Glu Arg Val Val Glu Val Gly
        35                  40                  45

Pro Ser Pro Thr Leu Ala Gly Met Ala Gln Arg Thr Leu Lys Ser Lys
    50                  55                  60

Tyr Glu Ser Tyr Asp Ala Ala Leu Ser Leu Gln Arg Glu Ile Leu Cys
65                  70                  75                  80

Tyr Ser Lys Asp Gln Lys Asp Ile Tyr Tyr Leu Ala Asp Glu Ala Asp
                85                  90                  95

Glu Ala Pro Ala Pro Ala Ala Gly Gly Asp Ala Pro Ala Ala Pro Ala
            100                 105                 110

Ala Ala Ala Pro Ala Ala Ala Ala Ala Pro Ala Ala Ala Ala Ala Pro
        115                 120                 125

Ser Gly Pro Val Ala Lys Val Glu Asp Ala Pro Val Lys Ala Gln Glu

130                 135                 140

Ile Leu His Ala Leu Val Ala His Lys Leu Lys Lys Thr Pro Glu Gln
145                 150                 155                 160

Val Pro Leu Ser Lys Ala Ile Lys Asp Leu Val Gly Gly Lys Ser Thr
                165                 170                 175

Ile Gln Asn Glu Ile Leu Gly Asp Leu Gly Lys Glu Phe Gly Ala Thr
            180                 185                 190

Pro Glu Lys Pro Glu Asp Thr Pro Leu Gly Glu Leu Ala Glu Ser Phe
        195                 200                 205

Gln Ala Ser Phe Asp Gly Lys Leu Gly Lys Gln Ser Ser Ser Leu Ile
    210                 215                 220

Ala Arg Leu Met Ser Ser Lys Met Pro Gly Gly Phe Ser Leu Thr Ser
225                 230                 235                 240

Ala Arg Ser Tyr Leu Asp Ser Arg Trp Gly Leu Ala Ala Gly Arg Gln
                245                 250                 255

Asp Ser Val Leu Leu Val Ala Leu Met Asn Glu Pro Lys Asn Arg Leu
            260                 265                 270

Gly Ser Glu Ala Glu Ala Lys Ala Tyr Leu Asp Glu Gln Thr Gln Lys
        275                 280                 285

Tyr Ala Ala Ser Ala Gly Leu Asn Leu Ser Ala Pro Ala Gly Gly Ala
    290                 295                 300

Glu Gly Gly Asn Gly Gly Gly Ala Val Ile Asp Ser Ala Ala Phe Asp
305                 310                 315                 320

Ala Leu Thr Lys Asp Gln Arg Tyr Leu Val Gln Gln Gln Leu Glu Leu
                325                 330                 335

Phe Ala Asn Tyr Leu Lys Gln Asp Leu Arg Gln Gly Ser Lys Val Ala
            340                 345                 350

Ala Ala Gln Lys Glu Ala Met Asp Ile Leu Gln Ala Glu Leu Asp Leu
        355                 360                 365

Trp Asn Ser Glu His Gly Glu Val Tyr Ala Glu Gly Ile Lys Pro Ala
    370                 375                 380

Phe Ser Ala Leu Lys Ala Arg Val Tyr Asp Ser Tyr Trp Asn Trp Ala
385                 390                 395                 400

Arg Gln Asp Ser Leu Ser Met Tyr Phe Asp Ile Val Phe Gly Arg Leu
                405                 410                 415

Ser Thr Val Asp Arg Glu Ile Met Ala Lys Cys Ile His Leu Met Asn
            420                 425                 430

Arg Thr Asn His Asn Leu Ile Asp Tyr Met Gln Tyr His Met Asp His
        435                 440                 445

Val Pro Val His Lys Gly Ala Thr Tyr Glu Leu Ala Lys Gln Leu Gly
    450                 455                 460

Leu Gln Leu Leu Glu Asn Cys Lys Glu Thr Leu Thr Glu Ala Pro Val
465                 470                 475                 480

Tyr Lys Asp Val Ser Tyr Pro Thr Gly Pro Gln Thr Thr Ile Asp Val
                485                 490                 495

Lys Gly Asn Ile Val Tyr Asn Glu Val Pro Arg Pro Asn Val Arg Lys
            500                 505                 510

Leu Glu Gln Tyr Val His Glu Met Ala Cys Gly Gly Glu Leu Thr Lys
        515                 520                 525

Asp Pro Ser Phe Val Gly Glu Gly Val Gln Gly Glu Leu Lys Lys Leu
    530                 535                 540

Tyr Ser Gln Ile Ser Ala Leu Ala Lys Thr Gln Thr Gly Ser Thr Leu
545                 550                 555                 560

Asp Ile Glu Ala Leu Tyr Ser Asp Leu Val Ala Lys Ile Ser Gln Ala
                565                 570                 575

Glu Asp Ala Ser Lys Pro Val Val Glu Asn Lys Ala Val Ser Ala Ser
            580                 585                 590

Ile Thr Pro Gly Thr Leu Pro Phe Leu His Ile Lys Lys Lys Thr Glu
        595                 600                 605

Leu Gly Ala Trp Asn Tyr Asp Ser Glu Thr Thr Ala Thr Tyr Leu Asp
    610                 615                 620

Gly Leu Glu Val Ala Ala Arg Asp Gly Leu Thr Phe Gln Gly Lys Thr
625                 630                 635                 640

Ala Leu Ile Thr Gly Ala Gly Ala Gly Ser Ile Gly Ala Ser Ile Leu
                645                 650                 655

Gln Gly Leu Ile Ser Gly Gly Cys Lys Val Ile Val Thr Thr Ser Arg
            660                 665                 670

Tyr Ser Arg Lys Val Thr Glu Tyr Tyr Gln Ser Leu Tyr Thr Lys Phe
        675                 680                 685

Gly Ala Lys Gly Ser Thr Leu Ile Val Val Pro Phe Asn Gln Gly Ser
    690                 695                 700

Lys Lys Asp Val Asp Glu Leu Val Ser Phe Ile Tyr Asn Asp Pro Lys
705                 710                 715                 720

Asn Gly Gly Leu Gly Trp Asp Leu Asp Phe Val Val Pro Phe Ala Ala
                725                 730                 735

Leu Pro Glu Asn Gly Ile Glu Leu Glu His Ile Asp Ser Lys Ser Glu
            740                 745                 750

Leu Ala His Arg Ile Met Leu Thr Asn Leu Leu Arg Leu Leu Gly Asn
        755                 760                 765

Val Lys Lys Gln Lys Val Ala His Ser Tyr Glu Thr Arg Pro Ala Gln
    770                 775                 780

Val Met Leu Pro Leu Ser Pro Asn His Gly Asn Phe Gly Ser Asp Gly
785                 790                 795                 800

Leu Tyr Ser Glu Ser Lys Ile Ser Leu Glu Thr Leu Phe Asn Arg Trp
                805                 810                 815

His Thr Glu Ser Trp Gly Ser Tyr Leu Thr Ile Val Gly Val Val Ile
            820                 825                 830

Gly Trp Thr Arg Gly Thr Gly Leu Met Ser Ala Asn Asn Ile Thr Ala
        835                 840                 845

Glu Gly Leu Glu Gln Leu Gly Val Arg Thr Phe Ser Gln Thr Glu Met
    850                 855                 860

Ala Phe Ser Ile Met Gly Leu Met Thr Lys Asp Ile Val Arg Leu Ala
865                 870                 875                 880

Gln Asn Ser Pro Val Trp Ala Asp Leu Asn Gly Gly Phe Gln Tyr Ile
                885                 890                 895

Pro Asp Leu Lys Gly Val Val Gly Lys Ile Arg Arg Asp Ile Val Glu
            900                 905                 910

Thr Ser Glu Ile Arg Arg Ala Val Ala Gln Glu Thr Ala Ile Glu Gln
        915                 920                 925

Lys Val Val Asn Gly Pro His Ala Asp Leu Pro Tyr Gln Lys Val Glu
    930                 935                 940

Val Lys Pro Arg Ala Asn Leu Lys Phe Asp Phe Pro Thr Leu Lys Ser
945                 950                 955                 960

Tyr Ala Glu Val Lys Glu Leu Ser Pro Ala Gly Asp Ala Leu Glu Gly
                965                 970                 975

Leu Leu Asp Leu Ser Ser Val Ile Val Val Thr Gly Phe Ala Glu Val
            980                 985                 990

Gly Pro Trp Gly Asn Ala Arg Thr Arg Trp Asp Met Glu Ala Asn Gly
        995                 1000                1005

Val Phe Ser Leu Glu Gly Ala Ile Glu Met Ala Trp Ile Met Gly
    1010                1015                1020

Leu Ile Lys His His Asn Gly Pro Leu Pro Gly Met Pro Gln Tyr
    1025                1030                1035

Ser Gly Trp Ile Asp Thr Lys Thr Lys Gln Pro Val Asp Asp Arg
    1040                1045                1050

Asp Ile Lys Thr Lys Tyr Glu Asp Tyr Leu Leu Glu His Ala Gly
    1055                1060                1065

Ile Arg Leu Ile Glu Pro Glu Leu Phe His Gly Tyr Asn Pro Lys
    1070                1075                1080

Lys Lys Thr Phe Leu Gln Glu Val Ile Val Glu His Asp Leu Glu
    1085                1090                1095

Pro Phe Glu Ala Ser Lys Glu Ser Ala Glu Gln Phe Ala Leu Glu
    1100                1105                1110

Gln Gly Ala Asn Val Glu Ile Phe Ala Val Pro Glu Ser Asp Gln
    1115                1120                1125

Trp Thr Val Arg Leu Leu Lys Gly Ala Lys Leu Leu Ile Pro Lys

1130                1135                1140

Ala Leu Lys Phe Asp Arg Leu Val Ala Gly Gln Ile Pro Thr Gly
    1145                1150                1155

Trp Asp Ala Arg Arg Tyr Gly Ile Pro Glu Asp Ile Cys Asp Gln
    1160                1165                1170

Val Asp Pro Ile Thr Leu Tyr Ala Leu Val Ser Thr Val Glu Ala
    1175                1180                1185

Leu Leu Ala Ser Gly Ile Thr Asp Pro Tyr Glu Phe Tyr Lys Tyr
    1190                1195                1200

Val His Val Ser Glu Val Gly Asn Cys Ser Gly Ser Gly Met Gly
    1205                1210                1215

Gly Ile Thr Ala Leu Arg Gly Met Phe Lys Asp Arg Phe Met Asp
    1220                1225                1230

Lys Pro Val Gln Asn Asp Ile Leu Gln Glu Ser Phe Ile Asn Thr
    1235                1240                1245

Met Ser Ala Trp Val Asn Met Leu Leu Leu Ser Ser Ser Gly Pro
    1250                1255                1260

Ile Lys Thr Pro Val Gly Ala Cys Ala Thr Ala Val Glu Ser Val
    1265                1270                1275

Asp Ile Gly Cys Glu Thr Ile Leu Ser Gly Lys Ala Arg Ile Cys
    1280                1285                1290

Leu Val Gly Gly Tyr Asp Asp Phe Gln Glu Glu Ser Ser Gln Glu
    1295                1300                1305

Phe Ala Asn Met Asn Ala Thr Ser Asn Ala Glu Thr Glu Ile Thr
    1310                1315                1320

His Gly Arg Thr Pro Ala Glu Met Ser Arg Pro Ile Thr Ser Thr
    1325                1330                1335

Arg Ala Gly Phe Met Glu Ala Gln Gly Ala Gly Thr Gln Val Leu
    1340                1345                1350

Met Ala Ala Asp Leu Ala Ile Ala Met Gly Val Pro Ile Tyr Cys
    1355                1360                1365

Ile Val Gly Tyr Val Asn Thr Ala Thr Asp Lys Ile Gly Arg Ser
    1370                1375                1380

Val Pro Ala Pro Gly Lys Gly Ile Leu Thr Thr Ala Arg Glu His
    1385                1390                1395

Gln Thr Leu Lys His Ala Asn Pro Leu Leu Asn Ile Lys Tyr Arg
    1400                1405                1410

Lys Arg Gln Leu Asp Ser Arg Leu Arg Asp Ile Lys Arg Trp Ala
    1415                1420                1425

Glu Gly Glu Met Glu Ala Ile Asp Ile Glu Leu Asp Asp Val Ser
    1430                1435                1440

Asp Ala Asp Lys Glu Ser Phe Ile Gln Glu Arg Ser Ala His Ile
    1445                1450                1455

Gln Ser Gln Ser Asp Arg Met Ile Arg Glu Ala Lys Asn Ser Trp
    1460                1465                1470

Gly Asn Ala Phe Phe Lys Gln Asp Ala Arg Ile Ser Pro Ile Arg
    1475                1480                1485

Gly Ala Leu Ala Thr Tyr Gly Leu Thr Ile Asp Asp Ile Ser Val
    1490                1495                1500

Ala Ser Phe His Gly Thr Ser Thr Lys Ala Asn Glu Lys Asn Glu
    1505                1510                1515

Thr Thr Thr Val Asn Ala Met Leu Glu His Leu Gly Arg Thr Arg
    1520                1525                1530

Gly Asn Pro Val Tyr Gly Ile Phe Gln Lys Tyr Leu Thr Gly His
    1535                1540                1545

Pro Lys Gly Ala Ala Gly Ala Trp Met Leu Asn Gly Ala Ile Gln
    1550                1555                1560

Cys Leu Asn Ser Gly Ile Ile Pro Gly Asn Arg Asn Ala Asp Asn
    1565                1570                1575

Val Asp Ala Tyr Phe Glu Gln Cys Gln His Val Val Phe Pro Ser
    1580                1585                1590

Arg Ser Leu Gln Thr Asp Gly Leu Lys Ala Ala Ser Val Thr Ser
    1595                1600                1605

Phe Gly Phe Gly Gln Lys Gly Ala Gln Ala Ile Val Ile His Pro
    1610                1615                1620

Asp Tyr Leu Tyr Ala Ala Leu Thr Pro Ser Glu Tyr Ser Glu Tyr
    1625                1630                1635

Thr Thr Arg Val Ala Gln Arg Tyr Lys Lys Ala Tyr Arg Tyr Tyr
    1640                1645                1650

His Asn Ala Ile Ala Glu Glu Ser Met Phe Gln Ala Lys Asp Lys
    1655                1660                1665

Ala Pro Tyr Ser Ala Glu Leu Glu Gln Glu Val Tyr Leu Asp Pro
    1670                1675                1680

Leu Val Arg Val His Gln Asn Glu Asp Thr Glu Gln Tyr Ser Phe
    1685                1690                1695

Asn Ala Lys Asp Leu Ala Ala Ser Ala Phe Val Lys Asn Ser His
    1700                1705                1710

Lys Asp Thr Ala Lys Val Leu Ala Asn Leu Thr Ser Gln Val Ser
    1715                1720                1725

Gly Ser Gly Lys Asn Val Gly Val Asp Val Glu Ala Ile Ser Ala
    1730                1735                1740

Ile Asn Ile Asp Asn Asp Thr Phe Leu Asp Arg Asn Phe Thr Ala
    1745                1750                1755

Asn Glu Gln Ala Tyr Cys Phe Lys Ala Pro Ser Pro Gln Ser Ser
    1760                1765                1770

Phe Ala Gly Thr Trp Ser Ala Lys Glu Ala Val Phe Lys Ser Leu
    1775                1780                1785

Gly Val Lys Ser Gln Gly Gly Gly Ala Glu Leu Lys Ser Ile Glu
    1790                1795                1800

Ile Thr Arg Asp Gly Asn Gly Ala Pro Val Val Val Leu His Gly
    1805                1810                1815

Ala Ala Lys Asp Ala Ala Ala Ser Lys Gly Ile Ser Thr Val Lys
    1820                1825                1830

Val Ser Ile Ser His Asp Asp Ser Gln Ala Val Ala Val Ala Val
    1835                1840                1845

Ala Glu
    1850

<210>  2
<211>  2716
<212>  PRT
<213>  Rhodotorula sp. JG-1b

<400>  2

Met Tyr Asp Gln Pro Glu Gln Asp Lys Pro Ala Leu Pro Leu Glu Phe
1               5                   10                  15

Lys Tyr Lys Tyr Asp Pro Ser Thr Pro Tyr Ala Pro Ile His Glu Ile
            20                  25                  30

Val Glu Asp Arg Asn Thr Arg Ile Lys Gln His Tyr Trp Asp Leu Trp
        35                  40                  45

Asn Leu Gly Gly Lys Ser Gly Glu Gln Phe Thr Gln Leu Lys Val Thr
    50                  55                  60

Asp Glu Phe Val Asp Asp Gly Ala Thr Ile Ser Ala Asp Glu Val Glu
65                  70                  75                  80

Ala Phe Cys Arg Val Val Gly Ile Glu Gly Glu Ala Tyr Lys Lys Ser
                85                  90                  95

Tyr Lys His Gly Met Gln Ile Pro Leu Asp Phe Ala Ile Lys Met Gly
            100                 105                 110

Trp Arg Ser Ile Met Lys Pro Ile Phe Pro Pro Ala Ile Asp Gly Asp
        115                 120                 125

Leu Leu Lys Leu Val His Leu Ser Asn Gly Phe Arg Val Phe Asp Asp
    130                 135                 140

Ala Pro Ala Leu Lys Val Gly Asp Val Val Lys Thr Val Ser Arg Ile
145                 150                 155                 160

Glu Ser Ile Thr Asn Ser Asp Thr Gly Lys Thr Val Thr Val Arg Gly
                165                 170                 175

Val Leu Tyr Leu Thr Ser Ser Ala Asp Ala Lys Gly Lys Glu Ala Ser
            180                 185                 190

Ser Asp Asp Gln Ile Pro Ile Ile Glu Val Thr Ser Ser Phe Phe Tyr
        195                 200                 205

Arg Gly Lys Phe Thr Asp Phe Ser Gln Thr Phe Ser Arg Val Thr Gln
    210                 215                 220

Pro Thr Tyr Ser Val Pro Ile Asn Thr Pro Gln Ala Leu Ala Val Leu
225                 230                 235                 240

Gln Ala Lys Glu Trp Phe Gln Trp Asp Asp Asp Ser Lys Pro Leu Glu
                245                 250                 255

Val Gly Thr Thr Leu Gln Phe Lys Leu Glu Ser His Tyr Thr Phe His
            260                 265                 270

Asp Lys Ser Ser Tyr Thr Met Met Asn Thr Thr Gly Gly Ala Tyr Ile
        275                 280                 285

Ile Thr Pro Glu Leu Lys Leu Ala Val Lys Val Ala Thr Ile Asp Tyr
    290                 295                 300

Thr Ser Glu Gly Glu Gly Val Val Ile Gly Asp Ala Cys Ile Glu Tyr
305                 310                 315                 320

Leu Lys Arg His Gly Thr Ala Leu Asn Gln Pro Asn Val Leu Glu Asn
                325                 330                 335

Gly Gly Tyr Thr Leu Thr Lys Ser Gly Gln Cys Thr Phe Thr Thr Pro
            340                 345                 350

Ala Ser Asn Leu Asp Tyr Ser Leu Thr Ser Gly Asp Thr Asn Pro Ile
        355                 360                 365

His Thr Asn Pro Tyr Phe Ala Ser Leu Ala Ser Leu Pro Gly Thr Ile
    370                 375                 380

Thr His Gly Met His Thr Ser Ala Arg Thr Arg Lys Tyr Val Glu Gln
385                 390                 395                 400

Val Val Ala Asp Asn Val Gly Pro Arg Val Arg Lys Tyr Glu Val Ser
                405                 410                 415

Phe Thr Ala Met Cys Leu Pro Ser Arg Lys Met Glu Val Arg Leu Lys
            420                 425                 430

His Val Gly Met Thr Ser Asn Gly Asp Arg Leu Ile Lys Val Glu Thr
        435                 440                 445

Val Asp Val Glu Ala Gly Asn Val Val Leu Thr Gly Thr Ala Glu Val

450                 455                 460

Ala Gln Ala Pro Thr Ala Tyr Val Phe Thr Gly Gln Gly Ser Gln Glu
465                 470                 475                 480

Pro Gly Met Gly Met Glu Leu Tyr Asn Ser Ser Pro Val Ala Arg Ala
                485                 490                 495

Val Trp Asp Glu Ala Asp Arg His Leu Gly Glu Val Tyr Gly Phe Ser
            500                 505                 510

Ile Leu Glu Ile Val Arg Asn Asn Pro Lys Glu Lys Thr Val His Phe
        515                 520                 525

Gly Gly Leu Lys Gly Gln Ala Thr Arg Gln Lys Tyr Met Asp Met Thr
    530                 535                 540

Tyr Thr Ser Ala Asp Ala Glu Gly His Val Arg Thr Leu Pro Leu Phe
545                 550                 555                 560

Gly Asp Ile Asp Leu Arg Thr Ser Arg Tyr Thr Phe Gln Ser Pro Thr
                565                 570                 575

Gly Leu Leu Tyr Ala Thr Gln Phe Ala Gln Ile Ala Leu Val Val Thr
            580                 585                 590

Glu Lys Ala Ala Phe Glu Asp Met Arg Ala Lys Gly Leu Phe Gln Lys
        595                 600                 605

Asn Cys Met Phe Ala Gly His Ser Leu Gly Glu Tyr Ser Ala Leu Ala
    610                 615                 620

Ser Ile Ala Asp Ile Leu Pro Ile Ser Ala Leu Val Asp Val Val Phe
625                 630                 635                 640

Tyr Arg Gly Ile Thr Met Gln Arg Ala Val Glu Arg Asp His Leu Asn
                645                 650                 655

Arg Ser Ser Tyr Gly Met Val Ala Ala Asn Pro Ser Arg Ile Gly Lys
            660                 665                 670

Thr Phe Gly Asp Ala Ala Leu Arg Glu Val Val Glu Thr Ile Ser Arg
        675                 680                 685

Arg Gly Asn Ile Leu Ile Glu Val Val Asn Tyr Asn Val Glu Gly Gln
    690                 695                 700

Gln Tyr Val Val Ala Gly His Leu Val Ala Leu Gln Ser Leu Thr Asn
705                 710                 715                 720

Val Leu Asn Phe Leu Lys Val Gln Lys Ile Asp Leu Val Lys Leu Thr
                725                 730                 735

Glu Thr Met Ser Ile Glu Gln Val Lys Glu His Leu Cys Glu Ile Val
            740                 745                 750

Asp Glu Cys Val Gln Lys Ala Arg Asp Leu Gln Gly Lys Thr Gly Phe
        755                 760                 765

Ile Thr Leu Glu Arg Gly Phe Ala Thr Ile Pro Leu Pro Gly Ile Asp
    770                 775                 780

Val Pro Phe His Ser Arg Tyr Leu Trp Ala Gly Val Met Pro Phe Arg
785                 790                 795                 800

Thr Tyr Leu Ser Lys Lys Val Asn Pro Ala His Phe Asn Ala Asp Leu
                805                 810                 815

Leu Val Gly Arg Tyr Ile Pro Asn Leu Thr Ala Val Pro Tyr Glu Val
            820                 825                 830

Asn Lys Glu Tyr Ala Glu Arg Ile His Thr Gln Thr Ser Ser Pro Arg
        835                 840                 845

Leu Asn Lys Ile Leu Ser Ser Trp Asp Glu Glu Gln Trp Gly Ala Pro
    850                 855                 860

Glu Asn Arg Asn Lys Leu Gly Tyr Ala Ile Leu Ile Glu Leu Leu Ala
865                 870                 875                 880

Tyr Gln Phe Ala Ser Pro Val Arg Trp Ile Glu Thr Gln Asp Ile Leu
                885                 890                 895

Phe Lys Asp Phe Lys Phe Glu Arg Leu Val Glu Leu Gly Pro Ser Pro
            900                 905                 910

Thr Leu Thr Gly Met Ala Ser Arg Thr Gln Lys Leu Lys Tyr Asp Ala
        915                 920                 925

His Asp Ser Ala Leu Gly Ile Lys Arg Ala Ile Tyr Cys Ile Ala Lys
    930                 935                 940

Asn Gln Lys Glu Ile Tyr Tyr Gln Asn Asp Asp Val Thr Asp Asp Ala
945                 950                 955                 960

Pro Ala Pro Ala Ala Ala Ala Pro Ser Ala Pro Ala Pro Lys Ala Ala
                965                 970                 975

Ala Ala Pro Val Ala Ala Ala Pro Pro Pro Pro Ala Pro Val Ala Ala
            980                 985                 990

Ala Pro Ala Ala Ala Val Ala Asp  Glu Pro Leu Lys Ala  Ala Val Asp
        995                 1000                 1005

Thr Leu  Arg Ala Ile Ile Ala  Gln Lys Leu Lys Lys  Pro Ile Ala
    1010                 1015                 1020

Glu Val  Pro Leu Asn Lys Ser  Ile Lys Asp Leu Val  Gly Gly Lys
    1025                 1030                 1035

Ser Thr  Leu Gln Asn Glu Ile  Leu Gly Asp Leu Gln  Gly Glu Phe
    1040                 1045                 1050

Ala Ser  Ala Pro Glu Lys Gly  Glu Glu Met Pro Leu  Ser Glu Leu
    1055                 1060                 1065

Gly Ala  Ala Leu Asn Gln Gly  Tyr Pro Gly Ser Leu  Gly Lys Tyr
    1070                 1075                 1080

Thr Thr  Gly Leu Val Ala Arg  Met Met Gly Gly Lys  Met Pro Gly
    1085                 1090                 1095

Gly Phe  Gly Leu Ser Ala Ala  Lys Ala His Leu Ala  Lys Ala His
    1100                 1105                 1110

Gly Leu  Gly Pro Gly Arg Thr  Asp Gly Ala Leu Leu  Val Ala Leu
    1115                 1120                 1125

Thr Met  Glu Pro Glu Lys Arg  Leu Gly Ser Glu Ala  Asp Ala Lys
    1130                 1135                 1140

Ala Trp  Leu Asp Ser Val Ala  Ser Ala Tyr Ala Ala  Gln Ala Gly
    1145                 1150                 1155

Ile Ser  Leu Gly Ala Ala Gly  Gly Gly Gly Gly Gly  Gly Ala Val
    1160                 1165                 1170

Gly Gly  Gly Met Met Ile Asn  Thr Glu Gln Leu Asp  Lys Leu Gln
    1175                 1180                 1185

Glu Lys  Gln Asp Asn Phe Val  Ser Gln Gln Val Asp  Leu Phe Leu
    1190                 1195                 1200

Arg Tyr Leu Gly Lys Asp Ser Arg Glu Gly His Arg Leu Ala Asp
    1205                1210                1215

Leu Gln Lys Ala Glu Val Ala Ser Leu Gln Asp Lys Leu Asp Ala
    1220                1225                1230

Ile Ser Arg Glu His Gly Asp Ala Tyr Val Gln Gly Ile Gln Pro
    1235                1240                1245

Val Phe Asn Pro Leu Lys Ala Arg His Phe Asn Ser Ser Trp Asn
    1250                1255                1260

Trp Val Arg Gln Asp Ala Leu Met Met Trp Met Asp Ile Leu Phe
    1265                1270                1275

Gly Arg Leu Thr Thr Val Asp Arg Asp Ile Thr Ala Arg Cys Leu
    1280                1285                1290

Val Ile Met Asn Arg Ala Asp Pro Ala Leu Leu Asp Tyr Met Gln
    1295                1300                1305

Tyr Val Ile Asp Asn Thr Pro Thr Glu Arg Gly Glu Thr Tyr Val
    1310                1315                1320

Leu Ala Lys Ala Phe Gly Gln Thr Leu Leu Asp Asn Cys Arg Glu
    1325                1330                1335

Met Val Gly Gln Ala Pro Leu Tyr Lys Asp Val Thr Phe Pro Thr
    1340                1345                1350

Ala Pro Lys Thr Thr Val Thr Val Lys Gly Glu Ile Leu Ser Glu
    1355                1360                1365

Glu Val Asn Arg Pro Gly Val Ser Arg Leu Glu Lys Tyr Val Ala
    1370                1375                1380

Glu Met Ala Ala Gly Ser Lys Val Thr Val Gln Ser Val Asn Leu
    1385                1390                1395

Asp Lys Val Gln Asp Gln Val Glu Lys Leu Tyr Lys Leu Val Lys
    1400                1405                1410

Ser Gln Pro Gln Ile Ser Lys Thr His Met Gln Ser Ile Lys Ser
    1415                1420                1425

Leu Tyr Thr Glu Val Val Arg Gly Leu Gly Lys Asp Ala Pro Pro

1430                1435                1440

Pro Thr Thr His Lys Thr Gly Thr Arg Ala Arg Arg Pro Ser Ser
    1445                1450                1455

Gln Phe Leu Arg Pro Ala Ala Val Gln Glu Ala Thr Tyr Leu Pro
    1460                1465                1470

Glu Asp Lys Val Pro Leu Leu His Leu Lys Arg Lys Ile Gly Asn
    1475                1480                1485

Glu Trp Gln Tyr Ser Ser Lys Leu Thr Ser Leu Tyr Leu Asp Ile
    1490                1495                1500

Leu Lys Glu Ile Ala Thr Ser Gly Val Thr Phe Glu His Lys Asn
    1505                1510                1515

Ala Leu Met Thr Gly Val Gly Lys Gly Ser Ile Gly Val Glu Ile
    1520                1525                1530

Val Lys Gly Leu Leu Ala Gly Gly Ala Arg Val Val Val Thr Thr
    1535                1540                1545

Ser Arg Tyr Ser Arg Ser Thr Val Glu Tyr Tyr Gln Ala Ile Tyr
    1550                1555                1560

Gln Glu Val Gly Ala Lys Gly Ser Ser Leu Thr Val Val Pro Phe
    1565                1570                1575

Asn Gln Gly Ser Lys Gln Asp Val Glu Ala Leu Val Asp Tyr Ile
    1580                1585                1590

Tyr Asn Lys Glu Lys Gly Leu Gly Met Asp Leu Asp Tyr Ile Leu
    1595                1600                1605

Pro Phe Ala Ala Leu Pro Glu Asn Gly Arg Glu Ile Asp Gly Ile
    1610                1615                1620

Asp Asp Arg Ser Glu Leu Ala His Arg Ile Met Leu Thr Asn Val
    1625                1630                1635

Leu Arg Leu Leu Gly Ala Val Lys Ser Lys Lys Ala Ala Leu Lys
    1640                1645                1650

Leu Thr Thr Arg Pro Thr Glu Val Val Leu Pro Leu Ser Pro Asn
    1655                1660                1665

His Gly Leu Phe Gly Asn Asp Gly Leu Tyr Ser Glu Ser Lys Ile
    1670                1675                1680

Ser Leu Glu Thr Leu Phe Asn Arg Trp Ser Ser Glu Ser Trp Gly
    1685                1690                1695

Glu Tyr Leu Cys Ile Ala Gly Ala Ile Ile Gly Trp Thr Arg Gly
    1700                1705                1710

Thr Gly Leu Met Ser Ala Thr Asn Ser Val Ala Glu Gly Ile Glu
    1715                1720                1725

Ala His Gly Cys Arg Thr Phe Ser Ala Lys Glu Met Ala Phe Asn
    1730                1735                1740

Ile Leu Gly Leu Met His Pro Leu Val Phe Asp Val Ala Gln Ile
    1745                1750                1755

Glu Pro Val Trp Ala Asp Leu Asn Gly Gly Met Asp Lys Leu Pro
    1760                1765                1770

Asp Leu Ala Thr Leu Thr Thr Asp Ile Arg Thr Lys Leu Asn Leu
    1775                1780                1785

Thr Ala Asn Asn Arg Arg Ala Val Ala Lys Asp His Ser Leu Asp
    1790                1795                1800

Tyr Lys Val Gln His Gly Pro Ala Met Glu Gln Ile His Gln Gln
    1805                1810                1815

Val Lys Val Ala Pro Arg Ala Asn Phe Ser Leu Pro Phe Pro Glu
    1820                1825                1830

Leu Lys Pro Ile Asp Ala Thr Ser Glu Leu Ala Lys Leu Arg Gly
    1835                1840                1845

Leu Ile Asp Leu Glu Gln Val Val Val Leu Thr Gly Tyr Ala Glu
    1850                1855                1860

Val Gly Pro Phe Gly Ser Ser Arg Thr Arg Trp Glu Met Glu Ala
    1865                1870                1875

Asn Gly Thr Phe Ser Ile Gln Gly Thr Leu Glu Leu Ala Tyr Val
    1880                1885                1890

Met Gly Leu Ile Lys His Phe Glu Gly Arg Leu Lys Asp Gly Thr
    1895                1900                1905

Leu Tyr Val Gly Trp Val Asp Ala Lys Thr Asn Glu Pro Leu Asp
    1910                1915                1920

Asp Lys Asp Val Lys Ala Ala Tyr Glu Lys His Ile Leu Ala His
    1925                1930                1935

Thr Gly Ile Arg Leu Ile Glu Pro Glu Ile Phe Asn Gly Tyr Asp
    1940                1945                1950

Pro Lys Arg Lys Gly Phe Thr Gln Glu Ile Glu Ile Gln His Asp
    1955                1960                1965

Leu Glu Pro Ile Glu Ala Ser Glu Glu Asp Ala Ala Arg Phe Lys
    1970                1975                1980

Arg Glu His Gly Val Leu Val Asp Val Tyr Thr Glu Asp Gly Ser
    1985                1990                1995

Lys Phe Phe Val Lys Phe Lys Lys Gly Ala Lys Leu Asn Ile Pro
    2000                2005                2010

Lys Ala Val Ala Phe Asp Arg Leu Val Ala Gly Gln Ile Pro Thr
    2015                2020                2025

Gly Trp Ser His Lys Ala Phe Gly Ile Pro Asp Asp Ile Ala Ser
    2030                2035                2040

Gln Val Asp Arg Thr Ser Leu Trp Ala Leu Val Ser Val Ala Glu
    2045                2050                2055

Ala Leu Met Met Ala Gly Ile Thr Asp Pro Tyr Glu Leu Tyr Lys
    2060                2065                2070

Phe Val His Pro Ser Glu Val Gly Ser Ser Leu Gly Ser Gly Met
    2075                2080                2085

Gly Gly Ile Thr Ser Ile Ser Lys Met Phe Arg Asp Arg Arg Glu
    2090                2095                2100

Glu Lys Asp Val Gln Lys Asp Ile Leu Gln Glu Thr Phe Ile Asn
    2105                2110                2115

Thr Val Ala Gly Trp Val Asn Leu Leu Leu Leu Ser Ser Ser Gly
    2120                2125                2130

Pro Ile Lys Val Pro Val Gly Ala Cys Ala Thr Ala Leu Gln Ser
    2135                2140                2145

Val Glu Ile Ala Cys Asp Thr Ile Leu Ser Gly Lys Ala Lys Ile
    2150                2155                2160

Met Val Ala Gly Gly Tyr Asp Asp Phe Ser Glu Glu Gly Ser Tyr
    2165                2170                2175

Glu Phe Ala Asn Met Lys Ala Thr Ser Asn Ser Glu Thr Glu Phe
    2180                2185                2190

Ala Ala Gly Arg Glu Pro Asn Glu Met Ser Arg Pro Thr Thr Ser
    2195                2200                2205

Thr Arg Ala Gly Phe Met Glu Ser Met Gly Cys Gly Ala Gln Val
    2210                2215                2220

Leu Met Ser Ala Lys Thr Ala Ile Glu Met Gly Ala Thr Ile Tyr
    2225                2230                2235

Gly Val Val Ala Tyr Thr Ala Thr Ala Thr Asp Lys Ala Gly Arg
    2240                2245                2250

Ser Ile Pro Ala Pro Gly Arg Gly Val Val Gly Thr Ala Arg Glu
    2255                2260                2265

Leu Ser Ser Lys Tyr Pro Ser Pro Ile Leu Asp Val Thr Tyr Arg
    2270                2275                2280

Arg Arg Gln Leu Glu Phe Arg Arg Arg Gln Ile Ser Gln Trp Leu
    2285                2290                2295

Glu Asn Glu Thr Glu Leu Leu Gln Met Glu Ile Glu Ser Arg Ser
    2300                2305                2310

Asp Ala Asp Lys Leu Pro Glu Asp Tyr Val Ala Glu Arg Phe Ala
    2315                2320                2325

Ser Ile Glu Arg Glu Ala Lys Arg Gln Glu Ser Glu Ala Leu Ala
    2330                2335                2340

Thr Tyr Gly Met Leu Ala Gly Gln Asp Pro Ser Ile Ala Pro Leu
    2345                2350                2355

Arg Arg Ala Leu Ala Val Trp Gly Leu Thr Ile Asp Asp Val Gly
    2360                2365                2370

Val Ala Ser Phe His Gly Thr Ser Thr Val Ala Asn Asp Lys Asn

2375                2380                2385

Glu Ser Asn Ala Tyr Asn Glu Gln Phe Arg His Leu Gly Arg Ala
    2390                2395                2400

Lys Gly Asn Ala Cys Pro Val Ile Ala Gln Lys Trp Leu Thr Gly
    2405                2410                2415

His Pro Lys Gly Gly Ala Ala Ala Trp Met Leu Asn Gly Met Ala
    2420                2425                2430

Gln Val Ile Leu Ser Gly Leu Val Pro Gly Asn Arg Asn Ala Asp
    2435                2440                2445

Asn Ile Gly Glu Glu Leu Arg Ala Phe Glu Tyr Leu Leu Tyr Pro
    2450                2455                2460

Ser Lys Ser Ile Gln Thr Asp Gly Ile Lys Ala Gly Leu Leu Thr
    2465                2470                2475

Ser Phe Gly Phe Gly Gln Val Gly Gly Gln Ala Leu Ile Val His
    2480                2485                2490

Pro Ser Tyr Leu Ile Gly Ser Leu Glu Pro Lys Gln Phe Glu Ala
    2495                2500                2505

Tyr Lys Gln Lys Asn Asp Val Arg Lys Lys Trp Ser Tyr Arg Arg
    2510                2515                2520

Phe Asn Asp Phe Phe Val Asn Gly Lys Leu Val Ile Ile Lys Glu
    2525                2530                2535

Gly Ala Pro Phe Thr Pro Glu Leu Glu Thr Pro Val Leu Leu Asn
    2540                2545                2550

Pro Leu Ala Arg Thr Val Glu Asp Lys Lys Gly Ser Tyr Ser Met
    2555                2560                2565

Pro Lys Glu Leu Pro Gln Ser Pro Tyr Pro Ser Gly Asn Ser Asn
    2570                2575                2580

Ala Ala Ile Ala Ala Lys Leu Val Ser Ser Ala Thr Ser Gly Val
    2585                2590                2595

His Gly Val Gly Val Asp Thr Glu Met Ile Ser Ala Ile Pro Thr
    2600                2605                2610

Ser Asp Ser Phe Leu Glu Arg Asn Phe Thr Asp Ala Glu Ile Ala
    2615                2620                2625

Tyr Val Arg Lys Ala Ser Asp Phe Lys Ala Ser Leu Ala Ala Arg
    2630                2635                2640

Trp Ser Ala Lys Glu Ala Val Phe Lys Ala Leu Lys Thr Val Ser
    2645                2650                2655

Lys Gly Ala Ala Ala Ser Leu Lys Asp Ile Glu Ile Val Ser Thr
    2660                2665                2670

Ser Gly Ala Pro Gln Val Ala Leu His Gly Glu Ala Lys Ala Val
    2675                2680                2685

Ala Asp Ala Ala Gly Ile Lys Ser Phe Glu Leu Ser Met Ser His
    2690                2695                2700

Ser Glu Asp Val Ala Cys Ala Val Ala Ile Ala Gln Lys
    2705                2710                2715

<210> 3
<211> 2928
<212> PRT
<213> Rhodotorula toruloides

<400> 3

Met Val Ala Ala Gln Asp Leu Pro Leu Ala Leu Ser Ile Ser Phe Ala
1               5                   10                  15

Pro Glu Ser Ser Thr Ile Ser Met Thr Leu Phe Asn Gln Pro Glu Ala
            20                  25                  30

Ser Lys Pro Ala Leu Pro Leu Glu Leu Lys Tyr Lys Tyr Asp Pro Ser
        35                  40                  45

Thr Pro Tyr Ala Pro Ile His Glu Ile Thr Glu Asp Arg Asn Gln Arg
    50                  55                  60

Ile Lys Gln His Tyr Trp Asp Leu Trp Gly Leu Gly Asn Lys Ala Asp
65                  70                  75                  80

Gln Gly Ile Ser Gln Leu Lys Ile Thr Asp Glu Phe Gln Gly Asp Leu
                85                  90                  95

Val Thr Ile Ser Ala Asp Glu Ile Glu Ala Phe Cys Arg Val Val Gly
            100                 105                 110

Ile Glu Gly Glu Ala Tyr Lys Arg Asn His Lys Ala Gly Met Gln Val
        115                 120                 125

Pro Leu Asp Phe Ala Ile Lys Leu Gly Trp Lys Ala Ile Met Lys Pro
    130                 135                 140

Ile Phe Pro Ser Thr Ile Asp Gly Asp Leu Leu Lys Leu Val His Leu
145                 150                 155                 160

Ser Asn Gly Phe Arg Val Leu Pro Asp Thr Pro Thr Leu Gln Val Gly
                165                 170                 175

Asp Val Val Thr Thr Thr Ser Arg Ile Glu Ser Ile Thr Asn Ser Asp
            180                 185                 190

Thr Gly Lys Thr Val Ser Val Arg Gly Val Ile Ser Leu Val Ser Ser
        195                 200                 205

Ala Asp Ser Lys Gly Lys Asp Ala Ser Thr Glu Asp Arg Ile Pro Leu
    210                 215                 220

Ile Glu Val Thr Ser Ser Phe Phe Tyr Arg Gly Lys Phe Ser Asp Tyr
225                 230                 235                 240

Ala Gln Thr Phe Ser Arg Val Ala His Pro Thr Tyr Ser Val Pro Ile
                245                 250                 255

Thr Thr Pro Glu Ala Val Ala Val Leu Gln Ser Lys Glu Trp Phe Gln
            260                 265                 270

Trp Asp Asp Asp Ser Lys Pro Leu Glu Val Gly Thr Lys Leu Gln Phe
        275                 280                 285

Lys Val Glu Ser Asn Tyr Val Tyr Ala Asp Lys Ser Ser Tyr Ala Met
    290                 295                 300

Ala Thr Val Thr Gly Gly Ala Tyr Val Ile Thr Pro Glu Leu Lys Leu
305                 310                 315                 320

Ala Val Lys Val Ala Thr Val Asp Tyr Thr Ser Glu Gly Glu Gly Val
                325                 330                 335

Ile Gln Gly Asp Pro Val Ile Glu Tyr Leu Lys Arg His Gly Ser Ala
            340                 345                 350

Leu Asp Gln Pro Ile Met Leu Glu Asn Gly Gly Tyr Ser Leu Thr Lys
        355                 360                 365

Ala Gly Gln Cys Thr Phe Thr Thr Pro Ala Ser Asn Leu Asp Tyr Ser
    370                 375                 380

Leu Thr Ser Gly Asp Thr Asn Pro Ile His Thr Asn Pro Tyr Phe Ala
385                 390                 395                 400

Ser Leu Ala Tyr Leu Pro Gly Thr Ile Thr His Gly Met His Ser Ser
                405                 410                 415

Ala Arg Thr Arg Lys Phe Val Glu Gln Val Ala Ala Asp Asn Val Gly
            420                 425                 430

Ala Arg Val Arg Lys Tyr Glu Val Gly Phe Thr Ala Met Cys Leu Pro
        435                 440                 445

Ser Arg Lys Met Glu Val Arg Leu Lys His Val Gly Met Thr Ala Asp
    450                 455                 460

Gly Asn Arg Leu Ile Lys Val Glu Thr Val Asp Val Glu Gly Gly Asn
465                 470                 475                 480

Val Val Leu Ser Gly Thr Ala Glu Val Ala Gln Ala Pro Thr Ala Tyr
                485                 490                 495

Val Phe Thr Gly Gln Gly Ser Gln Glu Pro Gly Met Gly Met Glu Leu
            500                 505                 510

Tyr Ala Asn Ser Pro Val Ala Arg Ala Val Trp Asp Glu Ala Asp Arg
        515                 520                 525

His Leu Gly Glu Val Tyr Gly Phe Ser Ile Leu Glu Ile Val Arg Thr
    530                 535                 540

Asn Pro Lys Glu Lys Thr Val His Phe Gly Gly Leu Lys Gly Gln Ala
545                 550                 555                 560

Thr Arg Gln Lys Tyr Met Asp Met Ser Tyr Thr Thr Thr Asp His Glu
                565                 570                 575

Gly Asn Val Lys Thr Leu Pro Leu Phe Gly Asp Ile Asp Leu Arg Thr
            580                 585                 590

Ser Arg Tyr Thr Phe Ser Ser Pro Thr Gly Leu Leu Tyr Ala Thr Gln
        595                 600                 605

Phe Ala Gln Ile Ala Leu Val Val Thr Glu Lys Ala Ala Phe Glu Asp
    610                 615                 620

Met Arg Ala Lys Gly Leu Val Gln Lys Asp Cys Val Phe Ala Gly His
625                 630                 635                 640

Ser Leu Gly Glu Tyr Ser Ala Leu Ala Ser Ile Ala Asp Ile Leu Pro
                645                 650                 655

Ile Ser Ala Leu Val Asp Val Val Phe Tyr Arg Gly Ile Thr Met Gln
            660                 665                 670

Arg Ala Val Glu Arg Asp His Leu Asn Arg Ser Ser Tyr Gly Met Val
        675                 680                 685

Ala Val Asn Pro Ser Arg Ile Gly Lys Ser Phe Gly Asp Ala Ala Leu
    690                 695                 700

Arg Glu Val Val Asp Thr Ile Ala Arg Arg Gly Asn Ile Leu Ile Glu
705                 710                 715                 720

Val Val Asn Tyr Asn Val Glu Gly Gln Gln Tyr Val Val Ala Gly His
                725                 730                 735

Leu Val Ala Leu Gln Ser Leu Thr Asn Val Leu Asn Phe Leu Lys Ile
            740                 745                 750

Gln Lys Ile Asp Leu Ala Lys Leu Thr Glu Thr Met Ser Ile Glu Gln
        755                 760                 765

Val Lys Glu His Leu Cys Glu Ile Val Asp Glu Cys Val Gln Lys Ala
    770                 775                 780

Arg Asp Leu Gln Ala Lys Thr Gly Phe Ile Thr Leu Glu Arg Gly Phe
785                 790                 795                 800

Ala Thr Ile Pro Leu Pro Gly Ile Asp Val Pro Phe His Ser Arg Tyr
                805                 810                 815

Leu Trp Ala Gly Val Met Pro Phe Arg Thr Tyr Leu Ser Lys Lys Val
            820                 825                 830

Asn Pro Ala His Phe Asn Ala Asp Leu Leu Val Gly Arg Tyr Ile Pro
        835                 840                 845

Asn Leu Thr Ala Val His Tyr Glu Val Ser Lys Glu Tyr Ala Glu Arg
    850                 855                 860

Ile His Thr Gln Thr Ser Ser Pro Arg Leu Asn Lys Ile Leu Lys Ala

865                 870                 875                 880

Trp Asp Glu Glu Arg Trp Gly Ala Pro Glu Asn Arg Asn Lys Leu Gly
                885                 890                 895

Tyr Ala Ile Leu Ile Glu Leu Leu Ala Tyr Gln Phe Ala Ser Pro Val
            900                 905                 910

Arg Trp Ile Glu Thr Gln Asp Ile Leu Phe Arg Asp Phe Lys Phe Glu
        915                 920                 925

Arg Leu Val Glu Leu Gly Pro Ser Pro Thr Leu Thr Gly Met Ala Thr
    930                 935                 940

Arg Thr Gln Lys Leu Lys Tyr Asp Ala His Asp Ser Ser Val Gly Ile
945                 950                 955                 960

Lys Arg Ser Ile Tyr Cys Ile Ala Lys His Gln Lys Glu Ile Tyr Tyr
                965                 970                 975

Gln Phe Asp Asp Val Ala Gly Glu Glu Ala Pro Ala Pro Ala Ala Val
            980                 985                 990

Ala Pro Ser Ala Pro Ala Pro Lys  Ala Ala Pro Val Ala  Ala Ala Pro
        995                 1000                 1005

Pro Pro  Pro Ala Pro Val Ala  Ala Ala Pro Ala Ala  Ala Val Ala
    1010                 1015                 1020

Asp Glu  Pro Leu Lys Ala Val  Asp Thr Leu Arg Ile  Ile Ile Ala
    1025                 1030                 1035

Gln Lys  Leu Lys Lys Pro Val  Gly Glu Val Pro Leu  Thr Lys Ser
    1040                 1045                 1050

Ile Lys  Glu Leu Val Gly Gly  Lys Ser Thr Leu Gln  Asn Glu Ile
    1055                 1060                 1065

Leu Gly  Asp Leu Gln Gly Glu  Phe Ser Ser Ala Pro  Glu Lys Gly
    1070                 1075                 1080

Glu Glu  Met Pro Leu Gln Glu  Leu Gly Ala Ala Leu  Gln Gln Gly
    1085                 1090                 1095

Tyr Ser  Gly Lys Leu Gly Lys  Tyr Thr Thr Gly Val  Ile Ser Arg
    1100                 1105                 1110

Met Ile Gly Ala Lys Met Pro Gly Gly Phe Gly Leu Ser Ala Val
    1115                1120                1125

Gln Gly His Leu Gly Lys Thr Tyr Gly Leu Gly Ala Gly Arg Ile
    1130                1135                1140

Asp Gly Val Leu Leu Phe Ala Val Thr Gln Glu Pro Ala Lys Arg
    1145                1150                1155

Leu Ala Asn Glu Gly Glu Ala Lys Ala Trp Val Asp Ser Val Ala
    1160                1165                1170

Gln Gly Tyr Ala Ser Met Ala Gly Ile Ser Leu Ala Ala Gly Gly
    1175                1180                1185

Gly Ala Ala Ala Ala Ala Pro Ala Met Ala Phe Ala Ala Pro Ala
    1190                1195                1200

Ala Ala Gly Gly Gly Ala Pro Ala Ala Val Pro Asp Glu Pro Leu
    1205                1210                1215

Lys Ala Thr Asp Thr Leu Arg Ala Ile Ile Ala Gln Lys Leu Lys
    1220                1225                1230

Lys Gln Ile Pro Asp Val Pro Leu Thr Lys Ser Ile Lys Asp Leu
    1235                1240                1245

Val Gly Gly Lys Ser Thr Leu Gln Asn Glu Ile Leu Gly Asp Leu
    1250                1255                1260

Gln Gly Glu Phe Ser Ser Ala Pro Glu Lys Gly Glu Glu Met Pro
    1265                1270                1275

Leu Gln Glu Leu Gly Ala Ala Leu Asn Gln Gly Tyr Ser Gly Thr
    1280                1285                1290

Leu Gly Lys His Thr Ser Gly Leu Val Ala Arg Met Met Gly Ala
    1295                1300                1305

Lys Met Pro Gly Gly Phe Gly Leu Ser Ala Ala Lys Ala His Leu
    1310                1315                1320

Ser Lys Ala His Gly Leu Gly Pro Gly Arg Thr Asp Gly Ala Leu
    1325                1330                1335

Leu Val Ala Leu Thr Lys Glu Pro Glu Lys Arg Leu Gly Ser Glu
    1340                1345                1350

Ala Asp Ala Lys Ala Trp Leu Asp Gly Val Ala Gln Ala Tyr Ala
    1355                1360                1365

Ser Gln Ala Gly Ile Thr Leu Gly Ala Gly Gly Gly Gly Gly Gly
    1370                1375                1380

Ala Ala Val Gly Gly Ala Gly Phe Met Ile Asn Thr Glu Gln Leu
    1385                1390                1395

Asp Lys Met Gln Glu Lys Gln Asp Asn Phe Val Ser Gln Gln Val
    1400                1405                1410

Glu Leu Phe Leu Arg Tyr Leu Gly Lys Asp Ser Arg Glu Gly His
    1415                1420                1425

Arg Leu Ala Asp Met Gln Lys Ala Glu Val Ala Asn Leu Gln Glu
    1430                1435                1440

Lys Leu Asp Ser Ile Ala Arg Glu His Gly Asp Ala Tyr Val Gln
    1445                1450                1455

Gly Ile Gln Pro Val Phe Asp Pro Leu Lys Ala Arg His Phe Asn
    1460                1465                1470

Ser Ser Trp Asn Trp Val Arg Gln Asp Ala Leu Met Met Trp Met
    1475                1480                1485

Asp Ile Leu Phe Gly Arg Leu Thr Thr Val Asp Arg Asp Ile Thr
    1490                1495                1500

Ala Arg Cys Leu Val Ile Met Asn Arg Ala Asp Pro Ser Leu Ile
    1505                1510                1515

Asp Tyr Met Gln Tyr Thr Ile Asp Asn Thr Pro Val Glu Arg Gly
    1520                1525                1530

Glu His Tyr Val Leu Ala Lys Gln Phe Gly Gln Gln Leu Leu Asp
    1535                1540                1545

Asn Cys Arg Glu Met Ile Gly Gln Ala Pro Leu Tyr Lys Asp Val
    1550                1555                1560

Thr Phe Pro Thr Ala Pro Lys Thr Thr Val Asn Ala Lys Gly Asp
    1565                1570                1575

Ile Ile Thr Glu Glu Val Asn Arg Pro Gly Val Ser Arg Leu Glu
    1580                1585                1590

Lys Tyr Val Ala Glu Met Ala Ala Gly Ser Lys Val Thr Val Ala
    1595                1600                1605

Ser Val Asn Leu Asp Lys Val Gln Glu Gln Val Glu Lys Leu Tyr
    1610                1615                1620

Lys Leu Val Lys Ser Gln Pro Gln Ile Ser Lys Gln His Met Thr
    1625                1630                1635

Ser Ile Lys Ser Leu Tyr Ala Glu Val Val Arg Gly Leu Gly Lys
    1640                1645                1650

Asp Ala Gly Pro Pro Pro Val His Lys Ala Gly Thr Arg Ala Arg
    1655                1660                1665

Arg Pro Ser Ser Gln Phe Leu Arg Pro Ala Ala Val Ser Glu Ala
    1670                1675                1680

Thr Phe Leu Pro Glu Asp Lys Val Pro Leu Leu His Leu Lys Arg
    1685                1690                1695

Lys Ile Gly Asn Asp Trp Gln Tyr Ser Ser Lys Leu Thr Ser Leu
    1700                1705                1710

Tyr Leu Asp Ile Leu Lys Glu Ile Ala Thr Ser Gly Val Thr Phe
    1715                1720                1725

Glu His Lys Asn Ala Leu Met Thr Gly Val Gly Lys Gly Ser Ile
    1730                1735                1740

Gly Ile Glu Ile Val Lys Gly Leu Leu Ala Gly Gly Ala Arg Val
    1745                1750                1755

Val Ile Thr Thr Ser Arg Tyr Ser Arg Ser Thr Val Glu Tyr Tyr
    1760                1765                1770

Gln Ala Ile Tyr Gln Glu Val Gly Ser Lys Gly Ser Ser Leu Thr
    1775                1780                1785

Val Val Pro Phe Asn Gln Gly Ser Lys Gln Asp Val Glu Ala Leu
    1790                1795                1800

Val Asp Phe Ile Tyr Ser Lys Asp Lys Gly Leu Gly Met Asp Leu
    1805                1810                1815

Asp Tyr Ile Leu Pro Phe Ala Ala Leu Pro Glu Asn Gly Arg Glu

1820                1825                1830

Ile Asp Gly Ile Asp Asp Arg Ser Glu Leu Ala His Arg Ile Met
    1835                1840                1845

Leu Thr Asn Leu Leu Arg Leu Leu Gly Ala Val Lys Ser Lys Lys
    1850                1855                1860

Ala Ala Leu Lys Leu Thr Thr Arg Pro Thr Glu Val Val Leu Pro
    1865                1870                1875

Leu Ser Pro Asn His Gly Leu Phe Gly Asn Asp Gly Leu Tyr Ser
    1880                1885                1890

Glu Ser Lys Ile Ser Leu Glu Thr Leu Phe Asn Arg Trp Ser Ser
    1895                1900                1905

Glu Ser Trp Gly Glu Tyr Leu Cys Leu Ala Gly Ala Val Ile Gly
    1910                1915                1920

Trp Thr Arg Gly Thr Gly Leu Met Ser Ala Thr Asn Ser Val Ala
    1925                1930                1935

Glu Gly Ile Glu Ala Gln Gly Cys Arg Thr Phe Ser Ala Lys Glu
    1940                1945                1950

Met Ala Phe Asn Ile Leu Gly Leu Met His Pro Leu Val Phe Asp
    1955                1960                1965

Val Ala Gln Ile Glu Pro Val Trp Ala Asp Leu Asn Gly Gly Met
    1970                1975                1980

Asp Lys Leu Pro Asp Leu Ala Asn Leu Thr Thr Glu Ile Arg Lys
    1985                1990                1995

Lys Leu Asn Leu Thr Ala Ser Thr Arg Arg Ala Ile Ala Lys Asp
    2000                2005                2010

Asn Ser Phe Asp Tyr Lys Val Ala His Gly Pro Ala Met Glu Gln
    2015                2020                2025

Ile His Gln Arg Ile Asn Val Ala Pro Arg Ala Asn Phe Ser Leu
    2030                2035                2040

Pro Phe Pro Glu Leu Lys Pro Ile Asp Ala Lys Ser Glu Leu Ala
    2045                2050                2055

Lys Leu Arg Gly Leu Ile Asp Leu Glu Lys Val Val Val Met Thr
    2060                2065                2070

Gly Tyr Ala Glu Val Gly Pro Phe Gly Ser Ser Arg Thr Arg Trp
    2075                2080                2085

Glu Met Glu Ala Asn Gly Thr Phe Ser Ile Gln Gly Thr Leu Glu
    2090                2095                2100

Leu Ala Tyr Val Met Gly Leu Ile Lys His Phe Glu Gly Arg Leu
    2105                2110                2115

Lys Asp Gly Thr Leu Tyr Val Gly Trp Val Asp Ala Lys Thr Asn
    2120                2125                2130

Glu Pro Leu Asp Asp Lys Asp Val Lys Ala Ala Tyr Glu Lys His
    2135                2140                2145

Ile Leu Ala His Thr Gly Ile Arg Leu Ile Glu Pro Glu Ile Phe
    2150                2155                2160

Asn Gly Tyr Asp Pro Lys Arg Lys Gly Phe Thr Gln Glu Ile Glu
    2165                2170                2175

Ile Gln His Asp Leu Glu Pro Ile Glu Ala Ser Glu Glu Asp Ala
    2180                2185                2190

Ala Arg Phe Lys Arg Glu His Gly Ala Leu Val Asp Val Tyr Thr
    2195                2200                2205

Glu Asp Gly Ser Lys Phe Phe Val Lys Phe Lys Lys Gly Ala Lys
    2210                2215                2220

Leu His Ile Pro Lys Ala Val Ala Phe Asp Arg Leu Val Ala Gly
    2225                2230                2235

Gln Ile Pro Thr Gly Trp Ser His Lys Ala Phe Gly Ile Pro Asp
    2240                2245                2250

Asp Ile Ala Ser Gln Val Asp Arg Thr Ser Leu Trp Ala Leu Val
    2255                2260                2265

Ser Val Ala Glu Ala Leu Met Met Ala Gly Ile Thr Asp Pro Tyr
    2270                2275                2280

Glu Leu Tyr Lys Trp Ile His Pro Ser Glu Val Gly Ser Ser Leu
    2285                2290                2295

Gly Ser Gly Met Gly Gly Ile Thr Ser Ile Ser Lys Met Phe Arg
    2300                2305                2310

Asp Arg Arg Glu Glu Lys Asp Val Gln Lys Asp Ile Leu Gln Glu
    2315                2320                2325

Thr Phe Ile Asn Thr Val Ala Gly Trp Val Asn Leu Leu Leu Leu
    2330                2335                2340

Ser Ser Ser Gly Pro Ile Lys Ile Pro Val Gly Ala Cys Ala Thr
    2345                2350                2355

Ala Leu Gln Ser Val Glu Ile Ala Cys Asp Thr Ile Leu Ser Gly
    2360                2365                2370

Lys Ala Lys Ile Met Val Ser Gly Gly Tyr Asp Asp Phe Ser Glu
    2375                2380                2385

Glu Gly Ser Tyr Glu Phe Ala Asn Met Lys Ala Thr Ser Asn Ser
    2390                2395                2400

Glu Thr Glu Phe Ala Ala Gly Arg Glu Pro Asn Glu Met Ser Arg
    2405                2410                2415

Pro Thr Thr Ser Thr Arg Ala Gly Phe Met Glu Ser Met Gly Cys
    2420                2425                2430

Gly Ala Gln Val Leu Met Ser Ala Lys Thr Ala Ile Glu Met Gly
    2435                2440                2445

Ala Thr Ile Tyr Gly Ile Val Ala Tyr Thr Ala Thr Ala Thr Asp
    2450                2455                2460

Lys Ala Gly Arg Ser Ile Pro Ala Pro Gly Arg Gly Val Met Gly
    2465                2470                2475

Thr Ala Arg Glu Ile Thr Ser Lys Tyr Pro Ser Pro Ile Leu Asp
    2480                2485                2490

Val Thr Tyr Arg Arg Arg Gln Leu Glu Phe Arg Arg Lys Gln Ile
    2495                2500                2505

Ser Gln Trp Leu Glu Asn Glu Thr Glu Leu Leu Lys Phe Glu Val
    2510                2515                2520

Ser Ser His Gly Gln Ala Thr Lys Leu Pro Asp Asp Tyr Val Ser
    2525                2530                2535

Glu Arg Leu Ala Ser Ile Glu Arg Glu Ala Lys Arg Gln Glu Ala
    2540                2545                2550

Glu Ala Leu Ala Thr Tyr Gly Met Leu Ala Gly Gln Asp Pro Thr
    2555                2560                2565

Ile Ala Pro Leu Arg Arg Ala Leu Ala Val Trp Gly Leu Thr Ile
    2570                2575                2580

Asp Asp Val Gly Val Ala Ser Phe His Gly Thr Ser Thr Val Ala
    2585                2590                2595

Asn Asp Lys Asn Glu Ser Asn Ala Tyr Asn Glu Gln Phe Arg His
    2600                2605                2610

Leu Gly Arg Ala Lys Gly Asn Ala Cys Pro Val Ile Ala Gln Lys
    2615                2620                2625

Trp Leu Thr Gly His Pro Lys Gly Gly Ala Ala Ala Trp Met Leu
    2630                2635                2640

Asn Gly Leu Ala Gln Val Ile Gln Ser Gly Leu Val Pro Gly Asn
    2645                2650                2655

Arg Asn Ala Asp Asn Ile Gly Glu Glu Leu Arg Ala Phe Glu Tyr
    2660                2665                2670

Leu Leu Tyr Pro Ser Lys Ser Ile Gln Thr Asp Gly Ile Lys Ala
    2675                2680                2685

Gly Leu Leu Thr Ser Phe Gly Phe Gly Gln Val Gly Gly Gln Ala
    2690                2695                2700

Leu Ile Val His Pro Ser Leu Leu Ile Gly Ala Leu Glu Pro Ala
    2705                2710                2715

Gln Phe Glu Ala Tyr Lys Lys Leu Asn Asp Gln Arg Lys Lys Trp
    2720                2725                2730

Ser Tyr Arg Arg Phe Asn Asp Phe Phe Thr Asn Gly Lys Leu Val
    2735                2740                2745

Ile Ile Lys Asp Gly Thr Pro Phe Thr Pro Glu Gln Glu Asn Thr
    2750                2755                2760

Thr Leu Leu Asn Pro Leu Val Arg Ala Val Pro Asp Lys Thr Gly

2765                    2770                    2775

Ser Tyr  Ser Met Pro Lys Glu  Phe Pro Ala Thr Val  Pro Arg Ser
    2780                    2785                    2790

Asn Asn  Ala Glu Val Ala Asn  Lys Leu Val Ser Ala  Ala Val Gly
    2795                    2800                    2805

Gly Ala  Phe Gly Val Gly Thr  Asp Val Glu Leu Ile  Ser Ala Val
    2810                    2815                    2820

Pro Thr  Ser Glu Ser Phe Leu  Glu Arg Asn Phe Thr  Gln Asp Glu
    2825                    2830                    2835

Ile Ala  Tyr Cys Lys Ala Ala  Pro Asp Phe Arg Ala  Ser Leu Ala
    2840                    2845                    2850

Ala Arg  Trp Ser Ala Lys Glu  Ala Thr Phe Lys Ala  Leu Lys Thr
    2855                    2860                    2865

Glu Ser  Lys Gly Ala Ala Ala  Ser Met Gln Asp Ile  Glu Val Val
    2870                    2875                    2880

Ser Thr  Ser Gln Gly Pro Thr  Ile Lys Leu His Gly  Glu Val Glu
    2885                    2890                    2895

Lys Ile  Ala Gln Ala Ala Gly  Ile Thr Ala Phe Glu  Val Ser Leu
    2900                    2905                    2910

Ser His  Ser Glu Asp Val Ala  Cys Ala Val Val Ile  Ala Gln Lys
    2915                    2920                    2925

<210>  4
<211>  1884
<212>  PRT
<213>  Candida albicans

<400>  4

Met Lys Pro Glu Ile Glu Gln Glu Leu Ser His Thr Leu Leu Thr Glu
1               5                   10                  15

Leu Leu Ala Tyr Gln Phe Ala Ser Pro Val Arg Trp Ile Glu Thr Gln
            20                  25                  30

Asp Val Phe Leu Lys Gln His Asn Thr Glu Arg Ile Ile Glu Ile Gly
        35                  40                  45

Pro Ser Pro Thr Leu Ala Gly Met Ala Asn Arg Thr Ile Lys Ala Lys

50                  55                  60

Tyr Glu Ser Tyr Asp Ala Ala Leu Ser Leu Gln Arg Gln Val Leu Cys
65                  70                  75                  80

Tyr Ser Lys Asp Ala Lys Glu Ile Tyr Tyr Lys Pro Asp Pro Ala Asp
                85                  90                  95

Leu Ala Pro Lys Glu Thr Pro Lys Gln Glu Glu Ser Thr Pro Ser Ala
            100                 105                 110

Pro Ala Ala Ala Thr Pro Thr Pro Ala Ala Ala Ala Ala Pro Thr Pro
        115                 120                 125

Ala Pro Ala Pro Ala Ser Ala Gly Pro Val Glu Ser Ile Pro Asp Glu
    130                 135                 140

Pro Val Lys Ala Asn Leu Leu Ile His Val Leu Val Ala Gln Lys Leu
145                 150                 155                 160

Lys Lys Pro Leu Asp Ala Val Pro Met Thr Lys Ala Ile Lys Asp Leu
                165                 170                 175

Val Asn Gly Lys Ser Thr Val Gln Asn Glu Ile Leu Gly Asp Leu Gly
            180                 185                 190

Lys Glu Phe Gly Ser Thr Pro Glu Lys Pro Glu Asp Thr Pro Leu Glu
        195                 200                 205

Glu Leu Ala Glu Gln Phe Gln Asp Ser Phe Ser Gly Gln Leu Gly Lys
    210                 215                 220

Thr Ser Thr Ser Leu Ile Gly Arg Leu Met Ser Ser Lys Met Pro Gly
225                 230                 235                 240

Gly Phe Ser Ile Thr Thr Ala Arg Lys Tyr Leu Glu Ser Arg Phe Gly
                245                 250                 255

Leu Gly Ala Gly Arg Gln Asp Ser Val Leu Leu Met Ala Leu Thr Asn
            260                 265                 270

Glu Pro Ala Asn Arg Leu Gly Ser Glu Ala Asp Ala Lys Ala Phe Phe
        275                 280                 285

Asp Gly Ile Ala Gln Lys Tyr Ala Ser Ser Ala Gly Ile Ser Leu Ser
    290                 295                 300

Ser Gly Ala Ala Ser Gly Ala Gly Ala Ala Asn Ser Gly Gly Ala Val
305                 310                 315                 320

Val Asp Ser Ala Ala Leu Asp Ala Leu Thr Ala Glu Asn Lys Lys Leu
                325                 330                 335

Ala Lys Gln Gln Leu Glu Val Leu Ala Arg Tyr Leu Gln Val Asp Leu
            340                 345                 350

Asn Lys Gly Ala Lys Ser Phe Ile Lys Glu Lys Glu Ala Ser Ala Val
        355                 360                 365

Leu Gln Lys Glu Leu Asp Leu Trp Glu Ala Glu His Gly Glu Phe Tyr
    370                 375                 380

Ala Lys Gly Ile Gln Pro Thr Phe Ser Ala Leu Lys Ser Arg Thr Tyr
385                 390                 395                 400

Asp Ser Tyr Trp Asn Trp Ala Arg Gln Asp Val Leu Ser Met Tyr Phe
                405                 410                 415

Asp Ile Ile Phe Gly Lys Leu Thr Ser Val Asp Arg Glu Thr Ile Asn
            420                 425                 430

Gln Cys Ile Gln Ile Met Asn Arg Ala Asn Pro Thr Leu Ile Lys Phe
        435                 440                 445

Met Gln Tyr His Ile Asp His Cys Pro Glu Tyr Lys Gly Glu Thr Tyr
    450                 455                 460

Lys Leu Ala Lys Arg Leu Gly Gln Gln Leu Ile Asp Asn Cys Lys Gln
465                 470                 475                 480

Val Leu Thr Glu Asp Pro Val Tyr Lys Asp Val Ser Arg Ile Thr Gly
                485                 490                 495

Pro Lys Thr Lys Val Ser Ala Lys Gly Asn Ile Glu Tyr Glu Glu Thr
            500                 505                 510

Gln Lys Asp Ser Val Arg Lys Phe Glu Gln Tyr Val Tyr Glu Met Ala
        515                 520                 525

Gln Gly Gly Ala Met Thr Lys Val Ser Gln Pro Thr Ile Gln Glu Asp
    530                 535                 540

Leu Ala Arg Val Tyr Lys Ala Ile Ser Lys Gln Ala Ser Lys Asp Ser
545                 550                 555                 560

Lys Leu Glu Leu Gln Arg Val Tyr Glu Asp Leu Leu Lys Val Val Glu
                565                 570                 575

Ser Ser Lys Glu Ile Glu Thr Glu Gln Leu Thr Lys Asp Ile Leu Gln
            580                 585                 590

Ala Ala Thr Val Pro Thr Thr Pro Thr Glu Glu Val Asp Asp Pro Cys
        595                 600                 605

Thr Pro Ser Ser Asp Asp Glu Ile Ala Ser Leu Pro Asp Lys Thr Ser
    610                 615                 620

Ile Ile Gln Pro Val Ser Ser Thr Ile Pro Ser Gln Thr Ile Pro Phe
625                 630                 635                 640

Leu His Ile Gln Lys Lys Thr Lys Asp Gly Trp Glu Tyr Asn Lys Lys
                645                 650                 655

Leu Ser Ser Leu Tyr Leu Asp Gly Leu Glu Ser Ala Ala Ile Asn Gly
            660                 665                 670

Leu Thr Phe Lys Asp Lys Tyr Val Leu Val Thr Gly Ala Gly Ala Gly
        675                 680                 685

Ser Ile Gly Ala Glu Ile Leu Gln Gly Leu Ile Ser Gly Gly Ala Lys
    690                 695                 700

Val Ile Val Thr Thr Ser Arg Phe Ser Lys Lys Val Thr Glu Tyr Tyr
705                 710                 715                 720

Gln Asn Met Tyr Ala Arg Tyr Gly Ala Ala Gly Ser Thr Leu Ile Val
                725                 730                 735

Val Pro Phe Asn Gln Gly Ser Lys Gln Asp Val Asp Ala Leu Val Gln
            740                 745                 750

Tyr Ile Tyr Asp Glu Pro Lys Lys Gly Gly Leu Gly Trp Asp Leu Asp
        755                 760                 765

Ala Ile Ile Pro Phe Ala Ala Ile Pro Glu Asn Gly Asn Gly Leu Asp
    770                 775                 780

Asn Ile Asp Ser Lys Ser Glu Phe Ala His Arg Ile Met Leu Thr Asn
785                 790                 795                 800

Leu Leu Arg Leu Leu Gly Ala Val Lys Ser Lys Lys Thr Thr Asp Thr
                805                 810                 815

Arg Pro Ala Gln Cys Ile Leu Pro Leu Ser Pro Asn His Gly Thr Phe
            820                 825                 830

Gly Phe Asp Gly Leu Tyr Ser Glu Ser Lys Ile Ser Leu Glu Thr Leu
        835                 840                 845

Phe Asn Arg Trp Tyr Ser Glu Asp Trp Gly Ser Lys Leu Thr Val Cys
    850                 855                 860

Gly Ala Val Ile Gly Trp Thr Arg Gly Thr Gly Leu Met Ser Ala Asn
865                 870                 875                 880

Asn Ile Ile Ala Glu Gly Ile Glu Lys Leu Gly Val Arg Thr Phe Ser
                885                 890                 895

Gln Lys Glu Met Ala Phe Asn Ile Leu Gly Leu Leu Thr Pro Glu Ile
            900                 905                 910

Val Gln Leu Cys Gln Glu Glu Pro Val Met Ala Asp Leu Asn Gly Gly
        915                 920                 925

Leu Gln Phe Ile Asp Asn Leu Lys Asp Phe Thr Ser Lys Leu Arg Thr
    930                 935                 940

Asp Leu Leu Glu Thr Ala Asp Ile Arg Arg Ala Val Ser Ile Glu Ser
945                 950                 955                 960

Ala Ile Glu Gln Lys Val Val Asn Gly Asp Asn Val Asp Ala Asn Tyr
                965                 970                 975

Ser Lys Val Met Val Glu Pro Arg Ala Asn Met Lys Phe Asp Phe Pro
            980                 985                 990

Thr Leu Lys Ser Tyr Asp Glu Ile  Lys Gln Ile Ala Pro  Glu Leu Glu
        995                 1000                 1005

Gly Met  Leu Asp Leu Glu Asn  Val Val Val Val Thr  Gly Phe Ala
    1010                 1015                 1020

Glu Val  Gly Pro Trp Gly Asn  Ser Arg Thr Arg Trp  Glu Met Glu
    1025                 1030                 1035

Ala Tyr  Gly Glu Phe Ser Leu  Glu Gly Ala Ile Glu  Met Ala Trp
    1040                 1045                 1050

Ile Met  Gly Phe Ile Lys Tyr  His Asn Gly Asn Leu  Lys Gly Lys

1055                1060                1065

Pro Tyr  Ser Gly Trp Val Asp  Ala Lys Thr Gln Thr  Pro Ile Asp
    1070                1075                1080

Glu Lys  Asp Ile Lys Ser Lys  Tyr Glu Glu Glu Ile  Leu Glu His
    1085                1090                1095

Ser Gly  Ile Arg Leu Ile Glu  Pro Glu Leu Phe Asn  Gly Tyr Asp
    1100                1105                1110

Pro Lys  Lys Lys Gln Met Ile  Gln Glu Val Val Val  Gln His Asp
    1115                1120                1125

Leu Glu  Pro Phe Glu Cys Ser  Lys Glu Thr Ala Glu  Gln Tyr Lys
    1130                1135                1140

His Glu  His Gly Glu Lys Cys  Glu Ile Phe Glu Ile  Glu Glu Ser
    1145                1150                1155

Gly Glu  Tyr Thr Val Arg Ile  Leu Lys Gly Ala Thr  Leu Tyr Val
    1160                1165                1170

Pro Lys  Ala Leu Arg Phe Asp  Arg Leu Val Ala Gly  Gln Ile Pro
    1175                1180                1185

Thr Gly  Trp Asp Ala Arg Thr  Tyr Gly Ile Pro Glu  Asp Thr Ile
    1190                1195                1200

Ser Gln  Val Asp Pro Ile Thr  Leu Tyr Val Leu Val  Ala Thr Val
    1205                1210                1215

Glu Ala  Leu Leu Ser Ala Gly  Ile Thr Asp Pro Tyr  Glu Phe Tyr
    1220                1225                1230

Lys Tyr  Val His Val Ser Glu  Val Gly Asn Cys Ser  Gly Ser Gly
    1235                1240                1245

Met Gly  Gly Val Ser Ala Leu  Arg Gly Met Phe Lys  Asp Arg Tyr
    1250                1255                1260

Ala Asp  Lys Pro Val Gln Asn  Asp Ile Leu Gln Glu  Ser Phe Ile
    1265                1270                1275

Asn Thr  Met Ser Ala Trp Val  Asn Met Leu Leu Leu  Ser Ser Ser
    1280                1285                1290

Gly Pro Ile Lys Thr Pro Val Gly Ala Cys Ala Thr Ala Val Glu
    1295                1300                1305

Ser Val Asp Ile Gly Ile Glu Thr Ile Leu Ser Gly Lys Ala Lys
    1310                1315                1320

Val Val Leu Val Gly Gly Tyr Asp Asp Phe Gln Glu Glu Gly Ser
    1325                1330                1335

Tyr Glu Phe Ala Asn Met Asn Ala Thr Ser Asn Ser Ile Glu Glu
    1340                1345                1350

Phe Lys His Gly Arg Thr Pro Lys Glu Met Ser Arg Pro Thr Thr
    1355                1360                1365

Thr Thr Arg Asn Gly Phe Met Glu Ala Gln Gly Ser Gly Ile Gln
    1370                1375                1380

Val Ile Met Thr Ala Asp Leu Ala Leu Lys Met Gly Val Pro Ile
    1385                1390                1395

His Ala Val Leu Ala Met Thr Ala Thr Ala Thr Asp Lys Ile Gly
    1400                1405                1410

Arg Ser Val Pro Ala Pro Gly Lys Gly Ile Leu Thr Thr Ala Arg
    1415                1420                1425

Glu His His Gly Asn Leu Lys Tyr Pro Ser Pro Leu Leu Asn Ile
    1430                1435                1440

Glu Tyr Arg Lys Arg Gln Leu Asn Lys Arg Leu Glu Gln Ile Lys
    1445                1450                1455

Ser Trp Glu Glu Thr Glu Leu Ser Tyr Leu Gln Glu Glu Ala Glu
    1460                1465                1470

Leu Ala Lys Glu Glu Phe Gly Asp Glu Phe Ser Met His Glu Phe
    1475                1480                1485

Leu Lys Glu Arg Thr Glu Glu Val Tyr Arg Glu Ser Lys Arg Gln
    1490                1495                1500

Val Ser Asp Ala Lys Lys Gln Trp Gly Asn Ser Phe Tyr Lys Ser
    1505                1510                1515

Asp Pro Arg Ile Ala Pro Leu Arg Gly Ala Leu Ala Ala Phe Asn
    1520                1525                1530

Leu Thr Ile Asp Asp Ile Gly Val Ala Ser Phe His Gly Thr Ser
    1535                1540                1545

Thr Val Ala Asn Asp Lys Asn Glu Ser Ala Thr Ile Asn Asn Met
    1550                1555                1560

Met Lys His Leu Gly Arg Ser Glu Gly Asn Pro Val Phe Gly Val
    1565                1570                1575

Phe Gln Lys Tyr Leu Thr Gly His Pro Lys Gly Ala Ala Gly Ala
    1580                1585                1590

Trp Met Leu Asn Gly Ala Ile Gln Ile Leu Glu Ser Gly Leu Val
    1595                1600                1605

Pro Gly Asn Arg Asn Ala Asp Asn Val Asp Lys Leu Leu Glu Gln
    1610                1615                1620

Tyr Glu Tyr Val Leu Tyr Pro Ser Arg Ser Ile Gln Thr Asp Gly
    1625                1630                1635

Ile Lys Ala Val Ser Val Thr Ser Phe Gly Phe Gly Gln Lys Gly
    1640                1645                1650

Ala Gln Ala Val Val Val His Pro Asp Tyr Leu Phe Ala Val Leu
    1655                1660                1665

Asp Arg Ser Thr Tyr Glu Glu Tyr Ala Thr Lys Val Ser Ala Arg
    1670                1675                1680

Asn Lys Lys Thr Tyr Arg Tyr Met His Asn Ala Ile Thr Arg Asn
    1685                1690                1695

Thr Met Phe Val Ala Lys Asp Lys Ala Pro Tyr Ser Asp Glu Leu
    1700                1705                1710

Glu Gln Pro Val Tyr Leu Asp Pro Leu Ala Arg Val Glu Glu Asn
    1715                1720                1725

Lys Lys Lys Leu Val Phe Ser Asp Lys Thr Ile Gln Ser Ser Gln
    1730                1735                1740

Ser Tyr Val Gly Glu Val Ala Gln Lys Thr Ala Lys Ala Leu Ser
    1745                1750                1755

Ser Leu Asn Lys Ser Ser Lys Gly Val Gly Val Asp Val Glu Leu
    1760                1765                1770

Leu Ser Ala Ile Asn Ile Asp Asn Glu Thr Phe Ile Glu Arg Asn
    1775                1780                1785

Phe Thr Gly Asn Glu Val Glu Tyr Cys Leu Asn Thr Ala His Pro
    1790                1795                1800

Gln Ala Ser Phe Thr Gly Thr Trp Ser Ala Lys Glu Ala Val Phe
    1805                1810                1815

Lys Ala Leu Gly Val Glu Ser Lys Gly Ala Gly Ala Ser Leu Ile
    1820                1825                1830

Asp Ile Glu Ile Thr Arg Asp Val Asn Gly Ala Pro Lys Val Ile
    1835                1840                1845

Leu His Gly Glu Ala Lys Lys Ala Ala Ala Lys Ala Gly Val Lys
    1850                1855                1860

Asn Val Asn Ile Ser Ile Ser His Asp Asp Phe Gln Ala Thr Ala
    1865                1870                1875

Val Ala Leu Ser Glu Phe
    1880

<210> 5
<211> 1883
<212> PRT
<213> Candida tropicalis

<400> 5

Met Lys Pro Glu Ile Glu Gln Glu Leu Ser His Thr Leu Leu Thr Glu
1               5                   10                  15

Leu Leu Ala Tyr Gln Phe Ala Ser Pro Val Arg Trp Ile Glu Thr Gln
            20                  25                  30

Asp Val Phe Leu Lys Gln His Asn Thr Glu Arg Ile Ile Glu Ile Gly
        35                  40                  45

Pro Ser Pro Thr Leu Ala Gly Met Ala Asn Arg Thr Ile Lys Ala Lys
    50                  55                  60

Tyr Glu Ser Tyr Asp Ala Ala Leu Ser Leu Gln Arg Glu Val Leu Cys
65                  70                  75                  80

Tyr Ser Lys Asp Ala Lys Glu Ile Tyr Tyr Lys Pro Asp Pro Ala Asp
                85                  90                  95

Leu Ala Pro Lys Glu Glu Pro Lys Lys Glu Glu Ala Ala Ala Thr Pro
            100                 105                 110

Ala Ala Ala Ala Pro Ala Ala Ala Ala Ala Ala Pro Val Ala Ala Ala
        115                 120                 125

Pro Ala Pro Ala Ala Ala Ala Gly Pro Val Glu Ser Ile Pro Asp Glu
    130                 135                 140

Pro Val Lys Ala Ser Leu Leu Ile His Val Leu Val Ala Gln Lys Leu
145                 150                 155                 160

Lys Lys Pro Leu Asp Ala Val Pro Met Ser Lys Ala Ile Lys Asp Leu
                165                 170                 175

Val Asn Gly Lys Ser Thr Val Gln Asn Glu Ile Leu Gly Asp Leu Gly
            180                 185                 190

Lys Glu Phe Gly Ser Thr Pro Glu Lys Pro Glu Asp Thr Pro Leu Glu
        195                 200                 205

Glu Leu Ala Glu Gln Phe Gln Asp Ser Phe Ser Gly Gln Leu Gly Lys
    210                 215                 220

Thr Ser Thr Ser Leu Ile Gly Arg Leu Met Ser Ser Lys Met Pro Gly
225                 230                 235                 240

Gly Phe Ser Ile Thr Thr Ala Arg Lys Tyr Leu Glu Ser Arg Phe Gly
                245                 250                 255

Leu Gly Ser Gly Arg Gln Asp Ser Val Leu Leu Val Ala Leu Thr Asn
            260                 265                 270

Glu Pro Ala Ala Arg Leu Gly Ser Glu Ala Glu Ala Lys Thr Phe Leu
        275                 280                 285

Asp Thr Met Ala Gln Lys Tyr Ala Ser Ser Ala Gly Ile Ser Leu Thr
    290                 295                 300

Ser Ala Ser Ala Gly Ala Gly Ala Gly Gly Ala Ala Gly Gly Ala Val
305                 310                 315                 320

Val Asp Ser Ala Ala Leu Asp Ala Leu Thr Ala Glu Asn Lys Lys Leu
                325                 330                 335

Ala Arg Gln Gln Leu Glu Val Leu Ala Arg Tyr Leu Gln Val Asp Leu

340                 345                 350

Asn Gln Gly Ala Lys Ser Tyr Ile Lys Glu Lys Glu Ala Ser Ala Val
        355                 360                 365

Leu Gln Lys Glu Leu Asp Leu Trp Glu Ala Glu His Gly Glu Phe Tyr
    370                 375                 380

Ala Lys Gly Ile Lys Pro Thr Phe Ser Ser Leu Lys Ala Arg Thr Tyr
385                 390                 395                 400

Asp Ser Tyr Trp Asn Trp Ala Arg Gln Asp Val Leu Ser Met Tyr Phe
                405                 410                 415

Asp Ile Ile Phe Gly Lys Leu Thr Ser Val Asp Arg Glu Thr Ile Asn
            420                 425                 430

Gln Cys Ile Gln Ile Met Asn Arg Ser Asn Pro Thr Leu Ile Lys Phe
        435                 440                 445

Met Gln Tyr His Ile Asp His Cys Pro Glu Tyr Lys Gly Glu Thr Tyr
    450                 455                 460

Lys Leu Ala Lys Arg Leu Gly Gln Gln Leu Ile Asp Asn Cys Lys Gln
465                 470                 475                 480

Thr Leu Thr Glu Asp Pro Val Tyr Lys Asp Val Ser Arg Ile Thr Gly
                485                 490                 495

Pro Lys Thr Thr Val Ser Ala Lys Gly Asn Ile Glu Tyr Glu Glu Ala
            500                 505                 510

Glu Lys Asp Ser Val Arg Lys Phe Glu Gln Tyr Val Tyr Glu Met Ala
        515                 520                 525

Gln Gly Gly Glu Met Thr Lys Ile Ala Gln Pro Thr Ile Gln Glu Asp
    530                 535                 540

Leu Ala Arg Val Tyr Lys Ala Ile Ser Lys Gln Ala Ser Arg Glu Ser
545                 550                 555                 560

Lys Leu Glu Leu Gln Lys Val Tyr Glu Gln Leu Leu Lys Val Val Ala
                565                 570                 575

Gly Ser Thr Glu Ile Glu Thr Gln Gln Leu Thr Lys Asp Ile Leu Gln
            580                 585                 590

Ala Pro Thr Gly Ala Asn Thr Pro Thr Asp Glu Asp Glu Ile Ser Thr
        595                 600                 605
Ala Asp Ser Asp Asp Glu Ile Ala Ser Leu Pro Asp Lys Thr Ala Ile
    610                 615                 620
Ser Gln Pro Val Ser Ser Thr Val Pro His Gln Thr Ile Pro Phe Leu
625                 630                 635                 640
His Ile Gln Lys Lys Thr Asn Asp Gly Trp Glu Tyr Asp Arg Lys Leu
                645                 650                 655
Ser Ala Leu Tyr Leu Asp Gly Leu Glu Ser Ala Ala Val Asn Gly Leu
            660                 665                 670
Thr Phe Lys Asp Lys Tyr Val Leu Val Thr Gly Ala Gly Ala Gly Ser
        675                 680                 685
Ile Gly Ala Glu Ile Leu Gln Gly Leu Ile Ser Gly Gly Ala Lys Val
    690                 695                 700
Val Val Thr Thr Ser Arg Phe Ser Lys Lys Val Thr Glu Tyr Tyr Gln
705                 710                 715                 720
Asn Met Tyr Ala Arg Tyr Gly Ala Ala Gly Ser Thr Leu Ile Val Val
                725                 730                 735
Pro Phe Asn Gln Gly Ser Lys Gln Asp Val Asp Ala Leu Val Glu Tyr
            740                 745                 750
Ile Tyr Asn Asp Pro Lys Lys Gly Gly Leu Gly Trp Asp Leu Asp Ala
        755                 760                 765
Ile Ile Pro Phe Ala Ala Ile Pro Glu Asn Gly Asn Gly Ile Asp Asn
    770                 775                 780
Ile Asp Ser Arg Ser Glu Phe Ala His Arg Ile Met Leu Thr Asn Leu
785                 790                 795                 800
Leu Arg Leu Leu Gly Ala Val Lys Ser Lys Lys Thr Thr Asp Thr Arg
                805                 810                 815
Pro Ala Gln Cys Ile Leu Pro Met Ser Pro Asn His Gly Thr Phe Gly
            820                 825                 830
Phe Asp Gly Leu Tyr Ser Glu Ser Lys Ile Ser Leu Glu Thr Leu Phe
        835                 840                 845

Asn Arg Trp Tyr Ser Glu Asp Trp Gly Ser Lys Leu Thr Val Cys Gly
    850                 855                 860

Ala Val Ile Gly Trp Thr Arg Gly Thr Gly Leu Met Ser Ala Asn Asn
865                 870                 875                 880

Ile Ile Ala Glu Gly Ile Glu Lys Met Gly Val Arg Thr Phe Ser Gln
                885                 890                 895

Lys Glu Met Ala Phe Asn Ile Leu Gly Leu Met Thr Pro Asp Ile Val
            900                 905                 910

Lys Leu Cys Gln Glu Glu Pro Val Met Ala Asp Leu Asn Gly Gly Leu
        915                 920                 925

Gln Phe Ile Glu Asn Leu Lys Asp Phe Thr Ser Lys Leu Arg Ser Asp
    930                 935                 940

Leu Met Glu Ser Ala Glu Val Arg Arg Ala Val Ser Ile Glu Ser Ala
945                 950                 955                 960

Ile Glu Gln Lys Val Val Asn Gly Asp Asn Val Asp Ala Asn Tyr Ser
                965                 970                 975

Lys Val Thr Val Gln Pro Arg Ala Asn Met Lys Phe Asp Phe Pro Thr
            980                 985                 990

Leu Lys Ser Tyr Asp Asp Ile Lys  Lys Ile Ala Pro Glu  Leu Glu Gly
        995                 1000                1005

Met Leu  Asp Leu Glu Ser Val  Ile Val Val Thr Gly  Phe Ala Glu
    1010                1015                1020

Val Gly  Pro Trp Gly Asn Ala  Arg Thr Arg Trp Glu  Met Glu Ala
    1025                1030                1035

His Gly  Glu Phe Ser Leu Glu  Gly Ala Ile Glu Met  Ala Trp Ile
    1040                1045                1050

Met Gly  Phe Ile Lys Tyr His  Asn Gly Asn Leu Lys  Gly Lys Pro
    1055                1060                1065

Tyr Ser  Gly Trp Val Asp Ala  Lys Thr Gln Thr Pro  Ile Asp Asp
    1070                1075                1080

Lys Asp  Ile Lys Ala Lys Tyr  Glu Glu Glu Ile Leu  Glu His Ser
    1085                1090                1095

Gly Ile Arg Leu Ile Glu Pro Glu Leu Phe His Gly Tyr Asp Pro
    1100                1105                1110

Lys Lys Lys Gln Met Ile Gln Glu Ile Val Val Gln His Asp Leu
    1115                1120                1125

Glu Pro Phe Glu Ala Ser Lys Glu Thr Ala Glu Gln Tyr Lys His
    1130                1135                1140

Glu His Gly Asp Lys Cys Glu Ile Phe Glu Ile Glu Glu Ser Gly
    1145                1150                1155

Glu Tyr Thr Val Arg Ile Leu Lys Gly Ala Thr Leu Phe Val Pro
    1160                1165                1170

Lys Ala Leu Arg Phe Asp Arg Leu Val Ala Gly Gln Ile Pro Thr
    1175                1180                1185

Gly Trp Asp Ala Arg Thr Tyr Gly Ile Pro Glu Asp Thr Ile Ser
    1190                1195                1200

Gln Val Asp Pro Ile Thr Leu Tyr Val Leu Val Ala Thr Val Glu
    1205                1210                1215

Ala Leu Leu Ser Ala Gly Ile Thr Asp Pro Tyr Glu Phe Tyr Lys
    1220                1225                1230

Tyr Val His Val Ser Glu Val Gly Asn Cys Ser Gly Ser Gly Met
    1235                1240                1245

Gly Gly Val Ser Ala Leu Arg Gly Met Phe Lys Asp Arg Tyr Ala
    1250                1255                1260

Asp Arg Pro Val Gln Asn Asp Ile Leu Gln Glu Ser Phe Ile Asn
    1265                1270                1275

Thr Met Ser Ala Trp Val Asn Met Leu Leu Leu Ser Ser Ser Gly
    1280                1285                1290

Pro Ile Lys Thr Pro Val Gly Ala Cys Ala Thr Ala Val Glu Ser
    1295                1300                1305

Val Asp Ile Gly Val Glu Thr Ile Leu Ser Gly Lys Ala Lys Val
    1310                1315                1320

Val Met Val Gly Gly Tyr Asp Asp Phe Gln Glu Glu Gly Ser Tyr

1325                1330                1335

Glu Phe Ala Asn Met Asn Ala Thr Ser Asn Ser Leu Asp Glu Phe
    1340                1345                1350

Ala His Gly Arg Thr Pro Lys Glu Met Ser Arg Pro Thr Thr Thr
    1355                1360                1365

Thr Arg His Gly Phe Met Glu Ala Gln Gly Ser Gly Ile Gln Val
    1370                1375                1380

Ile Met Thr Ala Asp Leu Ala Ile Lys Met Gly Val Pro Ile His
    1385                1390                1395

Ala Val Leu Ala Met Ser Ala Thr Ala Thr Asp Lys Ile Gly Arg
    1400                1405                1410

Ser Val Pro Ala Pro Gly Lys Gly Ile Leu Thr Thr Ala Arg Glu
    1415                1420                1425

His His Gly Asn Leu Lys Tyr Pro Ser Pro Ile Leu Asn Ile Lys
    1430                1435                1440

Tyr Arg Lys Arg Gln Leu Asn Ala Arg Leu Glu Gln Ile Lys Ala
    1445                1450                1455

Trp Glu Glu Ser Glu Ile Ala Tyr Leu Gln Glu Glu Ala Glu Leu
    1460                1465                1470

Ala Lys Glu Glu Met Gly Asn Glu Phe Ser Met His Glu Phe Leu
    1475                1480                1485

Lys Glu Arg Thr Glu Glu Val Tyr Arg Glu Ser Lys Arg Gln Val
    1490                1495                1500

Ser Asp Ala Lys Lys Gln Trp Gly Asn Gln Phe Phe Lys Ser Asp
    1505                1510                1515

Pro Arg Ile Ala Pro Leu Arg Gly Ser Leu Ala Ala Phe Asn Leu
    1520                1525                1530

Thr Ile Asp Asp Leu Asp Val Ala Ser Phe His Gly Thr Ser Thr
    1535                1540                1545

Val Ala Asn Asp Lys Asn Glu Ser Ala Thr Ile Asn Ser Met Met
    1550                1555                1560

Lys His Leu Gly Arg Ser Glu Gly Asn Pro Val Phe Gly Val Phe
    1565                1570                1575

Gln Lys Tyr Leu Thr Gly His Pro Lys Gly Ala Ala Gly Ala Trp
    1580                1585                1590

Met Leu Asn Gly Ala Ile Gln Ile Leu Glu Ser Gly Ile Val Pro
    1595                1600                1605

Gly Asn Arg Asn Ala Asp Asn Val Asp Lys Val Leu Glu Glu Tyr
    1610                1615                1620

Glu Tyr Val Leu Tyr Pro Ser Arg Ser Ile Gln Thr Asp Gly Ile
    1625                1630                1635

Lys Ala Val Ser Val Thr Ser Phe Gly Phe Gly Gln Lys Gly Ala
    1640                1645                1650

Gln Ala Val Val Val His Pro Asp Tyr Leu Tyr Ala Val Leu Asp
    1655                1660                1665

Arg Ser Thr Tyr Glu Asp Tyr Ala Val Arg Val Ser Ala Arg Asn
    1670                1675                1680

Lys Lys Thr Tyr Arg Tyr Met His Asn Ala Ile Thr Arg Asn Thr
    1685                1690                1695

Met Phe Val Ala Lys Asp Lys Ala Pro Tyr Ala Asp Glu Leu Glu
    1700                1705                1710

Gln Pro Val Tyr Leu Asp Pro Leu Ala Arg Val Glu Asn Ala Lys
    1715                1720                1725

Glu Lys Leu Val Phe Ser Asn Lys Gly Ile Gln Ser Asn Gln Ala
    1730                1735                1740

Tyr Thr Gly Glu Asn Ala Arg Asn Thr Ala Lys Ala Leu Ala Ser
    1745                1750                1755

Leu Asn Lys Ser Ser Lys Gly Val Gly Val Asp Val Glu Leu Leu
    1760                1765                1770

Ser Ala Ile Asn Leu Glu Asn Glu Thr Phe Ile Glu Arg Asn Phe
    1775                1780                1785

Thr Ala Gly Glu Val Glu Tyr Cys Thr Lys Thr Ser Ser Pro Gln
    1790                1795                1800

Ala Ser Phe Thr Gly Thr Trp Ser Ala Lys Glu Ala Val Phe Lys
    1805                1810                1815

Ala Leu Gly Val Glu Ser Lys Gly Ala Gly Ala Ser Leu Ile Asp
    1820                1825                1830

Ile Glu Ile Thr Arg Asp Val Asn Gly Ala Pro Gln Val Ser Leu
    1835                1840                1845

His Gly Asp Ala Ala Lys Ala Ala Ala Lys Ala Gly Val Lys Asn
    1850                1855                1860

Val Lys Ile Ser Ile Ser His Asp Asp Phe Gln Ala Thr Ala Val
    1865                1870                1875

Ala Leu Ser Glu Phe
    1880

<210>  6
<211>  1440
<212>  PRT
<213>  Trichosporon oleaginosus

<400>  6

Met Ser Thr Ala Thr Arg Asp Gln Glu Ala Gln Val Arg Arg Glu Leu
1               5                   10                  15

Thr Ser Arg Cys Ile Ala Ile Met Asn Arg Ala Asp Gln Asn Leu Leu
            20                  25                  30

Asp Tyr Met Lys Tyr His Ile Asp Ser Val Asp Pro Ser Lys Gly Pro
        35                  40                  45

Asn Tyr Glu Lys Val Lys Lys Phe Gly Gln Ile Leu Ile Asp Asn Cys
    50                  55                  60

Lys Glu Val Val Asp Leu Pro Pro Val Tyr Arg Asp Val Ala Leu Pro
65                  70                  75                  80

Thr Ala Pro His Thr Glu Val Ser Ala Lys Gly Asp Ile Gln Tyr Ser
                85                  90                  95

Glu Ile Pro Arg Thr Lys Val Arg Lys Leu Glu Ser Tyr Val Lys Glu
            100                 105                 110

Met Ala Asn Gly Gly Glu Ile Glu Ser Gln Val Asn Leu Glu Lys Val
        115                 120                 125

Gln Ala Asp Ile Glu Lys Leu Trp Asp Leu Val Asn Ala Gln Pro Ser
    130                 135                 140

Ile Thr Pro Ala Gln Lys Ala Ala Ile Lys Ser Met Tyr Gly Glu Val
145                 150                 155                 160

Val Lys Ser Leu Gly Gln Gln Ala Glu Ala Asp Asp Pro Val Thr Arg
                165                 170                 175

Ala Arg Asn Ala Ala Asp Lys Pro Arg Arg Asp Ser Ser Gln Phe Leu
            180                 185                 190

Arg Pro Asn Val Gln Asp Arg Thr Glu Val Asp Glu Glu His Leu Pro
        195                 200                 205

Phe Leu His Leu Lys Arg Lys Thr Gly Thr Ser Trp Ala Tyr Ser Lys
    210                 215                 220

Lys Leu Thr Asn Ile Tyr Leu Asp Val Leu Thr Glu Ile Ala Thr Ser
225                 230                 235                 240

Gly Val Thr Phe Gln Lys Lys Ala Ala Leu Leu Thr Gly Val Gly Arg
                245                 250                 255

Gly Ser Ile Gly Val Glu Ile Leu Gln Gly Leu Leu Ala Gly Gly Ala
            260                 265                 270

Thr Cys Val Val Thr Thr Ser Arg Tyr Ser Arg Ala Val Val Asp Tyr
        275                 280                 285

Tyr Lys Gly Ile Phe His Glu Val Gly Ser Lys Gly Ser Lys Leu Ile
    290                 295                 300

Val Val Pro Phe Asn Gly Ala Ser Arg Gln Asp Thr Glu Ala Leu Val
305                 310                 315                 320

Asp Tyr Ile Tyr Asn Thr Leu Asn Ile Asp Leu Asp Tyr Ile Val Pro
                325                 330                 335

Phe Ala Ala Leu Pro Glu Asn Gly Arg Glu Ile Asp Asn Ile Asp Asp
            340                 345                 350

Lys Ser Glu Leu Ala His Arg Leu Met Leu Thr Asn Leu Leu Arg Leu
        355                 360                 365

Leu Gly Ala Val Lys Thr Lys Lys Ala Ala Lys Lys Phe Val Thr Arg
    370                 375                 380

Pro Thr Gln Val Val Leu Pro Leu Ser Pro Asn His Gly Leu Phe Gly
385                 390                 395                 400

Asn Asp Gly Leu Tyr Ser Glu Ser Lys Ile Ser Leu Glu Thr Leu Phe
                405                 410                 415

Asn Arg Trp Ser Ala Glu Ser Trp Gly Glu Tyr Leu Cys Ile Ala Gly
            420                 425                 430

Ala Val Ile Gly Trp Thr Arg Gly Thr Gly Leu Met Ser Ala Thr Asn
        435                 440                 445

Phe Val Ala Glu Gly Leu Glu Lys Leu Gly Val Arg Thr Phe Ser Ala
    450                 455                 460

Lys Glu Met Ala Phe Asn Ile Leu Gly Leu Met His Pro Leu Ile Phe
465                 470                 475                 480

Asp Ile Thr Gln Ile Glu Pro Leu Trp Ala Asp Leu Asn Gly Gly Met
                485                 490                 495

Asp Arg Val Ala Gly Leu Ala Asp Val Met Thr Ser Ile Arg Leu Asp
            500                 505                 510

Ile Asn Lys Val Ala Asp Leu Arg Lys Ala Ile Ala Leu Asp Asn Gly
        515                 520                 525

Ala Asp Phe Lys Val Thr Asn Gly Ser Glu Ala Glu Arg Leu His Gln
    530                 535                 540

Lys Val Ser Val Ala Pro Arg Ala Asn Phe Ser Phe Asp Phe Pro Thr
545                 550                 555                 560

Val Glu Asp Asp Ser Val Leu Asn Glu Leu Lys His Leu Glu Gly Leu
                565                 570                 575

Ile Asp Leu Asp Lys Val Val Val Cys Thr Gly Phe Ala Glu Ile Gly
            580                 585                 590

Pro Trp Gly Ser Ala Arg Thr Arg Trp Glu Met Glu Ala Arg Gly Glu
        595                 600                 605

Phe Thr Ile Glu Gly Ile Ile Glu Met Ala Trp Met Met Gly Met Ile
    610                 615                 620

Lys His Phe Glu Gly Met Leu Pro Asn Ala Gly Gly Met Ala Asp Pro

625                 630                 635                 640

Tyr Val Gly Trp Val Asp Ala Lys Ser Gly Glu Pro Val Asp Asp Lys
                645                 650                 655

Asp Ile Arg Asn Lys Tyr Glu Lys Glu Ile Leu Asn His Ala Gly Ile
            660                 665                 670

Arg Ile Ile Glu Pro Asp Leu Phe Phe Gly Tyr Asp Pro Glu Lys Lys
        675                 680                 685

Gly Phe Thr Gln Glu Ile Glu Leu Asn His Asp Leu Glu Pro Leu Glu
    690                 695                 700

Val Ser Ala Asp Asp Ala Ala Lys Phe Lys Arg Glu Gln Gly Asp Ala
705                 710                 715                 720

Val Asp Ile Trp Ala Gln Asp Ser Gly Glu Trp Phe Val Lys Phe Lys
                725                 730                 735

Lys Gly Ala Arg Val Leu Leu Pro Lys Ala Val Lys Phe Asp Arg Val
            740                 745                 750

Val Ala Gly Gln Ile Pro Thr Gly Trp Asp Ala Lys Arg Tyr Gly Leu
        755                 760                 765

Pro Asp Asp Ile Ile Ser Gln Val Asp Arg Thr Ala Leu Trp Ala Leu
    770                 775                 780

Val Ser Val Thr Glu Ala Leu Ile Met Ser Gly Val Thr Asp Pro Tyr
785                 790                 795                 800

Glu Leu Tyr Lys Tyr Ile His Pro Ser Glu Val Gly Thr Ser Leu Gly
                805                 810                 815

Ser Gly Met Gly Gly Met Arg Ser Leu Ser Glu Met Phe Lys Gly Arg
            820                 825                 830

Arg Glu Glu Lys Asp Val Gln Lys Asp Ile Leu Gln Glu Thr Phe Ile
        835                 840                 845

Asn Thr Val Ala Gly Trp Val Asn Leu Leu Leu Met Ser Ala Ser Gly
    850                 855                 860

Pro Val Lys Ile Pro Val Gly Ala Cys Ala Thr Ala Leu Gln Ser Val
865                 870                 875                 880

Glu Ile Gly Cys Asp Ser Ile Leu Ser Gly Lys Ala Lys Val Met Ile
                885                 890                 895

Ala Gly Gly Tyr Asp Asp Phe Ser Glu Glu Gly Ser Phe Glu Phe Ala
            900                 905                 910

Asn Met Lys Ala Thr Ser Asn Ala Glu Thr Glu Phe Ala Asn Gly Arg
        915                 920                 925

Glu Pro Asn Glu Phe Ser Arg Pro Met Thr Ser Thr Arg Ala Gly Phe
    930                 935                 940

Met Glu Ser Gln Gly Cys Gly Val His Ile Leu Met Ser Ala Lys Thr
945                 950                 955                 960

Ala Ile Glu Met Gly Ala Ser Ile Gln Gly Ile Val Ala Tyr Thr Ser
                965                 970                 975

Thr His Thr Asp Lys Ala Gly Arg Ser Val Pro Ala Pro Gly Arg Gly
            980                 985                 990

Ile Leu Ser Thr Ala Arg Glu Val Thr Pro Lys Gln Ser Leu Pro Ile
        995                 1000                1005

Leu Asp Leu Lys Tyr Arg Ala Arg Gln Leu Ala Phe Arg Arg Lys
    1010                1015                1020

Gln Ile Ser Gln Trp Phe Glu Asn Glu Leu Asp Asn Leu Arg Asp
    1025                1030                1035

Glu Ala Ser Val Ala Gly Lys Thr Asp Asp Ala Glu Trp Phe Ala
    1040                1045                1050

Gln Arg Val Asp Phe Ile Glu Arg Glu Ala Lys Arg Gln Glu Lys
    1055                1060                1065

Glu Ala Leu Ala Thr Tyr Gly Met Leu Glu Gly Ser Asp Pro Asn
    1070                1075                1080

Ile Ala Pro Leu Arg Arg Ala Leu Ala Val Trp Gly Leu Asn Ala
    1085                1090                1095

Asp Ser Val Gly Val Ile Ser Cys His Gly Thr Ser Thr Lys Ala
    1100                1105                1110

Asn Asp Lys Asn Glu Ser Gly Val Tyr Asn Leu Gln Phe Glu Gln
    1115                1120                1125

Leu Gly Arg Ser Lys Gly Asn Ala Val Pro Val Ile Thr Gln Lys
    1130                1135                1140

Tyr Leu Thr Gly His Pro Lys Gly Gly Ala Ala Ala Trp Met Phe
    1145                1150                1155

Asn Gly Met Leu Gln Thr Ile Gln Ser Ala Leu Ile Pro Gly Asn
    1160                1165                1170

Ala Asn Ala Asp Asn Ile Ser Glu Glu Leu Arg Ala Phe Pro His
    1175                1180                1185

Leu Phe Tyr Pro Ser Lys Ala Ile Gln His Thr Arg Leu Glu Ala
    1190                1195                1200

Gly Leu Leu Thr Ser Phe Gly Phe Gly Gln Val Gly Gly Gln Ala
    1205                1210                1215

Ala Ile Leu His Pro Arg Tyr Leu Phe Ala Ala Ile Pro Lys Gly
    1220                1225                1230

Gln Leu Glu Glu Tyr Lys Lys Lys Arg His Ala Arg Gln Leu Asp
    1235                1240                1245

Ser Tyr Ala Arg Gln Ser Gln Ala Met Ile Arg Asn Asn Leu Val
    1250                1255                1260

Gln Ile Lys Asp Ala Pro Pro Tyr Gly Pro Glu Leu Glu Gly Pro
    1265                1270                1275

Val Leu Leu Asn Pro Leu Ala Arg Ala Gly Pro Ser Lys Asn Gly
    1280                1285                1290

Ser Tyr Glu Phe Lys Gly Lys Leu Pro Ser Glu Val Pro Leu Ser
    1295                1300                1305

Thr Ala Asn Ala Asp Thr Ile Lys Ser Leu Leu Ser Gln Thr Lys
    1310                1315                1320

Gly Gly Ile Ala Gly Val Gly Val Asp Thr Glu Leu Ile Ser Ser
    1325                1330                1335

Val Pro Thr Ser Asp Ser Phe Arg Glu Arg Asn Phe Thr Ala Gly
    1340                1345                1350

Glu Ile Glu Tyr Cys Asn Ser Ala Pro Asp Ser Arg Ala Ser Tyr
    1355                1360                1365

Ala Gly Arg Trp Ala Ala Lys Glu Ala Val Phe Lys Ala Leu Ser
    1370                1375                1380

Val Pro Ser Lys Gly Ala Gly Ala Ser Met Lys Asp Ile Glu Ile
    1385                1390                1395

Val Ser Thr Gln Ser Gly Pro Glu Val Lys Leu His Gly Asp Ala
    1400                1405                1410

Ala Lys Ala Ala Gly Gly Lys Lys Ile Lys Val Ser Leu Ser His
    1415                1420                1425

Ser Asp Ser Ser Val Val Ala Phe Ala Val Ala Asn
    1430                1435                1440

<210>  7
<211>  1438
<212>  PRT
<213>  Cryptococcus neoformans

<400>  7

Met Ala Ala Glu Gly Met Asp Lys Glu Ser Ile Val Arg Arg Glu Leu
1               5                   10                  15

Thr Ser Arg Cys Ile Ala Ile Met Asn Arg Ala Asp Pro Ala Leu Leu
            20                  25                  30

Asp Tyr Met Lys Tyr His Ile Asp Asn Ala Asp Pro Ser Lys Gly Pro
        35                  40                  45

Thr Phe Lys Lys Ile Lys Glu Phe Gly Gln Ile Leu Leu Asp Asn Cys
    50                  55                  60

Lys Glu Val Val Asp Lys Pro Pro Val Tyr Arg Asp Val Ala Leu Pro
65                  70                  75                  80

Thr Ala Pro His Thr Glu Ile Ser Ala Lys Gly Asp Ile Ile Tyr Ser
                85                  90                  95

Glu Val Ser Arg Gln Asn Val Arg Lys Leu Glu Ser Tyr Val Lys Glu
            100                 105                 110

Met Ala Ser Gly Gly Glu Val Glu Pro Ala Val Asn Leu Glu Lys Val
        115                 120                 125

Gln Ser Asp Ile Glu Lys Leu Trp Asp Leu Val Asn Ser Gln Pro Ser
    130                 135                 140

Ile Thr Ala Ala Gln Lys Ser Ala Ile Lys Ser Met Tyr Ser Glu Val
145                 150                 155                 160

Ile Lys Ser Leu Gly Gln Ser Ser Gly Ser Ala Leu Glu Asp Thr Glu
                165                 170                 175

Ser Pro Ala Ile Ala Arg Ala Lys Gly Pro Lys Gln Arg Arg Ser Ser
            180                 185                 190

Ser Gln Phe Leu Arg Pro Asn Val Glu Asp Arg Thr Glu Val Glu Glu
        195                 200                 205

Ala His Leu Pro Phe Leu His Leu Lys Arg Lys Thr Gly Thr Ser Phe
    210                 215                 220

Ser Tyr Ser Ala Lys Leu Thr Asn Ile Tyr Phe Asp Val Leu Thr Glu
225                 230                 235                 240

Ile Ala Thr Ser Gly Val Thr Phe Ala Lys Lys Ala Ala Leu Leu Thr
                245                 250                 255

Gly Val Gly Lys Gly Ser Ile Gly Val Glu Ile Leu Lys Gly Leu Leu
            260                 265                 270

Ser Gly Gly Cys Thr Cys Ile Val Thr Thr Ser Arg Tyr Ser Arg Ala
        275                 280                 285

Ala Val Asp Tyr Tyr Lys Asn Ile Phe His Glu Ile Gly Ser Lys Gly
    290                 295                 300

Ser Lys Leu Ile Val Val Pro Phe Asn Gly Ala Ser Arg Gln Asp Val
305                 310                 315                 320

Glu Ala Leu Val Asp Tyr Ile Tyr Ser Thr Leu Gln Ile Asp Leu Asp
                325                 330                 335

Tyr Ile Ile Pro Phe Ala Ala Leu Pro Glu Asn Gly Arg Glu Ile Asp
            340                 345                 350

Ser Ile Asp Asp Lys Ser Glu Leu Ala His Arg Leu Met Leu Thr Asn
        355                 360                 365

Leu Leu Arg Leu Leu Gly Ala Val Lys Gln Lys Lys Ala Ala Arg Gln
    370                 375                 380

Phe Val Thr Arg Pro Thr Gln Val Val Leu Pro Leu Ser Pro Asn His

385                 390                 395                 400

Gly Ile Phe Gly Asn Asp Gly Leu Tyr Ser Glu Ser Lys Ile Ser Leu
                405                 410                 415

Glu Thr Leu Phe Asn Arg Trp Ser Ala Glu Ser Trp Gly Glu Tyr Leu
            420                 425                 430

Cys Ile Ala Gly Ala Val Ile Gly Trp Thr Arg Gly Thr Gly Leu Met
        435                 440                 445

Ser Ala Thr Asn Phe Val Ala Glu Gly Leu Glu Lys Leu Gly Val Arg
    450                 455                 460

Thr Phe Ser Ala Lys Glu Met Ala Phe Asn Ile Leu Gly Leu Met His
465                 470                 475                 480

Pro Leu Leu Phe Asp Ile Thr Gln Ile Glu Pro Ile Trp Ala Asp Leu
                485                 490                 495

Asn Gly Gly Met Asp Arg Val Ala Gly Leu Ala Glu Val Met Thr Ser
            500                 505                 510

Ile Arg Val Asp Ile Asn Arg Val Ala Glu Leu Arg Lys Ala Ile Thr
        515                 520                 525

Ile Asp Asn Ala Ala Asp Phe Arg Val Ile Asn Gly Gly Asp Ala Glu
    530                 535                 540

Arg Leu His Gln Lys Val Ala Ile Ala Pro Arg Ala Asn Phe Ser Phe
545                 550                 555                 560

Asp Phe Pro Lys Ile Asp Gly Asp Asp Ile Leu Asn Glu Leu Lys His
                565                 570                 575

Leu Gln Gly Leu Ile Asp Leu Asp Lys Val Ile Val Cys Thr Gly Phe
            580                 585                 590

Ser Glu Val Gly Pro Trp Gly Ser Ser Arg Thr Arg Trp Glu Met Glu
        595                 600                 605

Ala Arg Gly Glu Phe Ser Ile Glu Gly Cys Ile Glu Met Ala Trp Met
    610                 615                 620

Met Gly Phe Ile Lys His Leu Asp Gly Lys Leu Ala Asn Gly Gln Thr
625                 630                 635                 640

Tyr Val Gly Trp Val Asp Ala Lys Ser Gly Glu Pro Val Asp Asp Lys
                645                 650                 655

Asp Val Lys Thr Lys Tyr Glu Lys Asp Ile Ile Lys His Ala Gly Ile
            660                 665                 670

Arg Leu Ile Glu Pro Asp Leu Phe Trp Gly Tyr Asn Pro Glu Lys Lys
        675                 680                 685

Gly Phe Ile Gln Glu Ile Glu Leu Asn His Asp Leu Glu Pro Leu Glu
    690                 695                 700

Val Ala Ala Glu Glu Ala Ala Arg Phe Lys Arg Glu His Gly Asp Lys
705                 710                 715                 720

Val Asp Ile Trp Ala Gln Glu Ser Gly Glu Trp Phe Val Lys Phe Asn
                725                 730                 735

Lys Gly Ala Arg Ile Phe Leu Pro Lys Ala Val Lys Phe Asp Arg Val
            740                 745                 750

Val Ala Gly Gln Leu Pro Thr Gly Trp Asp Ala Arg Arg Phe Gly Leu
        755                 760                 765

Pro Glu Asp Ile Ile Ala Gln Thr Asp Arg Thr Ala Leu Trp Ala Leu
    770                 775                 780

Val Cys Thr Met Glu Ala Leu Ile Met Ser Gly Val Thr Asp Pro Tyr
785                 790                 795                 800

Glu Leu Tyr Lys Tyr Ile His Pro Ser Glu Val Gly Thr Ser Leu Gly
                805                 810                 815

Ser Gly Met Gly Gly Met His Ser Met Ser Ala Met Phe Lys Asp Arg
            820                 825                 830

Arg Glu Glu Arg Asp Val Gln Lys Asp Ile Leu Gln Glu Thr Phe Ile
        835                 840                 845

Asn Thr Val Ala Gly Trp Val Asn Leu Leu Leu Leu Ser Ser Ser Gly
    850                 855                 860

Pro Val Lys Ile Pro Val Gly Ala Cys Ala Thr Ala Leu Gln Ser Val
865                 870                 875                 880

Glu Ile Ala Cys Asp Thr Ile Leu Thr Gly Lys Ala Lys Ile Met Ile
                885                 890                 895

Ala Gly Gly Phe Asp Asp Phe Ser Glu Glu Gly Ser Phe Glu Phe Ala
            900                 905                 910

Asn Met Lys Ala Thr Ser Asn Ala Glu Thr Glu Phe Ala Met Gly Arg
        915                 920                 925

Glu Pro Asn Glu Phe Ser Arg Pro Met Thr Ser Thr Arg Ala Gly Phe
    930                 935                 940

Met Glu Ser Gln Gly Cys Gly Val His Val Met Met Ser Ala Lys Thr
945                 950                 955                 960

Ala Ile Glu Met Gly Ala Ser Ile Gln Gly Ile Val Ala Tyr Thr Ser
                965                 970                 975

Thr His Thr Asp Lys Ala Gly Arg Ser Ile Pro Ala Pro Gly Arg Gly
            980                 985                 990

Ile Leu Ser Thr Ala Arg Glu Val Thr Pro Lys Glu Ala Leu Pro Leu
        995                 1000                1005

Leu Asp Ile Lys Tyr Arg Ser Arg Gln Leu Ala Phe Arg Arg Lys
    1010                1015                1020

Gln Ile Ser Gln Trp Leu Glu Asn Glu His Glu Leu Leu Arg Met
    1025                1030                1035

Glu Leu Glu Thr Arg Lys Gly Gly Asp Asn Glu Asp Trp Phe Gln
    1040                1045                1050

Asn Arg Val Ala Phe Ile Asp Asp Glu Ala Lys Arg Gln Glu Lys
    1055                1060                1065

Asp Ala Leu Ala Thr Phe Gly Met Leu Glu Gly Ser His Pro Asn
    1070                1075                1080

Ile Ala Pro Leu Arg Arg Ala Leu Ala Val Trp Gly Leu Asp Ala
    1085                1090                1095

Asp Ser Val Gly Ala Ile Ser Cys His Gly Thr Ser Thr Lys Ala
    1100                1105                1110

Asn Asp Lys Asn Glu Ser Gly Val Tyr Asn Leu Gln Phe Glu Gln
    1115                1120                1125

Leu Gly Arg Thr Pro Gly Asn Ala Val Pro Val Ile Ala Gln Lys
    1130                1135                1140

Ser Leu Thr Gly His Pro Lys Gly Gly Ala Ala Ala Trp Met Phe
    1145                1150                1155

Asn Gly Met Cys Gln Thr Ile Asn Ser Ala Leu Val Pro Gly Asn
    1160                1165                1170

His Asn Ala Asp Asn Ile Ser Glu Glu Leu Arg Ala Phe Arg His
    1175                1180                1185

Leu Phe Tyr Pro Ser Lys Pro Ile Gln His Val Arg Leu Glu Cys
    1190                1195                1200

Gly Leu Leu Thr Ser Phe Gly Phe Gly Gln Val Gly Gly Gln Val
    1205                1210                1215

Ala Ile Val His Pro Arg Tyr Leu Phe Ala Ala Leu Gln Ala His
    1220                1225                1230

Glu Leu Glu Ala Tyr Lys Lys Arg Arg Gln Ala Arg Glu Leu Asp
    1235                1240                1245

Thr Tyr Ser Arg Met Ser Ser Ala Ile Val Asn Asn Asn Met Val
    1250                1255                1260

Gln Ile Lys Glu Gly Pro Pro Tyr Thr Ala Glu Leu Glu Ser Lys
    1265                1270                1275

Val Leu Leu Asn Pro Leu Ala Arg Ala Gly Pro Ser Lys Asn Ser
    1280                1285                1290

Phe Ala Phe Gln Gly Lys Leu Pro Ala Lys Val Pro Val Asp Ile
    1295                1300                1305

Lys Asn Ala Glu Thr Leu Lys Ala Met Phe Asp Gln Ala Gly Ala
    1310                1315                1320

Leu Ser Gly Val Gly Val Asp Thr Glu Leu Ile Ser Ser Val Pro
    1325                1330                1335

Thr Ser Glu Thr Phe Arg Glu Arg Asn Phe Thr Ala Asp Glu Ile
    1340                1345                1350

Ser Tyr Cys Asn Ser Ala Ala Asp Pro Thr Ala Ser Phe Ala Gly
    1355                1360                1365

Arg Trp Ala Ala Lys Glu Ala Val Phe Lys Ala Leu Ser Val Pro

1370                1375                1380

Ser Lys  Gly Ala Gly Ala Pro  Leu Lys Glu Ile Glu  Ile Val Ser
    1385                1390                1395

Thr Ser  Ser Gly Pro Thr Val  Lys Leu Ser Gly Asp  Ala Leu Ala
    1400                1405                1410

Ala Ala  Gly Gly Lys Ser Val  Lys Val Ser Leu Ser  His Ser Asp
    1415                1420                1425

Thr Ser  Val Val Ala Phe Ala  Val Ala Gln
    1430                1435

<210>  8
<211>  1438
<212>  PRT
<213>  Cryptococcus gattii

<400>  8

Met Ala Ala Glu Gly Met Asp Lys Glu Ser Ile Val Arg Arg Glu Leu
1               5                   10                  15

Thr Ser Arg Cys Ile Ala Ile Met Asn Arg Ala Asp Pro Ala Leu Leu
            20                  25                  30

Glu Tyr Met Lys Tyr His Ile Asp Asn Ala Asp Pro Ser Lys Gly Pro
        35                  40                  45

Thr Phe Lys Lys Ile Lys Glu Phe Gly Gln Ile Leu Leu Asp Asn Cys
    50                  55                  60

Lys Glu Val Val Asp Lys Pro Pro Val Tyr Arg Asp Val Ala Leu Pro
65                  70                  75                  80

Thr Ala Pro His Thr Glu Val Ser Ala Lys Gly Asp Ile Ile Tyr Ser
                85                  90                  95

Glu Val Ser Arg Gln Asn Val Arg Lys Leu Glu Ser Tyr Val Lys Glu
            100                 105                 110

Met Ala Ser Gly Gly Glu Val Glu Pro Ala Val Asn Leu Glu Lys Val
        115                 120                 125

Gln Ser Asp Ile Glu Lys Leu Trp Glu Leu Val Asn Ser Gln Pro Ser
    130                 135                 140

Ile Thr Ala Ala Gln Lys Ser Ala Ile Lys Ser Met Tyr Ser Glu Val

145                 150                 155                 160

Ile Lys Ser Leu Gly Gln Ser Ser Gly Ser Ala Val Glu Asp Ala Glu
                165                 170                 175

Ser Pro Ala Ile Ala Arg Thr Lys Gly Thr Lys Gln Arg Arg Ser Ser
            180                 185                 190

Ser Gln Phe Leu Arg Pro Asn Val Glu Asp Arg Thr Glu Val Glu Glu
        195                 200                 205

Thr His Leu Pro Phe Leu His Leu Lys Arg Lys Thr Gly Thr Ser Phe
    210                 215                 220

Ser Tyr Ser Ala Lys Leu Thr Asn Ile Tyr Phe Asp Val Leu Thr Glu
225                 230                 235                 240

Ile Ala Thr Ser Gly Val Thr Phe Ala Lys Lys Ala Ala Leu Leu Thr
                245                 250                 255

Gly Val Gly Lys Gly Ser Ile Gly Val Glu Ile Leu Lys Gly Leu Leu
            260                 265                 270

Ser Gly Gly Cys Thr Cys Ile Val Thr Thr Ser Arg Tyr Ser Arg Ala
        275                 280                 285

Ala Val Asp Tyr Tyr Lys Ser Ile Phe His Glu Leu Gly Ser Lys Gly
    290                 295                 300

Ser Lys Leu Ile Val Val Pro Phe Asn Gly Ala Ser Arg Gln Asp Val
305                 310                 315                 320

Glu Ala Leu Val Asp Tyr Ile Tyr Ser Thr Leu Gln Ile Asp Leu Asp
                325                 330                 335

Tyr Ile Ile Pro Phe Ala Ala Leu Pro Glu Asn Gly Arg Glu Ile Asp
            340                 345                 350

Ser Ile Asp Asp Lys Ser Glu Leu Ala His Arg Leu Met Leu Thr Asn
        355                 360                 365

Leu Leu Arg Leu Leu Gly Ala Val Lys Gln Lys Lys Ala Ala Arg Gln
    370                 375                 380

Phe Val Thr Arg Pro Thr Gln Val Val Leu Pro Leu Ser Pro Asn His
385                 390                 395                 400

Gly Ile Phe Gly Asn Asp Gly Leu Tyr Ser Glu Ser Lys Ile Ser Leu
                405                 410                 415

Glu Thr Leu Phe Asn Arg Trp Ser Ala Glu Ser Trp Gly Glu Tyr Leu
            420                 425                 430

Cys Ile Ala Gly Ala Val Ile Gly Trp Thr Arg Gly Thr Gly Leu Met
        435                 440                 445

Ser Ala Thr Asn Phe Val Ala Glu Gly Leu Glu Lys Leu Gly Val Arg
    450                 455                 460

Thr Phe Ser Pro Lys Glu Met Ala Phe Asn Ile Leu Gly Leu Met His
465                 470                 475                 480

Pro Leu Leu Phe Asp Ile Thr Gln Ile Glu Pro Ile Trp Ala Asp Leu
                485                 490                 495

Asn Gly Gly Met Asp Arg Val Ala Gly Leu Ala Glu Val Met Thr Ser
            500                 505                 510

Ile Arg Val Asp Ile Asn Arg Met Ala Glu Leu Arg Lys Ala Ile Thr
        515                 520                 525

Leu Asp Asn Ser Ala Asp Phe Arg Val Ile Asn Gly Gly Asp Ala Glu
    530                 535                 540

Arg Leu Tyr Gln Lys Val Ala Ile Ala Pro Arg Ala Asn Phe Ser Phe
545                 550                 555                 560

Asp Phe Pro Lys Ile Asp Gly Asp Asp Ile Leu Asn Glu Leu Lys His
                565                 570                 575

Leu Gln Gly Leu Ile Asp Leu Asp Lys Val Ile Val Cys Thr Gly Phe
            580                 585                 590

Ala Glu Val Gly Pro Trp Gly Ser Ser Arg Thr Arg Trp Glu Met Glu
        595                 600                 605

Ala Arg Gly Glu Phe Thr Ile Glu Gly Cys Ile Glu Met Ala Trp Met
    610                 615                 620

Met Gly Phe Ile Lys His Leu Asp Gly Lys Leu Gly Asn Gly Gln Thr
625                 630                 635                 640

Tyr Val Gly Trp Val Asp Ala Lys Ser Gly Glu Pro Val Asp Asp Lys
                645                 650                 655

Asp Val Lys Thr Lys Tyr Glu Lys Asp Ile Ile Lys His Ala Gly Ile
        660                 665                 670

Arg Leu Ile Glu Pro Asp Leu Phe Trp Gly Tyr Asn Pro Glu Lys Lys
    675                 680                 685

Gly Phe Ile Gln Glu Ile Glu Leu Asn His Asp Leu Glu Pro Leu Glu
    690                 695                 700

Val Ala Ala Glu Glu Ala Ala Arg Phe Lys Arg Glu His Gly Asp Lys
705                 710                 715                 720

Val Asp Val Trp Ala Gln Glu Ser Gly Glu Trp Phe Val Lys Phe Asn
                725                 730                 735

Lys Gly Ala Arg Ile Phe Leu Pro Lys Ala Val Lys Phe Asp Arg Val
        740                 745                 750

Val Ala Gly Gln Leu Pro Thr Gly Trp Asp Ala Arg Arg Phe Gly Leu
    755                 760                 765

Pro Asp Asp Ile Ile Ala Gln Thr Asp Arg Thr Ala Leu Trp Ala Leu
    770                 775                 780

Val Cys Thr Met Glu Ala Leu Ile Met Ser Gly Val Thr Asp Pro Tyr
785                 790                 795                 800

Glu Leu Tyr Lys Tyr Ile His Pro Ser Glu Val Gly Thr Ser Leu Gly
                805                 810                 815

Ser Gly Met Gly Gly Met His Ser Met Ser Ala Met Phe Lys Asp Arg
        820                 825                 830

Arg Glu Glu Arg Asp Val Gln Lys Asp Ile Leu Gln Glu Thr Phe Ile
    835                 840                 845

Asn Thr Val Ala Gly Trp Val Asn Leu Leu Leu Leu Ser Ser Ser Gly
    850                 855                 860

Pro Val Lys Ile Pro Val Gly Ala Cys Ala Thr Ala Leu Gln Ser Val
865                 870                 875                 880

Glu Ile Ala Cys Asp Thr Ile Ile Ser Gly Lys Ala Lys Val Met Ile
                885                 890                 895

Ala Gly Gly Phe Asp Asp Phe Ser Glu Glu Gly Ser Phe Glu Phe Ala
        900                 905                 910

Asn Met Lys Ala Thr Ser Asn Ala Glu Thr Glu Phe Ala Met Gly Arg
        915                 920                 925

Glu Pro Asn Glu Phe Ser Arg Pro Met Thr Ser Thr Arg Ala Gly Phe
    930                 935                 940

Met Glu Ser Gln Gly Cys Gly Val His Val Met Met Ser Ala Lys Thr
945                 950                 955                 960

Ala Ile Glu Met Gly Ala Ser Ile Gln Gly Ile Val Ala Tyr Ser Ser
                965                 970                 975

Thr His Thr Asp Lys Ala Gly Arg Ser Ile Pro Ala Pro Gly Arg Gly
            980                 985                 990

Ile Leu Ser Thr Ala Arg Glu Ile Thr Pro Lys Glu Ala Leu Pro Leu
        995                 1000                1005

Leu Asp Val Lys Tyr Arg Ser Arg Gln Leu Ala Phe Arg Arg Lys
    1010                1015                1020

Gln Ile Ser Gln Trp Leu Glu Asn Glu His Glu Leu Leu Arg Met
    1025                1030                1035

Glu Leu Glu Thr Arg Lys Gly Gly Asp Asn Glu Glu Trp Phe Gln
    1040                1045                1050

Asn Arg Val Ala Phe Ile Asp Asp Glu Ala Lys Arg Gln Glu Lys
    1055                1060                1065

Glu Ala Leu Ala Thr Phe Gly Met Leu Glu Gly Ser His Pro Asn
    1070                1075                1080

Val Ala Pro Leu Arg Arg Ala Leu Ala Val Trp Gly Leu Asp Ala
    1085                1090                1095

Asp Ser Val Gly Ala Ile Ser Cys His Gly Thr Ser Thr Lys Ala
    1100                1105                1110

Asn Asp Lys Asn Glu Ser Gly Val Tyr Asn Leu Gln Phe Glu Gln
    1115                1120                1125

Leu Gly Arg Thr Pro Gly Asn Ala Val Pro Val Ile Ala Gln Lys
    1130                1135                1140

Ser Leu Thr Gly His Pro Lys Gly Gly Ala Ala Ala Trp Met Phe

1145                1150                1155

Asn Gly Met Cys Gln Thr Ile Asn Ser Ala Leu Val Pro Gly Asn
    1160                1165                1170

His Asn Ala Asp Asn Ile Ser Glu Glu Leu Arg Ala Phe Pro His
    1175                1180                1185

Leu Phe Tyr Pro Ser Lys Pro Ile Gln His Val Arg Leu Glu Cys
    1190                1195                1200

Gly Leu Leu Thr Ser Phe Gly Phe Gly Gln Val Gly Gly Gln Ile
    1205                1210                1215

Ala Ile Val His Pro Arg Tyr Leu Phe Ala Ala Leu Gln Ala His
    1220                1225                1230

Glu Leu Glu Ala Tyr Lys Lys Arg Arg Gln Asp Arg Glu Leu Asp
    1235                1240                1245

Thr Tyr Ser Arg Met Ser Ser Ala Leu Val Asn Asn Asn Met Val
    1250                1255                1260

Gln Ile Lys Glu Gly Pro Pro Tyr Thr Ala Glu Leu Glu Gly Gly
    1265                1270                1275

Val Leu Leu Asn Pro Leu Ala Arg Ala Gly Pro Ser Lys Asn Ser
    1280                1285                1290

Phe Ala Phe Gln Gly Lys Leu Pro Thr Lys Val Pro Val Asp Val
    1295                1300                1305

Lys Asn Ala Glu Thr Leu Lys Ala Met Phe Asp Gln Ala Gly Ala
    1310                1315                1320

Leu Ser Gly Val Gly Val Asp Thr Glu Leu Ile Ser Ser Val Pro
    1325                1330                1335

Thr Ser Glu Thr Phe Arg Glu Arg Asn Phe Thr Ala Asp Glu Ile
    1340                1345                1350

Ser Tyr Cys Ser Ser Ala Ala Asp Pro Val Ala Ser Phe Ala Gly
    1355                1360                1365

Arg Trp Ala Ala Lys Glu Ala Val Phe Lys Ala Leu Ser Val Pro
    1370                1375                1380

Ser Lys Gly Ala Gly Ala Pro Leu Lys Glu Ile Glu Ile Val Ser
    1385                1390                1395

Thr Pro Ser Gly Pro Thr Val Lys Leu Ser Gly Asp Ala Leu Ala
    1400                1405                1410

Ala Ala Gly Gly Lys Ser Val Lys Val Ser Leu Ser His Ser Asp
    1415                1420                1425

Thr Ser Val Val Ala Phe Ala Val Ala Gln
    1430                1435

<210> 9
<211> 1887
<212> PRT
<213> Saccharomyces cerevisiae

<400> 9

Met Lys Pro Glu Val Glu Gln Glu Leu Ala His Ile Leu Leu Thr Glu
1               5                   10                  15

Leu Leu Ala Tyr Gln Phe Ala Ser Pro Val Arg Trp Ile Glu Thr Gln
            20                  25                  30

Asp Val Phe Leu Lys Asp Phe Asn Thr Glu Arg Val Val Glu Ile Gly
        35                  40                  45

Pro Ser Pro Thr Leu Ala Gly Met Ala Gln Arg Thr Leu Lys Asn Lys
    50                  55                  60

Tyr Glu Ser Tyr Asp Ala Ala Leu Ser Leu His Arg Glu Ile Leu Cys
65                  70                  75                  80

Tyr Ser Lys Asp Ala Lys Glu Ile Tyr Tyr Thr Pro Asp Pro Ser Glu
                85                  90                  95

Leu Ala Ala Lys Glu Glu Pro Ala Lys Glu Glu Ala Pro Ala Pro Thr
            100                 105                 110

Pro Ala Ala Ser Ala Pro Ala Pro Ala Ala Ala Ala Pro Ala Pro Val
        115                 120                 125

Ala Ala Ala Ala Pro Ala Ala Ala Ala Ala Glu Ile Ala Asp Glu Pro
    130                 135                 140

Val Lys Ala Ser Leu Leu Leu His Val Leu Val Ala His Lys Leu Lys
145                 150                 155                 160

Lys Ser Leu Asp Ser Ile Pro Met Ser Lys Thr Ile Lys Asp Leu Val
                165                 170                 175

Gly Gly Lys Ser Thr Val Gln Asn Glu Ile Leu Gly Asp Leu Gly Lys
            180                 185                 190

Glu Phe Gly Thr Thr Pro Glu Lys Pro Glu Glu Thr Pro Leu Glu Glu
        195                 200                 205

Leu Ala Glu Thr Phe Gln Asp Thr Phe Ser Gly Ala Leu Gly Lys Gln
    210                 215                 220

Ser Ser Ser Leu Leu Ser Arg Leu Ile Ser Ser Lys Met Pro Gly Gly
225                 230                 235                 240

Phe Thr Ile Thr Val Ala Arg Lys Tyr Leu Gln Thr Arg Trp Gly Leu
                245                 250                 255

Pro Ser Gly Arg Gln Asp Gly Val Leu Leu Val Ala Leu Ser Asn Glu
            260                 265                 270

Pro Ala Ala Arg Leu Gly Ser Glu Ala Asp Ala Lys Ala Phe Leu Asp
        275                 280                 285

Ser Met Ala Gln Lys Tyr Ala Ser Ile Val Gly Val Asp Leu Ser Ser
    290                 295                 300

Ala Ala Ser Ala Ser Gly Ala Ala Gly Ala Gly Ala Ala Ala Gly Ala
305                 310                 315                 320

Ala Met Ile Asp Ala Gly Ala Leu Glu Glu Ile Thr Lys Asp His Lys
                325                 330                 335

Val Leu Ala Arg Gln Gln Leu Gln Val Leu Ala Arg Tyr Leu Lys Met
            340                 345                 350

Asp Leu Asp Asn Gly Glu Arg Lys Phe Leu Lys Glu Lys Asp Thr Val
        355                 360                 365

Ala Glu Leu Gln Ala Gln Leu Asp Tyr Leu Asn Ala Glu Leu Gly Glu
    370                 375                 380

Phe Phe Val Asn Gly Val Ala Thr Ser Phe Ser Arg Lys Lys Ala Arg
385                 390                 395                 400

Thr Phe Asp Ser Ser Trp Asn Trp Ala Lys Gln Ser Leu Leu Ser Leu
                405                 410                 415

Tyr Phe Glu Ile Ile His Gly Val Leu Lys Asn Val Asp Arg Glu Val
        420                 425                 430

Val Ser Glu Ala Ile Asn Ile Met Asn Arg Ser Asn Asp Ala Leu Ile
    435                 440                 445

Lys Phe Met Glu Tyr His Ile Ser Asn Thr Asp Glu Thr Lys Gly Glu
    450                 455                 460

Asn Tyr Gln Leu Val Lys Thr Leu Gly Glu Gln Leu Ile Glu Asn Cys
465                 470                 475                 480

Lys Gln Val Leu Asp Val Asp Pro Val Tyr Lys Asp Val Ala Lys Pro
                485                 490                 495

Thr Gly Pro Lys Thr Ala Ile Asp Lys Asn Gly Asn Ile Thr Tyr Ser
        500                 505                 510

Glu Glu Pro Arg Glu Lys Val Arg Lys Leu Ser Gln Tyr Val Gln Glu
    515                 520                 525

Met Ala Leu Gly Gly Pro Ile Thr Lys Glu Ser Gln Pro Thr Ile Glu
    530                 535                 540

Glu Asp Leu Thr Arg Val Tyr Lys Ala Ile Ser Ala Gln Ala Asp Lys
545                 550                 555                 560

Gln Asp Ile Ser Ser Ser Thr Arg Val Glu Phe Glu Lys Leu Tyr Ser
                565                 570                 575

Asp Leu Met Lys Phe Leu Glu Ser Ser Lys Glu Ile Asp Pro Ser Gln
        580                 585                 590

Thr Thr Gln Leu Ala Gly Met Asp Val Glu Asp Ala Leu Asp Lys Asp
    595                 600                 605

Ser Thr Lys Glu Val Ala Ser Leu Pro Asn Lys Ser Thr Ile Ser Lys
    610                 615                 620

Thr Val Ser Ser Thr Ile Pro Arg Glu Thr Ile Pro Phe Leu His Leu
625                 630                 635                 640

Arg Lys Lys Thr Pro Ala Gly Asp Trp Lys Tyr Asp Arg Gln Leu Ser
                645                 650                 655

Ser Leu Phe Leu Asp Gly Leu Glu Lys Ala Ala Phe Asn Gly Val Thr
        660                 665                 670

Phe Lys Asp Lys Tyr Val Leu Ile Thr Gly Ala Gly Lys Gly Ser Ile
        675                 680                 685

Gly Ala Glu Val Leu Gln Gly Leu Leu Gln Gly Gly Ala Lys Val Val
    690                 695                 700

Val Thr Thr Ser Arg Phe Ser Lys Gln Val Thr Asp Tyr Tyr Gln Ser
705                 710                 715                 720

Ile Tyr Ala Lys Tyr Gly Ala Lys Gly Ser Thr Leu Ile Val Val Pro
                725                 730                 735

Phe Asn Gln Gly Ser Lys Gln Asp Val Glu Ala Leu Ile Glu Phe Ile
            740                 745                 750

Tyr Asp Thr Glu Lys Asn Gly Gly Leu Gly Trp Asp Leu Asp Ala Ile
        755                 760                 765

Ile Pro Phe Ala Ala Ile Pro Glu Gln Gly Ile Glu Leu Glu His Ile
    770                 775                 780

Asp Ser Lys Ser Glu Phe Ala His Arg Ile Met Leu Thr Asn Ile Leu
785                 790                 795                 800

Arg Met Met Gly Cys Val Lys Lys Gln Lys Ser Ala Arg Gly Ile Glu
                805                 810                 815

Thr Arg Pro Ala Gln Val Ile Leu Pro Met Ser Pro Asn His Gly Thr
            820                 825                 830

Phe Gly Gly Asp Gly Met Tyr Ser Glu Ser Lys Leu Ser Leu Glu Thr
        835                 840                 845

Leu Phe Asn Arg Trp His Ser Glu Ser Trp Ala Asn Gln Leu Thr Val
    850                 855                 860

Cys Gly Ala Ile Ile Gly Trp Thr Arg Gly Thr Gly Leu Met Ser Ala
865                 870                 875                 880

Asn Asn Ile Ile Ala Glu Gly Ile Glu Lys Met Gly Val Arg Thr Phe
                885                 890                 895

Ser Gln Lys Glu Met Ala Phe Asn Leu Leu Gly Leu Leu Thr Pro Glu
            900                 905                 910

Val Val Glu Leu Cys Gln Lys Ser Pro Val Met Ala Asp Leu Asn Gly

        915                 920                 925

Gly Leu Gln Phe Val Pro Glu Leu Lys Glu Phe Thr Ala Lys Leu Arg
    930                 935                 940

Lys Glu Leu Val Glu Thr Ser Glu Val Arg Lys Ala Val Ser Ile Glu
945                 950                 955                 960

Thr Ala Leu Glu His Lys Val Val Asn Gly Asn Ser Ala Asp Ala Ala
                965                 970                 975

Tyr Ala Gln Val Glu Ile Gln Pro Arg Ala Asn Ile Gln Leu Asp Phe
            980                 985                 990

Pro Glu Leu Lys Pro Tyr Lys Gln Val Lys Gln Ile Ala Pro Ala Glu
        995                 1000                1005

Leu Glu Gly Leu Leu Asp Leu Glu Arg Val Ile Val Val Thr Gly
    1010                1015                1020

Phe Ala Glu Val Gly Pro Trp Gly Ser Ala Arg Thr Arg Trp Glu
    1025                1030                1035

Met Glu Ala Phe Gly Glu Phe Ser Leu Glu Gly Cys Val Glu Met
    1040                1045                1050

Ala Trp Ile Met Gly Phe Ile Ser Tyr His Asn Gly Asn Leu Lys
    1055                1060                1065

Gly Arg Pro Tyr Thr Gly Trp Val Asp Ser Lys Thr Lys Glu Pro
    1070                1075                1080

Val Asp Asp Lys Asp Val Lys Ala Lys Tyr Glu Thr Ser Ile Leu
    1085                1090                1095

Glu His Ser Gly Ile Arg Leu Ile Glu Pro Glu Leu Phe Asn Gly
    1100                1105                1110

Tyr Asn Pro Glu Lys Lys Glu Met Ile Gln Glu Val Ile Val Glu
    1115                1120                1125

Glu Asp Leu Glu Pro Phe Glu Ala Ser Lys Glu Thr Ala Glu Gln
    1130                1135                1140

Phe Lys His Gln His Gly Asp Lys Val Asp Ile Phe Glu Ile Pro
    1145                1150                1155

Glu Thr Gly Glu Tyr Ser Val Lys Leu Leu Lys Gly Ala Thr Leu
    1160                1165                1170

Tyr Ile Pro Lys Ala Leu Arg Phe Asp Arg Leu Val Ala Gly Gln
    1175                1180                1185

Ile Pro Thr Gly Trp Asn Ala Lys Thr Tyr Gly Ile Ser Asp Asp
    1190                1195                1200

Ile Ile Ser Gln Val Asp Pro Ile Thr Leu Phe Val Leu Val Ser
    1205                1210                1215

Val Val Glu Ala Phe Ile Ala Ser Gly Ile Thr Asp Pro Tyr Glu
    1220                1225                1230

Met Tyr Lys Tyr Val His Val Ser Glu Val Gly Asn Cys Ser Gly
    1235                1240                1245

Ser Gly Met Gly Gly Val Ser Ala Leu Arg Gly Met Phe Lys Asp
    1250                1255                1260

Arg Phe Lys Asp Glu Pro Val Gln Asn Asp Ile Leu Gln Glu Ser
    1265                1270                1275

Phe Ile Asn Thr Met Ser Ala Trp Val Asn Met Leu Leu Ile Ser
    1280                1285                1290

Ser Ser Gly Pro Ile Lys Thr Pro Val Gly Ala Cys Ala Thr Ser
    1295                1300                1305

Val Glu Ser Val Asp Ile Gly Val Glu Thr Ile Leu Ser Gly Lys
    1310                1315                1320

Ala Arg Ile Cys Ile Val Gly Gly Tyr Asp Asp Phe Gln Glu Glu
    1325                1330                1335

Gly Ser Phe Glu Phe Gly Asn Met Lys Ala Thr Ser Asn Thr Leu
    1340                1345                1350

Glu Glu Phe Glu His Gly Arg Thr Pro Ala Glu Met Ser Arg Pro
    1355                1360                1365

Ala Thr Thr Thr Arg Asn Gly Phe Met Glu Ala Gln Gly Ala Gly
    1370                1375                1380

Ile Gln Ile Ile Met Gln Ala Asp Leu Ala Leu Lys Met Gly Val
    1385                1390                1395

Pro Ile Tyr Gly Ile Val Ala Met Ala Ala Thr Ala Thr Asp Lys
    1400                1405                1410

Ile Gly Arg Ser Val Pro Ala Pro Gly Lys Gly Ile Leu Thr Thr
    1415                1420                1425

Ala Arg Glu His His Ser Ser Val Lys Tyr Ala Ser Pro Asn Leu
    1430                1435                1440

Asn Met Lys Tyr Arg Lys Arg Gln Leu Val Thr Arg Glu Ala Gln
    1445                1450                1455

Ile Lys Asp Trp Val Glu Asn Glu Leu Glu Ala Leu Lys Leu Glu
    1460                1465                1470

Ala Glu Glu Ile Pro Ser Glu Asp Gln Asn Glu Phe Leu Leu Glu
    1475                1480                1485

Arg Thr Arg Glu Ile His Asn Glu Ala Asp Ser Gln Leu Arg Ala
    1490                1495                1500

Ala Gln Gln Gln Trp Gly Asn Asp Phe Tyr Lys Arg Asp Pro Arg
    1505                1510                1515

Ile Ala Pro Leu Arg Gly Ala Leu Ala Thr Tyr Gly Leu Thr Ile
    1520                1525                1530

Asp Asp Leu Gly Val Ala Ser Phe His Gly Thr Ser Thr Lys Ala
    1535                1540                1545

Asn Asp Lys Asn Glu Ser Ala Thr Ile Asn Glu Met Met Lys His
    1550                1555                1560

Leu Gly Arg Ser Glu Gly Asn Pro Val Ile Gly Val Phe Gln Lys
    1565                1570                1575

Phe Leu Thr Gly His Pro Lys Gly Ala Ala Gly Ala Trp Met Met
    1580                1585                1590

Asn Gly Ala Leu Gln Ile Leu Asn Ser Gly Ile Ile Pro Gly Asn
    1595                1600                1605

Arg Asn Ala Asp Asn Val Asp Lys Ile Leu Glu Gln Phe Glu Tyr
    1610                1615                1620

Val Leu Tyr Pro Ser Lys Thr Leu Lys Thr Asp Gly Val Arg Ala
    1625                1630                1635

Val Ser Ile Thr Ser Phe Gly Phe Gly Gln Lys Gly Gly Gln Ala
    1640                1645                1650

Ile Val Val His Pro Asp Tyr Leu Tyr Gly Ala Ile Thr Glu Asp
    1655                1660                1665

Arg Tyr Asn Glu Tyr Val Ala Lys Val Ser Ala Arg Glu Lys Ser
    1670                1675                1680

Ala Tyr Lys Phe Phe His Asn Gly Met Ile Tyr Asn Lys Leu Phe
    1685                1690                1695

Val Ser Lys Glu His Ala Pro Tyr Thr Asp Glu Leu Glu Glu Asp
    1700                1705                1710

Val Tyr Leu Asp Pro Leu Ala Arg Val Ser Lys Asp Lys Lys Ser
    1715                1720                1725

Gly Ser Leu Thr Phe Asn Ser Lys Asn Ile Gln Ser Lys Asp Ser
    1730                1735                1740

Tyr Ile Asn Ala Asn Thr Ile Glu Thr Ala Lys Met Ile Glu Asn
    1745                1750                1755

Met Thr Lys Glu Lys Val Ser Asn Gly Gly Val Gly Val Asp Val
    1760                1765                1770

Glu Leu Ile Thr Ser Ile Asn Val Glu Asn Asp Thr Phe Ile Glu
    1775                1780                1785

Arg Asn Phe Thr Pro Gln Glu Ile Glu Tyr Cys Asn Ala Gln Pro
    1790                1795                1800

Ser Val Gln Ser Ser Phe Ala Gly Thr Trp Ser Ala Lys Glu Ala
    1805                1810                1815

Val Phe Lys Ser Leu Gly Val Lys Ser Leu Gly Gly Gly Ala Ala
    1820                1825                1830

Leu Lys Asp Ile Glu Ile Val Arg Val Asn Lys Asn Ala Pro Ala
    1835                1840                1845

Val Glu Leu His Gly Asn Ala Lys Lys Ala Ala Glu Glu Ala Gly
    1850                1855                1860

Val Thr Asp Val Lys Val Ser Ile Ser His Asp Asp Leu Gln Ala

1865                1870                1875

Val Ala Val Ala Val Ser Thr Lys Lys
    1880                1885

<210>  10
<211>  5553
<212>  DNA
<213>  Yarrowia lipolytica

<400>  10
atgcaccccg aagtcgaaca agaactcgcc cacgtgctcc tgacggagct gctggcctac      60

caatttgcct cgcccgtgcg atggatcgag acccaggacg tgctgttcaa gcagttcaat     120

gtcgagcgag tcgtcgaagt cggcccatcc ccaactctcg ccggcatggc ccagcgaacc     180

cttaagtcca agtacgagtc atacgacgct gctctgtctc tgcagcgaga gatcctgtgt     240

tactccaagg accagaagga catctactac cttgccgatg aggccgatga agcccctgcc     300

cccgctgctg gtggtgatgc ccccgctgct cctgccgctg ccgctcctgc cgccgctgcc     360

gctcctgctg ccgctgccgc cccctctggc cccgttgcca aggttgagga cgcccccgtc     420

aaggcccagg agattctcca cgccctggtc gcccataagc tcaagaagac ccccgagcag     480

gtgcccctgt ccaaggccat caaagacctt gttggtggta agtctaccat ccagaacgag     540

attctcggtg atctcggaaa ggaatttggt gccacccctg agaagcccga ggatactccc     600

cttggcgagc tggctgagtc cttccaggcc tcctttgacg gcaagctcgg taagcagtct     660

tcttctctca ttgcccgact catgtcctcc aagatgcccg gagggttctc tctcacctct     720

gctcgatcct acctcgacag cagatggggc ctggctgctg gccgacagga ctccgttctg     780

cttgttgctc tgatgaacga acccaagaac cgacttggct ctgaagccga ggccaaggcc     840

tacctcgacg agcagaccca gaagtatgct gcttctgccg gtcttaacct gtctgccccc     900

gctggtggtg ccgagggtgg caatggcggt ggcgccgtca ttgactccgc tgcctttgac     960

gctctcacca aggaccagcg atacctggtc cagcagcaac tcgagttgtt tgccaactac    1020

ctgaagcagg atctgcgaca gggctccaag gtggctgctg cccagaagga ggccatggat    1080

attctgcaag ctgaactgga tctttggaac tccgagcacg gcgaggtcta cgctgagggc    1140

atcaagcccg ccttctctgc cctgaaggcc cgtgtctacg actcgtactg gaactgggct    1200

cgacaggact cgctctccat gtactttgac attgttttcg gtcgtctctc caccgttgac    1260

cgagagatta tggctaagtg tatccacctg atgaaccgaa ccaaccacaa cctgatcgac    1320

tacatgcagt accacatgga ccacgtcccc gttcacaagg gagccaccta cgagcttgcc    1380

aagcagctcg gtctgcagct cctcgagaac tgtaaggaga ctctcaccga ggcccccgtc    1440

tacaaggatg tctcttaccc cactggaccc cagaccacca ttgatgtcaa gggtaacatt    1500

gtttacaacg aggtgccccg acccaatgtc cgaaagctcg agcagtatgt ccacgagatg 1560
gcctgtggtg gtgagctgac caaggacccc tcttttgttg gagaaggtgt ccagggcgag 1620
ctcaagaagc tgtactctca gatctctgct cttgccaaga cccagaccgg ctctaccctc 1680
gacatcgagg ctctgtactc cgacctggtc gctaagatct cccaggccga ggacgcgtcc 1740
aagcctgtcg ttgagaacaa ggctgtttct gcctccatca ctcccggcac tctccctttt 1800
ctccacatca agaagaagac cgaacttggt gcctggaatt acgacagcga gaccaccgcc 1860
acctacctcg atggtcttga ggttgctgcc cgtgatggtc tcactttcca gggcaagact 1920
gctctgatca ccggtgctgg tgctggctcc attggtgcct caatcctcca gggtctcatt 1980
tccggaggct gcaaagtcat tgtcacaacc tctcgatact cccgaaaggt gaccgagtac 2040
taccagtccc tctacaccaa gttcggtgct aagggttcca ctctgattgt tgtccccttc 2100
aaccaaggct ccaagaagga cgtggacgag ctggtgtcgt tcatctacaa cgaccccaag 2160
aacggcggtc ttggctggga tctggacttt gttgttccct ttgctgctct gcccgagaac 2220
ggtattgagc tggagcacat tgactcaaag tccgagcttg cccatcgaat catgctcacc 2280
aacctcctgc gtctgcttgg taacgtcaag aagcagaaag tggcccattc ctacgagact 2340
cgacccgccc aggtcatgct gcccctgtcg cccaaccatg gcaacttcgg ctccgatggt 2400
ctgtactccg agtccaagat ctctctcgag actctgttca accggtggca caccgagtcc 2460
tggggctctt atctcaccat tgttggtgtg gtgattggct ggacccgagg taccggtctg 2520
atgagcgcca acaacatcac cgccgagggt ctggagcagc tcggcgtccg aaccttctcc 2580
cagactgaga tggccttttc catcatgggt ctcatgacca aggacattgt gcgactggcc 2640
cagaactccc ccgtgtgggc cgatctcaac ggtggcttcc agtacattcc cgacctcaag 2700
ggagttgttg gaaagatccg acgagacatt gtggagacct ccgagatccg acgggctgtg 2760
gctcaggaga ctgccattga acagaaggtg gtcaacggcc cccacgccga tcttccttac 2820
cagaaggtcg aggtcaagcc ccgagccaac ctcaagtttg acttccccac cctcaaatcc 2880
tacgccgagg tcaaggagct gtctcctgct ggtgatgctc tggagggtct tctggatctc 2940
tcttccgtca ttgttgtcac tggtttcgcc gaggtcggtc cttggggtaa cgcccgaacc 3000
cgatgggaca tggaggccaa cggtgtcttc tcccttgagg gtgccattga gatggcctgg 3060
atcatgggtc tgatcaagca ccacaatggt cccctgcccg gcatgcctca gtactctggc 3120
tggatcgata ccaagaccaa gcagcccgtc gatgaccgag atatcaagac caagtacgag 3180
gactacctgc ttgagcacgc cggtatccga ctcattgagc ctgagctgtt ccacggctac 3240
aaccccaaga agaagacctt cctccaggag gttattgtgg agcacgatct cgagcccttt 3300
gaggcctcca aggagtctgc tgagcaattt gctctcgagc agggcgcgaa cgttgagatc 3360
ttcgccgtcc ccgagtccga ccagtggact gtgcgacttc tcaagggcgc caagctcctc 3420

attcccaagg ccctcaagtt tgaccgactt gtggccggcc agattcccac tggatgggat 3480

gcccgacgat acggtattcc cgaggacatt tgtgaccagg ttgaccccat cactctgtac 3540

gctcttgtct ccactgttga ggctctgttg gcctccggta ttaccgaccc ctacgagttc 3600

tacaagtacg tccacgtgtc cgaggtcggt aactgttccg gttccggtat gggtggtatc 3660

accgccctgc gaggcatgtt caaggaccgg ttcatggaca agcctgttca gaacgatatt 3720

ctccaggagt ccttcatcaa caccatgtct gcctgggtca acatgttgct gctctcctct 3780

tccggtccca tcaagacccc cgttggagct tgtgccactg ctgtcgagtc tgtggacatt 3840

ggttgcgaaa ccattctgtc cggcaaggcc agaatctgtc tggtcggtgg ttacgatgat 3900

ttccaggagg agtcttctca ggagtttgca aacatgaacg caacatccaa cgctgagacc 3960

gagatcactc acggccgaac tccggccgag atgtctcgac ccatcacttc cacacgagcc 4020

ggtttcatgg aggctcaggg tgctggaacc caggtgctga tggccgccga cctcgccatc 4080

gccatgggtg tgcccatcta ctgtatcgtt ggttacgtca acactgccac cgacaagatt 4140

ggccgatctg tgcctgctcc cggtaagggt atcctgacca ctgctcgaga gcaccagact 4200

ctcaaacacg ccaaccctct cctcaacatc aagtaccgaa agcgacagct cgattctcga 4260

ctccgagaca ttaagcgatg ggctgagggc gaaatggagg ctattgacat tgagcttgac 4320

gacgtgtctg acgccgacaa ggagtccttc atccaggagc gatctgccca catccagtct 4380

cagtccgatc gaatgatccg agaggctaag aactcttggg gtaacgcctt tttcaagcag 4440

gacgcccgaa tctcccccat ccgaggagcg ctggcaacct acggtctcac cattgatgac 4500

atctccgtcg cttctttcca tggtacatcc accaaggcca acgagaagaa cgagaccacc 4560

accgtcaacg ccatgctgga gcatctcggc agaacccggg gtaaccctgt ctacggtatc 4620

ttccagaagt accttactgg tcaccccaag ggagctgctg gtgcctggat gctcaacgga 4680

gccatccaat gcctcaactc tggtatcatc cctggtaacc gaaacgccga taacgtggat 4740

gcctactttg agcagtgcca gcacgtggtg ttcccctcgc gatctctgca gaccgatggc 4800

ctcaaggctg cttccgtgac ctcctttggt ttcggtcaga agggtgccca ggccattgtc 4860

atccaccccg actacctgta cgctgccctg acaccctccg agtactccga gtacaccacc 4920

cgagtcgccc agcgatacaa gaaggcttac cgatactacc acaacgccat tgccgaggag 4980

tccatgttcc aggccaagga caaggctccc tactctgctg agctggagca ggaggtctac 5040

ctggatcctc ttgtgcgagt ccaccagaac gaggacaccg agcagtactc cttcaacgcc 5100

aaggacctcg ctgcctccgc ctttgtcaag aactcccaca aggacaccgc caaggtgctt 5160

gccaacctca cctcccaggt gtccggttct ggtaagaacg ttggtgtcga cgttgaggcc 5220

atctccgcca tcaacattga taacgacacc ttccttgacc gaaacttcac cgccaacgag 5280

caggcctact gcttcaaggc cccctccccc cagtcttctt tcgctggcac ttggtctgcc 5340

aaggaggctg ttttcaagtc tctgggcgtc aagtcccagg gcggaggagc tgagctcaag 5400

tccattgaga tcactcgaga tggcaacgga gctcccgtcg tggttcttca cggagctgcc 5460

aaggacgctg ctgcttctaa gggtatctcc accgtcaagg tgtccatttc ccatgacgac 5520

tctcaggccg tggctgttgc tgttgccgag tag 5553

<210> 11
<211> 1860
<212> PRT
<213> Artificial Sequence

<220>
<223> alpha FAS consensus sequence

<220>
<221> Variant
<222> (94)..(94)
<223> Replace = nothing

<220>
<221> Variant
<222> (96)..(96)
<223> Replace = Ser, Glu or Thr

<220>
<221> Variant
<222> (97)..(97)
<223> Replace = Ala or Glu

<220>
<221> Variant
<222> (101)..(101)
<223> Replace = Glu

<220>
<221> Variant
<222> (102)..(102)
<223> Replace = Pro

<220>
<221> Variant
<222> (103)..(103)
<223> Replace =Pro or Ser

<220>
<221> Variant
<222> (104)..(104)
<223> Replace = nothing

<220>
<221> Variant
<222> (105)..(105)
<223> Replace = nothing

<220>
<221> Variant
<222> (106)..(106)

<223>  Replace = Ser or nothi

<220>
<221>  Variant
<222>  (106)..(106)
<223>  Replace = Ser or nothing

<220>
<221>  Variant
<222>  (107)..(107)
<223>  Replace = Ser, Thr or nothing

<220>
<221>  Variant
<222>  (108)..(108)
<223>  Replace = Glu

<220>
<221>  Variant
<222>  (109)..(109)
<223>  Replace = nothing

<220>
<221>  Variant
<222>  (110)..(110)
<223>  Replace = Ala

<220>
<221>  Variant
<222>  (111)..(111)
<223>  Replace = Pro

<220>
<221>  Variant
<222>  (112)..(112)
<223>  Replace = Ala or Gly

<220>
<221>  Variant
<222>  (113)..(113)
<223>  Replace = nothing

<220>
<221>  Variant
<222>  (114)..(114)
<223>  Replace = nothing

<220>
<221>  Variant
<222>  (115)..(115)
<223>  Replace = Ser, Asp or nothing

<220>
<221>  Variant
<222>  (116)..(116)
<223>  Replace = Pro or Ser

<220>
<221>  Variant
<222>  (117)..(117)
<223>  Replace = Pro, Ser or Thr

<220>

<221>  Variant
<222>  (118)..(118)
<223>  Replace = Ala or Val

<220>
<221>  Variant
<222>  (119)..(119)
<223>  Replace = Ala

<220>
<221>  Variant
<222>  (121)..(121)
<223>  Replace = Ala or Thr

<220>
<221>  Variant
<222>  (122)..(122)
<223>  Replace = Pro, Thr or His

<220>
<221>  Variant
<222>  (123)..(123)
<223>  Replace = Pro

<220>
<221>  Variant
<222>  (124)..(124)
<223>  Replace = Val or nothing

<220>
<221>  Variant
<222>  (125)..(125)
<223>  Replace = Ala

<220>
<221>  Variant
<222>  (127)..(127)
<223>  Replace = Ala

<220>
<221>  Variant
<222>  (130)..(130)
<223>  Replace = nothing

<220>
<221>  Variant
<222>  (131)..(131)
<223>  Replace = nothing

<220>
<221>  Variant
<222>  (132)..(132)
<223>  Replace = Val or nothing

<220>
<221>  Variant
<222>  (133)..(133)
<223>  Replace = nothing

<220>
<221>  Variant
<222>  (141)..(141)
<223>  Replace = Val

<220>
<221>  Variant
<222>  (145)..(145)
<223>  Replace = Glu

<220>
<221>  Variant
<222>  (147)..(147)
<223>  Replace = Ala

<220>
<221>  Variant
<222>  (152)..(152)
<223>  Replace = Lys or Val

<220>
<221>  Variant
<222>  (166)..(166)
<223>  Replace = Thr

<220>
<221>  Variant
<222>  (168)..(168)
<223>  Replace = Glu

<220>
<221>  Variant
<222>  (170)..(170)
<223>  Replace = Ile

<220>
<221>  Variant
<222>  (172)..(172)
<223>  Replace = Met

<220>
<221>  Variant
<222>  (211)..(211)
<223>  Replace = Ala or Asp

<220>
<221>  Variant
<222>  (249)..(249)
<223>  Replace = Thr

<220>
<221>  Variant
<222>  (252)..(252)
<223>  Replace = Thr or Gly

<220>
<221>  Variant
<222>  (255)..(255)
<223>  Replace = Asp

<220>
<221>  Variant
<222>  (256)..(256)
<223>  Replace = Asp or Thr

<220>
<221>  Variant

<222>  (261)..(261)
<223>  Replace = Ala

<220>
<221>  Variant
<222>  (262)..(262)
<223>  Replace = Ala

<220>
<221>  Variant
<222>  (271)..(271)
<223>  Replace = Met

<220>
<221>  Variant
<222>  (281)..(281)
<223>  Replace = Phe

<220>
<221>  Variant
<222>  (282)..(282)
<223>  Replace = Ala

<220>
<221>  Variant
<222>  (285)..(285)
<223>  Replace = Ala

<220>
<221>  Variant
<222>  (286)..(286)
<223>  Replace = Glu

<220>
<221>  Variant
<222>  (301)..(301)
<223>  Replace = Gly

<220>
<221>  Variant
<222>  (304)..(304)
<223>  Replace = Leu

<220>
<221>  Variant
<222>  (307)..(307)
<223>  Replace = Ala

<220>
<221>  Variant
<222>  (308)..(308)
<223>  Replace = Ala or Thr

<220>
<221>  Variant
<222>  (310)..(310)
<223>  Replace = Ala or Thr

<220>
<221>  Variant
<222>  (311)..(311)
<223>  Replace = nothing

<220>
<221>  Variant
<222>  (312)..(312)
<223>  Replace = Ala

<220>
<221>  Variant
<222>  (313)..(313)
<223>  Replace = Ala

<220>
<221>  Variant
<222>  (317)..(317)
<223>  Replace = Asn

<220>
<221>  Variant
<222>  (319)..(319)
<223>  Replace = Gly

<220>
<221>  Variant
<222>  (356)..(356)
<223>  Replace = Ser

<220>
<221>  Variant
<222>  (358)..(358)
<223>  Replace = Phe

<220>
<221>  Variant
<222>  (370)..(370)
<223>  Replace = Ile

<220>
<221>  Variant
<222>  (380)..(380)
<223>  Replace = Ala

<220>
<221>  Variant
<222>  (385)..(385)
<223>  Replace = Ile

<220>
<221>  Variant
<222>  (388)..(388)
<223>  Replace = Glu

<220>
<221>  Variant
<222>  (396)..(396)
<223>  Replace = Ala

<220>
<221>  Variant
<222>  (446)..(446)
<223>  Replace = Thr

<220>
<221>  Variant
<222>  (461)..(461)

<223>  Replace = His

<220>
<221>  Variant
<222>  (486)..(486)
<223>  Replace = Val

<220>
<221>  Variant
<222>  (487)..(487)
<223>  Replace = Ala

<220>
<221>  Variant
<222>  (527)..(527)
<223>  Replace = Ala or Asn

<220>
<221>  Variant
<222>  (531)..(531)
<223>  Replace = Ser, Cys or Val

<220>
<221>  Variant
<222>  (536)..(536)
<223>  Replace = Arg

<220>
<221>  Variant
<222>  (540)..(540)
<223>  Replace = Thr

<220>
<221>  Variant
<222>  (541)..(541)
<223>  Replace = Tyr

<220>
<221>  Variant
<222>  (542)..(542)
<223>  Replace = Ala or Val

<220>
<221>  Variant
<222>  (544)..(544)
<223>  Replace = Glu

<220>
<221>  Variant
<222>  (554)..(554)
<223>  Replace = Tyr

<220>
<221>  Variant
<222>  (555)..(555)
<223>  Replace = Ala

<220>
<221>  Variant
<222>  (559)..(559)
<223>  Replace = Gln

<220>

<221>  Variant
<222>  (560)..(560)
<223>  Replace = Leu

<220>
<221>  Variant
<222>  (563)..(563)
<223>  Replace = nothing

<220>
<221>  Variant
<222>  (564)..(564)
<223>  Replace = Gln

<220>
<221>  Variant
<222>  (565)..(565)
<223>  Replace = Thr

<220>
<221>  Variant
<222>  (567)..(567)
<223>  Replace = Ser

<220>
<221>  Variant
<222>  (568)..(568)
<223>  Replace = Thr

<220>
<221>  Variant
<222>  (570)..(570)
<223>  Replace = Lys or Asn

<220>
<221>  Variant
<222>  (573)..(573)
<223>  Replace = Glu

<220>
<221>  Variant
<222>  (574)..(574)
<223>  Replace = Leu

<220>
<221>  Variant
<222>  (576)..(576)
<223>  Replace = Ala

<220>
<221>  Variant
<222>  (580)..(580)
<223>  Replace = Ala

<220>
<221>  Variant
<222>  (581)..(581)
<223>  Replace = Arg or Glu

<220>
<221>  Variant
<222>  (583)..(583)
<223>  Replace = Arg or Gly

<220>
<221>  Variant
<222>  (584)..(584)
<223>  Replace = Lys

<220>
<221>  Variant
<222>  (585)..(585)
<223>  Replace = Ala or Val

<220>
<221>  Variant
<222>  (587)..(587)
<223>  Replace = Thr

<220>
<221>  Variant
<222>  (588)..(588)
<223>  Replace = Asp or Val

<220>
<221>  Variant
<222>  (589)..(589)
<223>  Replace = Thr, Asn or nothing

<220>
<221>  Variant
<222>  (590)..(590)
<223>  Replace = nothing

<220>
<221>  Variant
<222>  (591)..(591)
<223>  Replace = Ser

<220>
<221>  Variant
<222>  (592)..(592)
<223>  Replace = nothing

<220>
<221>  Variant
<222>  (593)..(593)
<223>  Replace = Ala, Lys or Asp

<220>
<221>  Variant
<222>  (594)..(594)
<223>  Replace = Ser

<220>
<221>  Variant
<222>  (595)..(595)
<223>  Replace = Ala, Lys, Glu or Asp

<220>
<221>  Variant
<222>  (596)..(596)
<223>  Replace = Asn

<220>
<221>  Variant

<222>  (598)..(598)
<223>  Replace = Ala or Thr

<220>
<221>  Variant
<222>  (601)..(601)
<223>  Replace = Ala or Asp

<220>
<221>  Variant
<222>  (602)..(602)
<223>  Replace = Ala, Gln or Val

<220>
<221>  Variant
<222>  (603)..(603)
<223>  Replace = Thr

<220>
<221>  Variant
<222>  (604)..(604)
<223>  Replace = Pro or Val

<220>
<221>  Variant
<222>  (615)..(615)
<223>  Replace = Thr

<220>
<221>  Variant
<222>  (626)..(626)
<223>  Replace = Ala

<220>
<221>  Variant
<222>  (627)..(627)
<223>  Replace = Gln, Pro or Lys

<220>
<221>  Variant
<222>  (630)..(630)
<223>  Replace = Ala

<220>
<221>  Variant
<222>  (631)..(631)
<223>  Replace = Met

<220>
<221>  Variant
<222>  (647)..(647)
<223>  Replace = Gln, Ala or Lys

<220>
<221>  Variant
<222>  (669)..(669)
<223>  Replace = Met

<220>
<221>  Variant
<222>  (693)..(693)
<223>  Replace = Gly

<220>
<221>  Variant
<222>  (696)..(696)
<223>  Replace = Arg

<220>
<221>  Variant
<222>  (697)..(697)
<223>  Replace = Tyr

<220>
<221>  Variant
<222>  (716)..(716)
<223>  Replace = Arg

<220>
<221>  Variant
<222>  (724)..(724)
<223>  Replace = Tyr

<220>
<221>  Variant
<222>  (727)..(727)
<223>  Replace = Asp

<220>
<221>  Variant
<222>  (728)..(728)
<223>  Replace = Glu

<220>
<221>  Variant
<222>  (731)..(731)
<223>  Replace = Val

<220>
<221>  Variant
<222>  (742)..(742)
<223>  Replace = Ile

<220>
<221>  Variant
<222>  (755)..(755)
<223>  Replace = Glu

<220>
<221>  Variant
<222>  (787)..(787)
<223>  Replace = His

<220>
<221>  Variant
<222>  (796)..(796)
<223>  Replace = Met

<220>
<221>  Variant
<222>  (799)..(799)
<223>  Replace = Met

<220>
<221>  Variant
<222>  (805)..(805)

<223>  Replace = Ile

<220>
<221>  Variant
<222>  (828)..(828)
<223>  Replace = Thr

<220>
<221>  Variant
<222>  (863)..(863)
<223>  Replace = Lys

<220>
<221>  Variant
<222>  (884)..(884)
<223>  Replace = Lys

<220>
<221>  Variant
<222>  (893)..(893)
<223>  Replace = Ala

<220>
<221>  Variant
<222>  (911)..(911)
<223>  Replace = Asp

<220>
<221>  Variant
<222>  (914)..(914)
<223>  Replace = Gly

<220>
<221>  Variant
<222>  (915)..(915)
<223>  Replace = Asn

<220>
<221>  Variant
<222>  (919)..(919)
<223>  Replace = Glu

<220>
<221>  Variant
<222>  (955)..(955)
<223>  Replace = Ile

<220>
<221>  Variant
<222>  (971)..(971)
<223>  Replace = His

<220>
<221>  Variant
<222>  (972)..(972)
<223>  Replace = Gln or Ser

<220>
<221>  Variant
<222>  (974)..(974)
<223>  Replace = Ile

<220>

<221>  Variant
<222>  (977)..(977)
<223>  Replace = Phe

<220>
<221>  Variant
<222>  (983)..(983)
<223>  Replace = Ala or Asp

<220>
<221>  Variant
<222>  (1039)..(1039)
<223>  Replace = Asn

<220>
<221>  Variant
<222>  (1043)..(1043)
<223>  Replace = Pro

<220>
<221>  Variant
<222>  (1045)..(1045)
<223>  Replace = Ile, Lys, Leu or nothing

<220>
<221>  Variant
<222>  (1046)..(1046)
<223>  Replace = Gln or Lys

<220>
<221>  Variant
<222>  (1047)..(1047)
<223>  Replace = Lys

<220>
<221>  Variant
<222>  (1054)..(1054)
<223>  Replace = Thr

<220>
<221>  Variant
<222>  (1056)..(1056)
<223>  Replace = Thr

<220>
<221>  Variant
<222>  (1058)..(1058)
<223>  Replace = Lys

<220>
<221>  Variant
<222>  (1067)..(1067)
<223>  Replace = Thr

<220>
<221>  Variant
<222>  (1068)..(1068)
<223>  Replace = Asn

<220>
<221>  Variant
<222>  (1071)..(1071)
<223>  Replace = Glu

<220>
<221>  Variant
<222>  (1075)..(1075)
<223>  Replace = Glu

<220>
<221>  Variant
<222>  (1077)..(1077)
<223>  Replace = Ala

<220>
<221>  Variant
<222>  (1084)..(1084)
<223>  Replace = Ser

<220>
<221>  Variant
<222>  (1088)..(1088)
<223>  Replace = Gln, Ser or Asn

<220>
<221>  Variant
<222>  (1091)..(1091)
<223>  Replace = Asp

<220>
<221>  Variant
<222>  (1147)..(1147)
<223>  Replace = Ala

<220>
<221>  Variant
<222>  (1191)..(1191)
<223>  Replace = Ala

<220>
<221>  Variant
<222>  (1270)..(1270)
<223>  Replace = Ala

<220>
<221>  Variant
<222>  (1332)..(1332)
<223>  Replace = Met or Leu

<220>
<221>  Variant
<222>  (1333)..(1333)
<223>  Replace = Ala or Thr

<220>
<221>  Variant
<222>  (1350)..(1350)
<223>  Replace = Ala

<220>
<221>  Variant
<222>  (1362)..(1362)
<223>  Replace = Ile

<220>
<221>  Variant

<222>  (1363)..(1363)
<223>  Replace = Leu

<220>
<221>  Variant
<222>  (1371)..(1371)
<223>  Replace = Thr

<220>
<221>  Variant
<222>  (1378)..(1378)
<223>  Replace = Ala

<220>
<221>  Variant
<222>  (1414)..(1414)
<223>  Replace = Ala

<220>
<221>  Variant
<222>  (1419)..(1419)
<223>  Replace = Asn

<220>
<221>  Variant
<222>  (1420)..(1420)
<223>  Replace = Ile

<220>
<221>  Variant
<222>  (1421)..(1421)
<223>  Replace = Arg

<220>
<221>  Variant
<222>  (1428)..(1428)
<223>  Replace = Glu

<220>
<221>  Variant
<222>  (1429)..(1429)
<223>  Replace = Ala or Thr

<220>
<221>  Variant
<222>  (1432)..(1432)
<223>  Replace = Gln

<220>
<221>  Variant
<222>  (1438)..(1438)
<223>  Replace = Ala

<220>
<221>  Variant
<222>  (1440)..(1440)
<223>  Replace = Ala

<220>
<221>  Variant
<222>  (1446)..(1446)
<223>  Replace = Glu or Asn

<220>
<221>  Variant
<222>  (1447)..(1447)
<223>  Replace = Ala or Met

<220>
<221>  Variant
<222>  (1450)..(1450)
<223>  Replace = Glu

<220>
<221>  Variant
<222>  (1451)..(1451)
<223>  Replace = Glu

<220>
<221>  Variant
<222>  (1452)..(1452)
<223>  Replace = Ile

<220>
<221>  Variant
<222>  (1453)..(1453)
<223>  Replace = Ala or Asp

<220>
<221>  Variant
<222>  (1454)..(1454)
<223>  Replace = Ala or Glu

<220>
<221>  Variant
<222>  (1455)..(1455)
<223>  Replace = Glu or Asn

<220>
<221>  Variant
<222>  (1456)..(1456)
<223>  Replace = Glu

<220>
<221>  Variant
<222>  (1457)..(1457)
<223>  Replace = Arg

<220>
<221>  Variant
<222>  (1458)..(1458)
<223>  Replace = Lys

<220>
<221>  Variant
<222>  (1462)..(1462)
<223>  Replace = Gln

<220>
<221>  Variant
<222>  (1469)..(1469)
<223>  Replace = Ala, His or Thr

<220>
<221>  Variant
<222>  (1472)..(1472)

<223>  Replace = Thr

<220>
<221>  Variant
<222>  (1473)..(1473)
<223>  Replace = Glu

<220>
<221>  Variant
<222>  (1476)..(1476)
<223>  Replace = Met, Thr or Val

<220>
<221>  Variant
<222>  (1486)..(1486)
<223>  Replace = Ala

<220>
<221>  Variant
<222>  (1488)..(1488)
<223>  Replace = Tyr

<220>
<221>  Variant
<222>  (1489)..(1489)
<223>  Replace = Arg

<220>
<221>  Variant
<222>  (1492)..(1492)
<223>  Replace = Pro

<220>
<221>  Variant
<222>  (1591)..(1591)
<223>  Replace = Val

<220>
<221>  Variant
<222>  (1597)..(1597)
<223>  Replace = His

<220>
<221>  Variant
<222>  (1606)..(1606)
<223>  Replace = Met

<220>
<221>  Variant
<222>  (1641)..(1641)
<223>  Replace = Thr

<220>
<221>  Variant
<222>  (1642)..(1642)
<223>  Replace = Pro or Ser

<220>
<221>  Variant
<222>  (1646)..(1646)
<223>  Replace = Ala

<220>

<221>  Variant
<222>  (1647)..(1647)
<223>  Replace = Asp or Gly

<220>
<221>  Variant
<222>  (1649)..(1649)
<223>  Replace = Ala or Val

<220>
<221>  Variant
<222>  (1650)..(1650)
<223>  Replace = Ser or Asn

<220>
<221>  Variant
<222>  (1653)..(1653)
<223>  Replace = Ala or Asp

<220>
<221>  Variant
<222>  (1665)..(1665)
<223>  Replace = Thr

<220>
<221>  Variant
<222>  (1682)..(1682)
<223>  Replace = Ala

<220>
<221>  Variant
<222>  (1687)..(1687)
<223>  Replace = Lys

<220>
<221>  Variant
<222>  (1700)..(1700)
<223>  Replace = Asn

<220>
<221>  Variant
<222>  (1701)..(1701)
<223>  Replace = Lys or Asp

<220>
<221>  Variant
<222>  (1708)..(1708)
<223>  Replace = Tyr

<220>
<221>  Variant
<222>  (1709)..(1709)
<223>  Replace = Ser or Asp

<220>
<221>  Variant
<222>  (1715)..(1715)
<223>  Replace = Ala

<220>
<221>  Variant
<222>  (1717)..(1717)
<223>  Replace = Ala

<220>
<221>  Variant
<222>  (1721)..(1721)
<223>  Replace = Ser or Asp

<220>
<221>  Variant
<222>  (1722)..(1722)
<223>  Replace = Asn

<220>
<221>  Variant
<222>  (1732)..(1732)
<223>  Replace = Thr

<220>
<221>  Variant
<222>  (1738)..(1738)
<223>  Replace = Ala

<220>
<221>  Variant
<222>  (1739)..(1739)
<223>  Replace = Gly

<220>
<221>  Variant
<222>  (1740)..(1740)
<223>  Replace = Gly

<220>
<221>  Variant
<222>  (1741)..(1741)
<223>  Replace = nothing

<220>
<221>  Variant
<222>  (1742)..(1742)
<223>  Replace = nothing

<220>
<221>  Variant
<222>  (1768)..(1768)
<223>  Replace = Glu or Thr

<220>
<221>  Variant
<222>  (1769)..(1769)
<223>  Replace = Glu or Thr

<220>
<221>  Variant
<222>  (1776)..(1776)
<223>  Replace = Arg

<220>
<221>  Variant
<222>  (1780)..(1780)
<223>  Replace = Pro

<220>
<221>  Variant

<222>  (1810)..(1810)
<223>  Replace = Arg

<220>
<221>  Variant
<222>  (1815)..(1815)
<223>  Replace = Thr

<220>
<221>  Variant
<222>  (1818)..(1818)
<223>  Replace = Ala or Asp

<220>
<221>  Variant
<222>  (1820)..(1820)
<223>  Replace = Lys

<220>
<221>  Variant
<222>  (1823)..(1823)
<223>  Replace = Gln

<220>
<221>  Variant
<222>  (1829)..(1829)
<223>  Replace = Asp or Val

<220>
<221>  Variant
<222>  (1832)..(1832)
<223>  Replace = Ala or Glu

<220>
<221>  Variant
<222>  (1835)..(1835)
<223>  Replace = Ser or Glu

<220>
<221>  Variant
<222>  (1836)..(1836)
<223>  Replace = Ala

<220>
<221>  Variant
<222>  (1837)..(1837)
<223>  Replace = Arg

<220>
<221>  Variant
<222>  (1839)..(1839)
<223>  Replace = Leu

<220>
<221>  Variant
<222>  (1840)..(1840)
<223>  Replace = Thr

<220>
<221>  Variant
<222>  (1841)..(1841)
<223>  Replace = Thr

<220>
<221>  Variant
<222>  (1851)..(1851)
<223>  Replace = Thr

<400>  11
   Met His Pro Glu Val Glu Gln Glu Leu Ala His Val Leu Leu Thr Glu
   1               5                   10                  15
   Leu Leu Ala Tyr Gln Phe Ala Ser Pro Val Arg Trp Ile Glu Thr Gln
               20                  25                  30
   Asp Val Leu Phe Lys Gln Phe Asn Val Glu Arg Val Val Glu Val Gly
           35                  40                  45
   Pro Ser Pro Thr Leu Ala Gly Met Ala Gln Arg Thr Leu Lys Ser Lys
       50                  55                  60
   Tyr Glu Ser Tyr Asp Ala Ala Leu Ser Leu Gln Arg Glu Ile Leu Cys
   65                  70                  75                  80
   Tyr Ser Lys Asp Gln Lys Asp Ile Tyr Tyr Leu Ala Asp Glu Glu Ala
                   85                  90                  95
   Asp Glu Ala Pro Ala Ala Ala Ala Ser Gly Gly Asp Ala Ser Ala Ser
               100                 105                 110
   Ala Asp Ala Ala Ala Pro Ser Ala Ser Ala Ala Ala Ser Ser Ser Ala
           115                 120                 125
   Pro Ala Ala Ala Ala Ala Ala Ala Ser Ser Gly Pro Ala Ala Lys Val
       130                 135                 140
   Asp Asp Ser Pro Val Lys Ala Gln Glu Ile Leu His Ala Leu Val Ala
   145                 150                 155                 160
   His Lys Leu Lys Lys Ser Pro Asp Gln Val Pro Leu Ser Lys Ala Ile
                   165                 170                 175
   Lys Asp Leu Val Gly Gly Lys Ser Thr Ile Gln Asn Glu Ile Leu Gly
               180                 185                 190
   Asp Leu Gly Lys Glu Phe Gly Ala Thr Pro Glu Lys Pro Glu Asp Thr
           195                 200                 205
   Pro Leu Gly Glu Leu Ala Glu Ser Phe Gln Ala Ser Phe Asp Gly Lys
       210                 215                 220
   Leu Gly Lys Gln Ser Ser Ser Leu Ile Ala Arg Leu Met Ser Ser Lys
   225                 230                 235                 240
   Met Pro Gly Gly Phe Ser Leu Thr Ser Ala Arg Ser Tyr Leu Gly Ser
                   245                 250                 255
   Arg Trp Gly Leu Gly Ser Gly Arg Gln Asp Ser Val Leu Leu Val Ala
               260                 265                 270
   Leu Met Asn Glu Pro Lys Asn Arg Leu Gly Ser Glu Gly Asp Ala Lys
           275                 280                 285
   Ala Tyr Leu Asp Glu Gln Thr Gln Lys Tyr Ala Ala Ser Ala Gly Ile
       290                 295                 300
   Asn Leu Ser Ser Pro Ser Gly Gly Ser Glu Gly Gly Ser Gly Ser Gly
   305                 310                 315                 320
   Ala Val Ile Asp Ser Ala Ala Phe Asp Ala Leu Thr Lys Asp Gln Arg
                   325                 330                 335
   Tyr Leu Val Gln Gln Gln Leu Glu Leu Phe Ala Asn Tyr Leu Lys Gln
               340                 345                 350
   Asp Leu Arg Gln Gly Ser Lys Val Ala Ala Ala Gln Lys Glu Ala Met
           355                 360                 365
   Asp Val Leu Gln Ala Glu Leu Asp Leu Trp Asn Ser Glu His Gly Glu
       370                 375                 380
   Val Tyr Ala Asp Gly Ile Lys Pro Ala Phe Ser Ser Leu Lys Ala Arg
   385                 390                 395                 400
   Val Tyr Asp Ser Tyr Trp Asn Trp Ala Arg Gln Asp Ser Leu Ser Met
                   405                 410                 415
   Tyr Phe Asp Ile Val Phe Gly Arg Leu Ser Thr Val Asp Arg Glu Ile
               420                 425                 430
   Met Ala Lys Cys Ile His Leu Met Asn Arg Thr Asn His Asn Leu Ile
           435                 440                 445
   Asp Tyr Met Gln Tyr His Met Asp His Val Pro Val Gln Lys Gly Ala
       450                 455                 460
   Thr Tyr Glu Leu Ala Lys Gln Leu Gly Leu Gln Leu Leu Glu Asn Cys
   465                 470                 475                 480
   Lys Glu Thr Leu Thr Glu Ser Pro Val Tyr Lys Asp Val Ser Tyr Pro
                   485                     490                     495
   Thr Gly Pro Gln Thr Thr Ile Asp Val Lys Gly Asn Ile Val Tyr Asn
               500                     505                     510
   Glu Val Pro Arg Pro Asn Val Arg Lys Leu Glu Gln Tyr Val His Glu
           515                     520                     525
   Met Ala Ala Gly Gly Glu Leu Thr Lys Asp Pro Ser Phe Thr Gly Asp
       530                     535                     540
   Gly Val Gln Gly Glu Leu Lys Lys Leu Phe Ser Gln Ile Ser Ala Ile
   545                     550                     555                     560
   Ala Lys Thr Glu Ala Gly Pro Ser Leu Asp Ile Glu Ala Ile Tyr Ser
                   565                     570                     575
   Asp Leu Val Ser Lys Ile Ser Gln Thr Glu Asp Ala Ser Lys Pro Asp
               580                     585                     590
   Val Val Gln Ser Lys Ser Val Ser Ser Ser Ile Thr Pro Gly Thr Leu
           595                     600                     605
   Pro Phe Leu His Ile Lys Lys Lys Thr Glu Leu Gly Ala Trp Asn Tyr
       610                     615                     620
   Asp Ser Glu Thr Thr Ser Thr Tyr Leu Asp Gly Leu Glu Val Ala Ala
   625                     630                     635                     640
   Arg Asp Gly Leu Thr Phe Glu Gly Lys Thr Ala Leu Ile Thr Gly Ala
                   645                     650                     655
   Gly Ala Gly Ser Ile Gly Ala Ser Ile Leu Gln Gly Leu Ile Ser Gly
               660                     665                     670
   Gly Cys Lys Val Ile Val Thr Thr Ser Arg Tyr Ser Arg Lys Val Thr
           675                     680                     685
   Glu Tyr Tyr Gln Ser Leu Tyr Thr Lys Phe Gly Ala Lys Gly Ser Thr
       690                     695                     700
   Leu Ile Val Val Pro Phe Asn Gln Gly Ser Lys Lys Asp Val Asp Glu
   705                     710                     715                     720
   Leu Val Ser Phe Ile Tyr Asn Asp Pro Lys Asn Gly Gly Leu Gly Trp
                   725                     730                     735
   Asp Leu Asp Phe Val Val Pro Phe Ala Ala Leu Pro Glu Asn Gly Ile
               740                 745                 750
   Glu Leu Asp His Ile Asp Ser Lys Ser Glu Leu Ala His Arg Ile Met
           755                 760                 765
   Leu Thr Asn Leu Leu Arg Leu Leu Gly Asn Val Lys Lys Gln Lys Val
       770                 775                 780
   Ala His Ser Tyr Glu Thr Arg Pro Ala Gln Val Leu Leu Pro Leu Ser
   785                 790                 795                 800
   Pro Asn His Gly Asn Phe Gly Ser Asp Gly Leu Tyr Ser Glu Ser Lys
                   805                 810                 815
   Ile Ser Leu Glu Thr Leu Phe Asn Arg Trp His Ser Glu Ser Trp Gly
               820                 825                 830
   Ser Tyr Leu Thr Ile Val Gly Val Val Ile Gly Trp Thr Arg Gly Thr
           835                 840                 845
   Gly Leu Met Ser Ala Asn Asn Ile Thr Ala Glu Gly Leu Glu Gln Leu
       850                 855                 860
   Gly Val Arg Thr Phe Ser Gln Thr Glu Met Ala Phe Ser Ile Met Gly
   865                 870                 875                 880
   Leu Met Thr Gln Asp Ile Val Arg Leu Ala Gln Asn Ser Pro Val Trp
                   885                 890                 895
   Ala Asp Leu Asn Gly Gly Phe Gln Tyr Ile Pro Asp Leu Lys Gly Val
               900                 905                 910
   Val Ser Lys Ile Arg Arg Asp Ile Val Glu Thr Ser Glu Ile Arg Arg
           915                 920                 925
   Ala Val Ala Gln Glu Thr Ala Ile Glu Gln Lys Val Val Asn Gly Pro
       930                 935                 940
   His Ala Asp Leu Pro Tyr Gln Lys Val Glu Val Lys Pro Arg Ala Asn
   945                 950                 955                 960
   Leu Lys Phe Asp Phe Pro Thr Leu Lys Ser Tyr Ala Glu Val Lys Glu
                   965                 970                 975
   Leu Ser Pro Ala Gly Asp Ser Leu Glu Gly Leu Leu Asp Leu Ser Ser
   980 985 990
   Val Ile Val Val Thr Gly Phe Ala Glu Val Gly Pro Trp Gly Asn Ala
   995 1000 1005
   Arg Thr Arg Trp Asp Met Glu Ala Asn Gly Val Phe Ser Leu Glu
   1010 1015 1020
   Gly Ala Ile Glu Met Ala Trp Ile Met Gly Leu Ile Lys His His
   1025 1030 1035
   Ser Gly Pro Leu Ala Gly Met Pro Gln Tyr Ser Gly Trp Ile Asp
   1040 1045 1050
   Ala Lys Ser Lys Gln Pro Val Asp Asp Arg Asp Ile Lys Asn Lys
   1055 1060 1065
   Tyr Glu Asp Tyr Leu Leu Asp His Ser Gly Ile Arg Leu Ile Glu
   1070 1075 1080
   Pro Glu Leu Phe His Gly Tyr Asn Pro Lys Lys Lys Thr Phe Leu
   1085 1090 1095
   Gln Glu Val Ile Val Glu His Asp Leu Glu Pro Phe Glu Ala Ser
   1100 1105 1110
   Lys Glu Ser Ala Glu Gln Phe Ala Leu Glu Gln Gly Ala Asn Val
   1115 1120 1125
   Glu Ile Phe Ala Val Pro Glu Ser Asp Gln Trp Thr Val Arg Leu
   1130 1135 1140
   Leu Lys Gly Ser Lys Leu Leu Ile Pro Lys Ala Leu Lys Phe Asp
   1145 1150 1155
   Arg Leu Val Ala Gly Gln Ile Pro Thr Gly Trp Asp Ala Arg Arg
   1160 1165 1170
   Tyr Gly Ile Pro Glu Asp Ile Cys Asp Gln Val Asp Pro Ile Thr
   1175 1180 1185
   Leu Tyr Cys Leu Val Ser Thr Val Glu Ala Leu Leu Ala Ser Gly
   1190 1195 1200
   Ile Thr Asp Pro Tyr Glu Phe Tyr Lys Tyr Val His Val Ser Glu
   1205 1210 1215
   Val Gly Asn Cys Ser Gly Ser Gly Met Gly Gly Ile Thr Ala Leu
       1220                1225                1230
   Arg Gly Met Phe Lys Asp Arg Phe Met Asp Lys Pro Val Gln Asn
       1235                1240                1245
   Asp Ile Leu Gln Glu Ser Phe Ile Asn Thr Met Ser Ala Trp Val
       1250                1255                1260
   Asn Met Leu Leu Leu Ser Ser Ser Gly Pro Ile Lys Thr Pro Val
       1265                1270                1275
   Gly Ala Cys Ala Thr Ala Val Glu Ser Val Asp Ile Gly Cys Glu
       1280                1285                1290
   Thr Ile Leu Ser Gly Lys Ala Arg Ile Cys Leu Val Gly Gly Tyr
       1295                1300                1305
   Asp Asp Phe Gln Glu Glu Ser Ser Gln Glu Phe Ala Asn Met Asn
       1310                1315                1320
   Ala Thr Ser Asn Ala Glu Thr Glu Ile Ser His Gly Arg Thr Pro
       1325                1330                1335
   Ala Glu Met Ser Arg Pro Ile Thr Ser Thr Arg Ser Gly Phe Met
       1340                1345                1350
   Glu Ala Gln Gly Ala Gly Thr Gln Val Ile Met Ala Ala Asp Leu
       1355                1360                1365
   Ala Ile Ala Met Gly Val Pro Ile Tyr Cys Ile Val Gly Tyr Val
       1370                1375                1380
   Asn Thr Ala Thr Asp Lys Ile Gly Arg Ser Val Pro Ala Pro Gly
       1385                1390                1395
   Lys Gly Ile Leu Thr Thr Ala Arg Glu His Gln Thr Leu Lys His
       1400                1405                1410
   Ser Asn Pro Leu Leu Ser Val Lys Tyr Arg Lys Arg Gln Leu Asp
       1415                1420                1425
   Ser Arg Leu Arg Asp Ile Lys Arg Trp Ser Glu Gly Glu Met Glu
       1430                1435                1440
   Ala Ile Asp Ile Glu Leu Asp Asp Val Ser Asp Ala Asp Lys Glu
       1445                1450                1455
   Ser Phe Ile Glu Glu Arg Ser Ala His Ile Gln Ser Gln Ser Asp
   1460 1465 1470
   Arg Met Ile Arg Glu Ala Lys Asn Ser Trp Gly Asn Ser Phe Phe
   1475 1480 1485
   Lys Gln Asp Ala Arg Ile Ser Pro Ile Arg Gly Ala Leu Ala Thr
   1490 1495 1500
   Tyr Gly Leu Thr Ile Asp Asp Ile Ser Val Ala Ser Phe His Gly
   1505 1510 1515
   Thr Ser Thr Lys Ala Asn Glu Lys Asn Glu Thr Thr Thr Val Asn
   1520 1525 1530
   Ala Met Leu Glu His Leu Gly Arg Thr Arg Gly Asn Pro Val Tyr
   1535 1540 1545
   Gly Ile Phe Gln Lys Tyr Leu Thr Gly His Pro Lys Gly Ala Ala
   1550 1555 1560
   Gly Ala Trp Met Leu Asn Gly Ala Ile Gln Cys Leu Asn Ser Gly
   1565 1570 1575
   Ile Ile Pro Gly Asn Arg Asn Ala Asp Asn Val Asp Ala Tyr Phe
   1580 1585 1590
   Glu Gln Cys Gln His Val Val Phe Pro Ser Arg Ser Leu Gln Thr
   1595 1600 1605
   Asp Gly Leu Lys Ala Ala Ser Val Thr Ser Phe Gly Phe Gly Gln
   1610 1615 1620
   Lys Gly Ala Gln Ala Ile Val Ile His Pro Asp Tyr Leu Tyr Ala
   1625 1630 1635
   Ala Leu Ser Ala Ser Glu Tyr Ser Glu Tyr Thr Thr Arg Val Gly
   1640 1645 1650
   Gln Arg Tyr Lys Lys Ala Tyr Arg Tyr Tyr His Asn Ala Ile Ala
   1655 1660 1665
   Glu Glu Ser Met Phe Gln Ala Lys Asp Lys Ala Pro Tyr Ser Ala
   1670 1675 1680
   Glu Leu Glu Gln Glu Val Tyr Leu Asp Pro Leu Val Arg Val His
   1685 1690 1695
   Gln Ser Glu Asp Thr Glu Gln Tyr Ser Phe Asn Ala Lys Asp Leu
    1700 1705 1710
   Ala Ser Ser Ser Phe Val Lys Asn Ser His Lys Asp Thr Ala Lys
    1715 1720 1725
   Val Leu Ala Asn Leu Thr Ser Gln Val Ser Ser Ser Gly Lys Asn
    1730 1735 1740
   Val Gly Val Asp Val Glu Ala Ile Ser Ala Ile Asn Ile Asp Asn
    1745 1750 1755
   Asp Thr Phe Leu Asp Arg Asn Phe Thr Ala Asn Glu Gln Ala Tyr
    1760 1765 1770
   Cys Phe Lys Ala Pro Ser Ala Gln Ser Ser Phe Ala Gly Thr Trp
    1775 1780 1785
   Ser Ala Lys Glu Ala Val Phe Lys Ser Leu Gly Val Lys Ser Gln
    1790 1795 1800
   Gly Gly Gly Ala Glu Leu Lys Ser Ile Glu Ile Ser Arg Asp Gly
    1805 1810 1815
   Asn Gly Ala Pro Val Val Val Leu His Gly Ala Ala Lys Asp Ala
    1820 1825 1830
   Ala Ala Ser Lys Gly Ile Ser Ser Val Lys Val Ser Ile Ser His
    1835 1840 1845
   Asp Asp Ser Gln Ala Val Ala Val Ala Val Ala Glu
    1850 1855 1860

<210> 12
<211> 325
<212> PRT
<213> Yarrowia lipolytica

<400> 12

Met Ser Ala Val Pro Ile Glu Phe Asn Val Pro Ser Val Asp Arg Pro
1               5                   10                  15

Phe Gly Ile Tyr Leu Trp Ala Ile Phe Asp Gln Ala Trp Glu Lys Leu
            20                  25                  30

Phe Gly Trp Pro Ala Ser Ser Phe Ile Phe Val Arg Asn Asp Pro Asn
        35                  40                  45

Ile Pro Phe Ser Ser Thr Pro Pro Val Ile Ile Ala Ile Ile Val Tyr
    50                  55                  60

Tyr Ile Val Ile Phe Gly Gly Arg Glu Val Met Arg Asn Leu Ser Pro
65                  70                  75                  80

Ile Arg Leu Asn Trp Leu Phe Gln Ile His Asn Ile Phe Leu Thr Leu
                85                  90                  95

Leu Ser Gly Met Leu Leu Leu Leu Leu Val Glu Gln Leu Phe Pro Ile
            100                 105                 110

Ile Val Arg Gln Gly Ile Leu Tyr Ala Ile Cys Asp Tyr Gly Ser Trp
        115                 120                 125

Thr Gln Pro Ile Val Phe Cys Tyr Tyr Leu Asn Tyr Leu Thr Lys Tyr
    130                 135                 140

Phe Glu Leu Ile Asp Thr Val Phe Leu Val Leu Arg Lys Lys Lys Leu
145                 150                 155                 160

Thr Phe Leu His Thr Tyr His His Gly Ala Thr Ala Leu Leu Cys Tyr
                165                 170                 175

Thr Gln Leu Ile Gly Lys Thr Ser Val Ser Trp Val Pro Ile Thr Leu
            180                 185                 190

Asn Leu Phe Val His Val Val Met Tyr Phe Tyr Tyr Phe Leu Ala Ala
        195                 200                 205

Arg Gly Ile Arg Val Trp Trp Lys Glu Trp Val Thr Arg Leu Gln Ile
    210                 215                 220

Ile Gln Phe Val Ile Asp Leu Gly Phe Val Tyr Phe Ala Ser Tyr Thr
225                 230                 235                 240

Tyr Phe Thr Ser Thr Tyr Trp Pro Trp Met Pro Asn Met Gly Ser Cys
                245                 250                 255

Ala Gly Glu Glu Phe Ala Ala Ile Tyr Gly Cys Gly Leu Leu Thr Ser
            260                 265                 270

Tyr Leu Phe Leu Phe Ile Ala Phe Tyr Ile Asn Ser Tyr Arg Lys Pro
        275                 280                 285

Ser Ser Lys Gly Pro Ser Lys Pro Val Val Ala Val Asp Gly Pro Val

290                 295                 300

Gly Gly Val Asn Ala Gln Thr Gly Ala Ser Arg Gly Gln Thr Thr Thr
305                 310                 315                 320

Arg Ser Arg Arg Ala
                325

<210>  13
<211>  304
<212>  PRT
<213>  Yarrowia lipolytica

<400>  13

Met Leu Ser Ser Ile Ser Pro Asp Leu Tyr Ser Ser Phe Ser Phe Lys
1               5                   10                  15

Asn Ser Leu Ala Glu Ala Met Pro Ser Val Pro His Glu Leu Ile Asn
            20                  25                  30

Ser Lys Thr Leu Ser Trp Met Tyr Asn Ala Ser Leu Asp Ile Arg Val
        35                  40                  45

Pro Leu Thr Ile Gly Thr Ile Tyr Ala Val Ser Val His Leu Thr Asn
    50                  55                  60

Ser Ser Glu Arg Ile Lys Lys Arg Gln Pro Ile Ala Phe Ala Lys Thr
65                  70                  75                  80

Ala Leu Phe Lys Trp Leu Cys Val Leu His Asn Ala Gly Leu Cys Leu
                85                  90                  95

Tyr Ser Ala Trp Thr Phe Val Gly Ile Leu Asn Ala Val Lys His Ala
            100                 105                 110

Tyr Gln Ile Thr Gly Asp Ser Ser Ala Pro Phe Ser Phe Asn Thr Leu
        115                 120                 125

Trp Gly Ser Phe Cys Ser Arg Asp Ser Leu Trp Val Thr Gly Leu Asn
    130                 135                 140

Tyr Tyr Gly Tyr Trp Phe Tyr Leu Ser Lys Phe Tyr Glu Val Val Asp
145                 150                 155                 160

Thr Met Ile Ile Leu Ala Lys Gly Lys Pro Ser Ser Met Leu Gln Thr
                165                 170                 175

Tyr His His Thr Gly Ala Met Phe Ser Met Trp Ala Gly Ile Arg Phe

180                 185                 190

Ala Ser Pro Pro Ile Trp Ile Phe Val Val Phe Asn Ser Leu Ile His
        195                 200                 205

Thr Ile Met Tyr Phe Tyr Tyr Thr Leu Thr Thr Leu Lys Ile Lys Val
    210                 215                 220

Pro Lys Ile Leu Lys Ala Ser Leu Thr Thr Ala Gln Ile Thr Gln Ile
225                 230                 235                 240

Val Gly Gly Gly Ile Leu Ala Ala Ser His Ala Phe Ile Tyr Tyr Lys
                245                 250                 255

Asp His Gln Thr Glu Thr Val Cys Ser Cys Leu Thr Thr Gln Gly Gln
            260                 265                 270

Phe Phe Ala Leu Ala Val Asn Val Ile Tyr Leu Ser Pro Leu Ala Tyr
        275                 280                 285

Leu Phe Ile Ala Phe Trp Ile Arg Ser Tyr Leu Lys Ala Lys Ser Asn
    290                 295                 300

<210>  14
<211>  1604
<212>  DNA
<213>  Yarrowia lipolytica

<400>  14
atgagtgagt atttcccaca aaacaaatgc ccccagcagc gaacacgccc cgacaccgat      60

acacgcagca gcgcggaggt gacacaaatg cctcggattt gcacctgtga tcatgaacac     120

gttacgtcga catgggacac acgtcctgtg taatcgacca ttcagtcaac gaaacagatg     180

ttttaggagg acacctcgag ccagaagatg atcgaccggg gatactaccg gtagttgtgt     240

caccaacact cagctcaagc gctgttatcg tcgtcgcggg gtgatttgac ccctgatcta     300

cgcgtcgcgt acaacaaaac gtggcagatt tggggtttaa tcgccggaca acagaaacga     360

atacgcagag acagtaacgg atggataata caaatcctac ctcgcccctt gatctatcta     420

cgtctctcac atgtcgcatg atccatatcg ttacttccga tctcatgttt gacaaaatcc     480

atacaaggcg aagtgaggca aaccccgaac atatacgtga caacaagcct cgtgtcacta     540

ctatgtggtg gccgccacaa cactgacgtg acgtcctttt tgggacacga cctctgtcac     600

acctttacta tccgctctat actaactcag gcgccgtccc tattgaattc aacgtcccct     660

ccgtggaccg accctttggt atctacctct gggccatctt tgaccaggcc tgggagaagc     720

ttttcggctg gcccgcgtcc tctttcattt tcgtgcgaaa tgaccccaac atcccctttt     780

cctctacccc tcccgtgatc attgccatca ttgtgtacta cattgtcatc tttggcggcc 840

gagaggtgat gcgaaacctg tctcccatcc gactcaactg gctcttccag atccacaaca 900

tcttcctcac ccttctgtcc ggtatgctcc tcctcctcct cgttgagcag ctcttcccca 960

tcattgtccg acagggtatc ctctacgcca tctgcgacta cggatcttgg actcagccca 1020

ttgtcttctg ctactacctc aactacctga ccaagtactt tgagctgatc gacaccgttt 1080

tccttgtgct gcgaaagaag aagctgactt tcctccacac ctaccaccat ggtgccactg 1140

ctcttctgtg ctacacccag ctcattggta agacctcggt ctcttgggtc cccatcaccc 1200

ttaacctgtt tgtccacgtt gtcatgtact tctactactt cctggctgcg cgaggtatcc 1260

gagtgtggtg gaaggagtgg gtcacccggc tccagatcat ccagttcgtt atcgatcttg 1320

gatttgtcta ctttgcctct tacacctact tcacctctac ctactggccc tggatgccca 1380

acatgggctc ttgtgccggc gaggagtttg ctgctattta cggctgtggt ctgctgacct 1440

cttacctctt cctcttcatc gccttctaca tcaactctta ccgaaagccc tcttccaagg 1500

gaccttccaa gcctgttgtt gctgtcgatg gccctgttgg cggcgtcaac gcccagactg 1560

gtgcttctcg aggccagacc actacccgat ctcgacgagc ataa 1604

<210> 15
<211> 915
<212> DNA
<213> Yarrowia lipolytica

<400> 15
atgctctcgt caatctcgcc cgacctatac tcgtccttct cgttcaaaaa ctcgctcgcc 60

gaggccatgc cctccgtgcc acacgaactc atcaactcaa aaacactctc atggatgtac 120

aatgcctctc tggacattcg ggttcctctg actatcggaa ccatctacgc cgtctccgtg 180

cacctgacca actcatctga acgaatcaag aaacgccagc ccattgcctt tgccaagacc 240

gcactcttca agtggctctg tgtcctccac aatgcaggtc tgtgtctcta ctcagcatgg 300

acctttgtcg gtatcctcaa cgccgtcaaa cacgcctacc aaatcacagg agacagctcc 360

gcccccttct ccttcaacac cctctgggga tcgttttgtt cacgtgactc cctctgggtc 420

accggcctca actactacgg atactggttc tatctgtcca aattctacga agtggtggac 480

accatgatca tcctcgcaaa gggaaaaccg tcctcaatgc tccagacata ccaccacacc 540

ggcgccatgt tctccatgtg ggccggcatc cgattcgcct ctccccccat ctggatcttt 600

gtggttttca actccctcat ccacacaatc atgtactttt actacaccct caccaccctc 660

aagatcaagg ttcccaagat cctcaaggca tctctgacca ccgcccagat cacccagatt 720

gtcggaggtg gcatcctggc tgcctcccac gcctttattt attacaagga ccaccagact 780

gagaccgtct gttcttgtct cactacccag ggtcagtttt tcgctctcgc cgtcaatgtc 840

atctatctga gtcctctggc ctatctcttt attgccttct ggattcgatc ttacttgaag 900

gccaagtcca actag 915

<210> 16
<211> 4354
<212> DNA
<213> Yarrowia lipolytica

<400> 16
ggatccgtat gccacctatc taaccactcc ggtcgttcag cattgatggg gtggtccaaa 60

tatgagattt ttttataagt aacagcattc taacagaaac taaaggactg tagcaaggac 120

taataggctg tggcgaacat ggcttcagtg taatgtgccc ttgatggacc tcttcaactg 180

tgagcaattg gtgaggttcc actgccctca accaaataga cagtctccct atagtgtact 240

tgtactgtct tgcagagtaa tcaaatgtga tcagaaaacc atgtaccgaa taattgcatc 300

atctacttgt agtatcttca ttttgaacca tgttccattc aaatcacatc tgaatcgact 360

ggtagcatgt gggtgaaagt ctggatttac ttacaacttt agttgggaat attgttgatc 420

caaatgatac cagaaaatac accaaaatat ggccgcaatg gggcatgaat agcactcata 480

ttgcgctaga ccagcttccc aattcactat attgcatata caagtagtag tgctcgttca 540

aaacgcgtcc cgaaaacctg tccaccagtt gcactgttgt ctcctttctg cttggcagat 600

atttacaagc ctcggttgac gatcacttat ttatccgaaa tgccgcagtg agagagtgca 660

ccttagatcg cgtgttttca ggtgtagttt cagcagctga ctacacatac atgtcaccca 720

ctcatcaaca caagcccaac tatcacccga tacaagacta cagtattgtt ttactgtagc 780

agccctgttg ggttccggct gtccttttct caatctcaac cgtctcccaa cgttgggagc 840

agcataaggt ttggaaacaa acgacgcagg aaaaaaaagt tggtttatca gttgggggag 900

ggcgttcaag tagggagagg atatatggta aatgggcgcg tggaatgaag tagagaatgg 960

tttgcttgag aaaacgaccc ttaaattaat catggccatt ttttccccac tcaaaatccc 1020

cccacaacac cctgcaacat acacttccca gcactcaaca aagttgatcc tccctcaacg 1080

tatgccactg catgcatgat acgggggtcg cgattctaaa taaaacgact gtcgtgcctc 1140

aattgaaacg tcacgagaaa tctgtgaact acacacacaa acagttcatt acgagtgagt 1200

cttgaaatat gggatatgag gaggggtttg aagaggttgc aatcgataac tcacgacacg 1260

gacgaaaaag aataaggacc aacacgatct ccagacaacc acagatcagc agtcgaaccc 1320

ccctcaacag cagacaaatg atgttgtgga attgcagtag atgatttctt ctgcgacgct 1380

agtattggct gtcggcgaca ctattctctg acagtgccca atggtctttt tattgtgcac 1440

caaccgctga tttgtggctc aggttttgtg acggcgagag tcattctcgt gatgcatggg 1500

atgattggtc tctttgaagc cgacagatcg acatatttcc acacacagca acgacaatgt 1560

tatcttatcc attgccattc taacccagtg ggtagggata acagggtaat tatcgcttcg 1620

gataactcct gctatacgaa gttatacgaa ttcgcgccca gagagccatt gacgttcttt 1680

ctaatttgga ccgatagccg tatagtccag tctatctata agttcaacta actcgtaact 1740

attaccataa catatacttc actgccccag ataaggttcc gataaaaagt tctgcagact 1800

aaatttattt cagtctcctc ttcaccacca aaatgccctc ctacgaagct cgagctaacg 1860

tccacaagtc cgcctttgcc gctcgagtgc tcaagctcgt ggcagccaag aaaaccaacc 1920

tgtgtgcttc tctggatgtt accaccacca aggagctcat tgagcttgcc gataaggtcg 1980

gaccttatgt gtgcatgatc aagacccata tcgacatcat tgacgacttc acctacgccg 2040

gcactgtgct ccccctcaag gaacttgctc ttaagcacgg tttcttcctg ttcgaggaca 2100

gaaagttcgc agatattggc aacactgtca agcaccagta ccggtgtcac cgaatcgccg 2160

agtggtccga tatcaccaac gcccacggtg tacccggaac cggaatcatt gctggcctgc 2220

gagctggtgc cgaggaaact gtctctgaac agaagaagga ggacgtctct gactacgaga 2280

actcccagta caaggagttc ctagtcccct ctcccaacga gaagctggcc agaggtctgc 2340

tcatgctggc cgagctgtct tgcaagggct ctctggccac tggcgagtac tccaagcaga 2400

ccattgagct tgcccgatcc gaccccgagt ttgtggttgg cttcattgcc cagaaccgac 2460

ctaagggcga ctctgaggac tggcttattc tgacccccgg ggtgggtctt gacgacaagg 2520

gagacgctct cggacagcag taccgaactg ttgaggatgt catgtctacc ggaacggata 2580

tcataattgt cggccgaggt ctgtacggcc agaaccgaga tcctattgag gaggccaagc 2640

gataccagaa ggctggctgg gaggcttacc agaagattaa ctgttagagg ttagactatg 2700

gatatgtaat ttaactgtgt atatagagag cgtgcaagta tggagcgctg ttcagcttgt 2760

atgatggtca gacgacctgt ctgatcgagt atgtatggta ctgcacaacc tgtgtatccg 2820

catggtctgt ccaatggggc atgttgttgt gtttctcgga attcagaata acttcgtata 2880

atgtatgcta tacgaagtta tgtagggata acagggtaat cgtagatcgt gatgagcgat 2940

gtgagatgaa atgaggagga ttttatatgt gttttaattg attgatttat gataggatgg 3000

gccgtaatat gtacatacag tagttcaaca gactggtccg tacgtcaagc catgggacga 3060

caagcctgtt atacagtatg tactgtagta tgtacagtac aatttgtgag agttcgtgat 3120

tcaaaaagaa gatgatgcct ctcacaataa aagagacaaa aataaatggt ccctagcagg 3180

atcgaactgc tgatcttcgc gttgcaaaga ggctaatttc ttattagcac gacgccttaa 3240

ccaactgggc caagggacct atttttatac aaaataaact gtggccccta ctatccaaat 3300

tcagtcaatt tcaaacctga atcatcagaa taaaaacaaa acaaaaaacg aaatcgcttc 3360

aaaacgacac tgatgtcaag gtggcagttt tataatactg tgatgatact aaattttaag 3420

tcaagctcga gtttccacta ctcataacgt atggtcatgc ggcagtgtct tgtgacattg 3480

aactccggat taatagagct ttgaaagccg aaatgtgagg aattttgggt agtgggtata 3540

aattgatctc gttattaaga ggaatactga cgttcccctt catctcttgt tgtactgtca 3600

cttgtcaaca cacctccagc gatacatgcg ccatgttatc acttatcgtt atgttagggt 3660

tctgcatttg catagtgcaa gtgctaaaaa aatatttgcc ctgtagagct ctacacgaca 3720

aaaccacaca ctcataatcg atacaaaatg agctacacga tctcagcagc agctctatat 3780

tctgcaacga tatacctgtt gtttctgcgc gacagaagtc gacaaagtct cgagcgaagt 3840

gaaattctgg ctgcaaggga ggctacaaac tacagcatca gcaatacgac tattttctcc 3900

ggagtgacag ctaaaagcta cggtttcaat gcgcattaca gaggggttcc cgcaacgtac 3960

tggtcgtggc tgctgttggt ccagggtctg tttgtgctgg gcatgttcta catggactgg 4020

ccaacagttg gatatggagc acttctcgtc tggagctttt atgcttatgc cacggagcag 4080

tgtgacaagc aggggcgcat gagtggcctc aagatccaga gcatgtttgg tccggtcaaa 4140

acgtttgctg gccgggacaa gttccactgg tacgctgttg tcaacacggg tacctctctt 4200

gatctgttgc cagagtcatc tgatgatgag ctttctgagg aggaggagga ggacgtcgag 4260

atggacggcg acgtcaacat ggaagaaact taccggactc tgagacaaaa tatgcatagt 4320

ttcgaggaga ttatggggga gggagaagaa gctt 4354

<210> 17
<211> 1642
<212> DNA
<213> Artificial Sequence

<220>
<223> TAL_KSr

<400> 17
aacctgaccc cggaccaagt ggtggctatc gccagcaaca atggcggcaa gcaagcgctc 60

gaaacggtgc agcggctgtt gccggtgctg tgccaggacc atggcctgac cccggaccaa 120

gtggtggcta tcgccagcaa cggtggcggc aagcaagcgc tcgaaacggt gcagcggctg 180

ttgccggtgc tgtgccagga ccatggcctg accccggacc aagtggtggc tatcgccagc 240

aacggtggcg gcaagcaagc gctcgaaacg gtgcagcggc tgttgccggt gctgtgccag 300

gaccatggcc tgactccgga ccaagtggtg gctatcgcca gccacgatgg cggcaagcaa 360

gcgctcgaaa cggtgcagcg gctgttgccg gtgctgtgcc aggaccatgg cctgactccg 420

gaccaagtgg tggctatcgc cagccacgat ggcggcaagc aagcgctcga aacggtgcag 480

cggctgttgc cggtgctgtg ccaggaccat ggcctgaccc cggaccaagt ggtggctatc 540

gccagcaaca atggcggcaa gcaagcgctc gaaacggtgc agcggctgtt gccggtgctg 600

tgccaggacc atggcctgac cccggaccaa gtggtggcta tcgccagcaa caatggcggc 660

aagcaagcgc tcgaaacggt gcagcggctg ttgccggtgc tgtgccagga ccatggcctg 720

accccggacc aagtggtggc tatcgccagc aacggtggcg gcaagcaagc gctcgaaacg 780

gtgcagcggc tgttgccggt gctgtgccag gaccatggcc tgaccccgga ccaagtggtg 840

gctatcgcca gcaacggtgg cggcaagcaa gcgctcgaaa cggtgcagcg gctgttgccg 900

gtgctgtgcc aggaccatgg cctgactccg gaccaagtgg tggctatcgc cagccacgat 960

ggcggcaagc aagcgctcga aacggtgcag cggctgttgc cggtgctgtg ccaggaccat 1020

ggcctgaccc cggaccaagt ggtggctatc gccagccacg atggcggcaa gcaagcgctc 1080

gaaacggtgc agcggctgtt gccggtgctg tgccaggacc atggcctgac cccggaccaa 1140

gtggtggcta tcgccagcaa caatggcggc aagcaagcgc tcgaaacggt gcagcggctg 1200

ttgccggtgc tgtgccagga ccatggcctg accccggacc aagtggtggc tatcgccagc 1260

aacaatggcg gcaagcaagc gctcgaaacg gtgcagcggc tgttgccggt gctgtgccag 1320

gaccatggcc tgaccccgga ccaagtggtg gctatcgcca gcaacggtgg cggcaagcaa 1380

gcgctcgaaa cggtgcagcg gctgttgccg gtgctgtgcc aggaccatgg cctgaccccg 1440

gaccaagtgg tggctatcgc cagcaacatt ggcggcaagc aagcgctcga aacggtgcag 1500

cggctgttgc cggtgctgtg ccaggaccat ggcctgaccc cggaccaagt ggtggctatc 1560

gccagcaacg gtggcggcaa gcaagcgctc gaaagcattg tggcccagct gagccggcct 1620

gatccggcgt tggccgcgtt ga 1642

<210> 18
<211> 1642
<212> DNA
<213> Artificial Sequence

<220>
<223> TAL_KS1

<400> 18
aacctgaccc cggaccaagt ggtggctatc gccagcaaca atggcggcaa gcaagcgctc 60

gaaacggtgc agcggctgtt gccggtgctg tgccaggacc atggcctgac cccggaccaa 120

gtggtggcta tcgccagcaa cattggcggc aagcaagcgc tcgaaacggt gcagcggctg 180

ttgccggtgc tgtgccagga ccatggcctg accccggacc aagtggtggc tatcgccagc 240

aacattggcg gcaagcaagc gctcgaaacg gtgcagcggc tgttgccggt gctgtgccag 300

gaccatggcc tgactccgga ccaagtggtg gctatcgcca gccacgatgg cggcaagcaa 360

gcgctcgaaa cggtgcagcg gctgttgccg gtgctgtgcc aggaccatgg cctgaccccg 420

gaccaagtgg tggctatcgc cagcaacatt ggcggcaagc aagcgctcga aacggtgcag 480

cggctgttgc cggtgctgtg ccaggaccat ggcctgaccc cggaccaagt ggtggctatc 540

gccagcaacg gtggcggcaa gcaagcgctc gaaacggtgc agcggctgtt gccggtgctg 600

tgccaggacc atggcctgac cccggaccaa gtggtggcta tcgccagcaa caatggcggc 660

aagcaagcgc tcgaaacggt gcagcggctg ttgccggtgc tgtgccagga ccatggcctg 720

actccggacc aagtggtggc tatcgccagc cacgatggcg gcaagcaagc gctcgaaacg 780

gtgcagcggc tgttgccggt gctgtgccag gaccatggcc tgactccgga ccaagtggtg 840

gctatcgcca gccacgatgg cggcaagcaa gcgctcgaaa cggtgcagcg gctgttgccg 900

gtgctgtgcc aggaccatgg cctgaccccg gaccaagtgg tggctatcgc cagcaacggt 960

ggcggcaagc aagcgctcga aacggtgcag cggctgttgc cggtgctgtg ccaggaccat 1020

ggcctgaccc cggaccaagt ggtggctatc gccagccacg atggcggcaa gcaagcgctc 1080

gaaacggtgc agcggctgtt gccggtgctg tgccaggacc atggcctgac cccggaccaa 1140

gtggtggcta tcgccagcaa caatggcggc aagcaagcgc tcgaaacggt gcagcggctg 1200

ttgccggtgc tgtgccagga ccatggcctg actccggacc aagtggtggc tatcgccagc 1260

cacgatggcg gcaagcaagc gctcgaaacg gtgcagcggc tgttgccggt gctgtgccag 1320

gaccatggcc tgaccccgga ccaagtggtg gctatcgcca gcaacattgg cggcaagcaa 1380

gcgctcgaaa cggtgcagcg gctgttgccg gtgctgtgcc aggaccatgg cctgaccccg 1440

gaccaagtgg tggctatcgc cagcaacaat ggcggcaagc aagcgctcga aacggtgcag 1500

cggctgttgc cggtgctgtg ccaggaccat ggcctgaccc cggaccaagt ggtggctatc 1560

gccagcaaca atggcggcaa gcaagcgctc gaaagcattg tggcccagct gagccggcct 1620

gatccggcgt tggccgcgtt ga 1642

<210> 19
<211> 2022
<212> DNA
<213> Artificial Sequence

<220>
<223> Matrix I1220F

<400> 19
aacggtggct tccagtacat tcccgacctc aagggagttg ttggaaagat ccgacgagac 60

attgtggaga cctccgagat ccgacgggct gtggctcagg agactgccat tgaacagaag 120

gtggtcaacg gcccccacgc cgatcttcct taccagaagg tcgaggtcaa gccccgagcc 180

aacctcaagt ttgacttccc caccctcaaa tcctacgccg aggtcaagga gctgtctcct 240

gctggtgatg ctctggaggg tcttctggat ctctcttccg tcattgttgt cactggtttc 300

gccgaggtcg gtccttgggg taacgcccga acccgatggg acatggaggc caacggtgtc 360

ttctcccttg agggtgccat tgagatggcc tggatcatgg gtctgatcaa gcaccacaat 420

ggtcccctgc ccggcatgcc tcagtactct ggctggatcg ataccaagac caagcagccc 480

gtcgatgacc gagatatcaa gaccaagtac gaggactacc tgcttgagca cgccggtatc 540

cgactcattg agcctgagct gttccacggc tacaacccca agaagaagac cttcctccag 600

gaggttattg tggagcacga tctcgagccc tttgaggcct ccaaggagtc tgctgagcaa 660

tttgctctcg agcagggcgc gaacgttgag atcttcgccg tccccgagtc cgaccagtgg 720

actgtgcgac ttctcaaggg cgccaagctc ctcattccca aggccctcaa gtttgaccga 780

cttgtggccg gccagattcc cactggatgg gatgcccgac gatacggtat tcccgaggac 840

atttgtgacc aggttgaccc catcactctg tacgctcttg tctccactgt tgaggctctg 900

ttggcctccg gtattaccga cccctacgag ttctacaagt acgtccacgt gtccgaggtc 960

ggtaactgct ctggatccgg catgggtggt ttcaccgccc tcagaggtat gtttaaggac 1020

cggttcatgg acaagcctgt tcagaacgat attctccagg agtccttcat caacaccatg 1080

tctgcctggg tcaacatgtt gctgctctcc tcttccggtc ccatcaagac ccccgttgga 1140

gcttgtgcca ctgctgtcga gtctgtggac attggttgcg aaaccattct gtccggcaag 1200

gccagaatct gtctggtcgg tggttacgat gatttccagg aggagtcttc tcaggagttt 1260

gcaaacatga acgcaacatc caacgctgag accgagatca ctcacggccg aactccggcc 1320

gagatgtctc gacccatcac ttccacacga gccggtttca tggaggctca gggtgctgga 1380

acccaggtgc tgatggccgc cgacctcgcc atcgccatgg gtgtgcccat ctactgtatc 1440

gttggttacg tcaacactgc caccgacaag attggccgat ctgtgcctgc tcccggtaag 1500

ggtatcctga ccactgctcg agagcaccag actctcaaac acgccaaccc tctcctcaac 1560

atcaagtacc gaaagcgaca gctcgattct cgactccgag acattaagcg atgggctgag 1620

ggcgaaatgg aggctattga cattgagctt gacgacgtgt ctgacgccga caaggagtcc 1680

ttcatccagg agcgatctgc ccacatccag tctcagtccg atcgaatgat ccgagaggct 1740

aagaactctt ggggtaacgc ctttttcaag caggacgccc gaatctcccc catccgagga 1800

gcgctggcaa cctacggtct caccattgat gacatctccg tcgcttcttt ccatggtaca 1860

tccaccaagg ccaacgagaa gaacgagacc accaccgtca acgccatgct ggagcatctc 1920

ggcagaaccc ggggtaaccc tgtctacggt atcttccaga agtaccttac tggtcacccc 1980

aagggagctg ctggtgcctg gatgctcaac ggagccatcc aa 2022

<210> 20
<211> 42
<212> DNA
<213> Artificial Sequence

<220>
<223> Primer alphaFAS F

<400> 20
acccgaagga tcccacaatg caccccgaag tcgaacaaga ac 42

<210>  21
<211>  40
<212>  DNA
<213>  Artificial Sequence

<220>
<223>  Primer alphaFAS R

<400>  21
accacagaca ccctaggcta ctcggcaaca gcaacagcca                                40


<210>  22
<211>  35
<212>  DNA
<213>  Artificial Sequence

<220>
<223>  Primer FAS_S1305T  F

<400>  22
atgatttcca ggaggagact tctcaggagt ttgca                                     35


<210>  23
<211>  35
<212>  DNA
<213>  Artificial Sequence

<220>
<223>  Primer FAS_S1305T  R

<400>  23
tgcaaactcc tgagaagtct cctcctggaa atcat                                     35


<210>  24
<211>  32
<212>  DNA
<213>  Artificial Sequence

<220>
<223>  Primer FAS_M1217F_F

<400>  24
ctgttccggt tccggtttcg gtggtatcac cg                                        32


<210>  25
<211>  32
<212>  DNA
<213>  Artificial Sequence

<220>
<223>  Primer FAS_M1217F_R

<400>  25
cggtgatacc accgaaaccg gaaccggaac ag                                        32


<210>  26

<211>  36
<212>  DNA
<213>  Artificial Sequence

<220>
<223>  Primer FAS_M1217Y_F

<400>  26
aactgttccg gttccggtta cggtggtatc accgcc                                36


<210>  27
<211>  36
<212>  DNA
<213>  Artificial Sequence

<220>
<223>  Primer FAS_M1217Y_R

<400>  27
ggcggtgata ccaccgtaac cggaaccgga acagtt                                36


<210>  28
<211>  27
<212>  DNA
<213>  Artificial Sequence

<220>
<223>  Primer FAS_I1220M F

<400>  28
tccggtatgg gtggtatgac cgccctg                                          27


<210>  29
<211>  27
<212>  DNA
<213>  Artificial Sequence

<220>
<223>  Primer FAS_I1220M R

<400>  29
cagggcggtc ataccaccca taccgga                                          27


<210>  30
<211>  27
<212>  DNA
<213>  Artificial Sequence

<220>
<223>  Primer FAS_I1220F F

<400>  30
ccggtatggg tggtttcacc gccctgc                                          27


<210>  31
<211>  27
<212>  DNA
<213>  Artificial Sequence

<220>
<223>  Primer FAS_I1220F R

<400>  31
gcagggcggt gaaaccaccc ataccgg                                          27


<210>  32
<211>  35
<212>  DNA
<213>  Artificial Sequence

<220>
<223>  Primer FAS_I1220W F

<400>  32
tccggttccg gtatgggtgg ttggaccgcc ctgcg                                 35


<210>  33
<211>  35
<212>  DNA
<213>  Artificial Sequence

<220>
<223>  Primer FAS_I1220W R

<400>  33
cgcagggcgg tccaaccacc cataccggaa ccgga                                 35


<210>  34
<211>  21
<212>  DNA
<213>  Artificial Sequence

<220>
<223>  Primer KS-T7_F

<400>  34
tggatgggat gcccgacgat a                                                21


<210>  35
<211>  22
<212>  DNA
<213>  Artificial Sequence

<220>
<223>  Primer KS-T7_R

<400>  35
gagtgatctc ggtctcagcg tt                                               22


<210>  36
<211>  20
<212>  DNA
<213>  Artificial Sequence

<220>
<223>  Primer HRscreening_R

<400>  36
ggtccttaaa catacctctg                                                   20


<210>  37
<211>  20
<212>  DNA
<213>  Artificial Sequence

<220>
<223>  Primer WTscreening_R

<400>  37
ggtccttgaa catgcctcgc                                                   20


<210>  38
<211>  25
<212>  DNA
<213>  Artificial Sequence

<220>
<223>  Primer KS_P1

<400>  38
gtcgttgaga acaaggctgt ttctg                                             25


<210>  39
<211>  25
<212>  DNA
<213>  Artificial Sequence

<220>
<223>  Primer KS_T2

<400>  39
   gaatttggat agtaggggcc acagt                                            25


<210>  40
<211>  23
<212>  DNA
<213>  Artificial Sequence

<220>
<223>  Primer Matrix KS-TALEN 1F

<400>  40
aacggtggct tccagtacat tcc                                               23


<210>  41
<211>  24
<212>  DNA
<213>  Artificial Sequence

<220>
<223>  Primer Matrix KS-TALEN 4R

<400>  41
ttggatggct ccgttgagca tcca                                              24

<210>  42
<211>  51
<212>  DNA
<213>  Artificial Sequence

<220>
<223>  Primer Matrix KSI1220L  3F

<400>  42
atgggtggtc tgaccgccct cagaggtatg tttaaggacc ggttcatgga c                51


<210>  43
<211>  50
<212>  DNA
<213>  Artificial Sequence

<220>
<223>  Primer Matrix KSI1220L 2R

<400>  43
agggcggtca gaccacccat gccggatcca gagcagttac cgacctcgga                50


<210>  44
<211>  51
<212>  DNA
<213>  Artificial Sequence

<220>
<223>  Primer Matrix KSI1220V 3F

<400>  44
atgggtggtg tcaccgccct cagaggtatg tttaaggacc ggttcatgga c                51


<210>  45
<211>  50
<212>  DNA
<213>  Artificial Sequence

<220>
<223>  Primer Matrix KSI1220V 2R

<400>  45
agggcggtga caccacccat gccggatcca gagcagttac cgacctcgga                50


<210>  46
<211>  51
<212>  DNA
<213>  Artificial Sequence

<220>
<223>  Primer Matrix KSI1220S 3F

<400>  46
atgggtggtt ccaccgccct cagaggtatg tttaaggacc ggttcatgga c                51


<210>  47

<211>  50
<212>  DNA
<213>  Artificial Sequence

<220>
<223>  Primer Matrix KSI1220S 2R

<400>  47
agggcggtgg aaccacccat gccggatcca gagcagttac cgacctcgga          50


<210>  48
<211>  51
<212>  DNA
<213>  Artificial Sequence

<220>
<223>  Primer Matrix KSI1220P 3F

<400>  48
atgggtggtc ccaccgccct cagaggtatg tttaaggacc ggttcatgga c          51


<210>  49
<211>  50
<212>  DNA
<213>  Artificial Sequence

<220>
<223>  Primer Matrix KSI1220P 2R

<400>  49
agggcggtgg gaccacccat gccggatcca gagcagttac cgacctcgga          50


<210>  50
<211>  51
<212>  DNA
<213>  Artificial Sequence

<220>
<223>  Primer Matrix KSI1220T 3F

<400>  50
atgggtggta ccaccgccct cagaggtatg tttaaggacc ggttcatgga c          51


<210>  51
<211>  50
<212>  DNA
<213>  Artificial Sequence

<220>
<223>  Primer Matrix KSI1220T 2R

<400>  51
agggcggtgg taccacccat gccggatcca gagcagttac cgacctcgga          50


<210>  52
<211>  51
<212>  DNA
<213>  Artificial Sequence

<220>
<223>  Primer Matrix KSI1220A 3F

<400>  52
atgggtggtg ccaccgccct cagaggtatg tttaaggacc ggttcatgga c          51


<210>  53
<211>  50
<212>  DNA
<213>  Artificial Sequence

<220>
<223>  Primer Matrix KSI1220A 2R

<400>  53
agggcggtgg caccacccat gccggatcca gagcagttac cgacctcgga          50


<210>  54
<211>  51
<212>  DNA
<213>  Artificial Sequence

<220>
<223>  Primer Matrix KSI1220Y 3F

<400>  54
atgggtggtt acaccgccct cagaggtatg tttaaggacc ggttcatgga c          51


<210>  55
<211>  50
<212>  DNA
<213>  Artificial Sequence

<220>
<223>  Primer Matrix KSI1220Y 2R

<400>  55
agggcggtgt aaccacccat gccggatcca gagcagttac cgacctcgga          50


<210>  56
<211>  51
<212>  DNA
<213>  Artificial Sequence

<220>
<223>  Primer Matrix KSI1220H 3F

<400>  56
atgggtggtc acaccgccct cagaggtatg tttaaggacc ggttcatgga c          51


<210>  57
<211>  50
<212>  DNA
<213>  Artificial Sequence

<220>
<223>  Primer Matrix KSI1220H 2R

<400>  57
agggcggtgt gaccacccat gccggatcca gagcagttac cgacctcgga                50


<210>  58
<211>  51
<212>  DNA
<213>  Artificial Sequence

<220>
<223>  Primer Matrix KSI1220Q 3F

<400>  58
atgggtggtc agaccgccct cagaggtatg tttaaggacc ggttcatgga c              51


<210>  59
<211>  50
<212>  DNA
<213>  Artificial Sequence

<220>
<223>  Primer Matrix KSI1220Q 2R

<400>  59
agggcggtct gaccacccat gccggatcca gagcagttac cgacctcgga                50


<210>  60
<211>  51
<212>  DNA
<213>  Artificial Sequence

<220>
<223>  Primer Matrix KSI1220N 3F

<400>  60
atgggtggta acaccgccct cagaggtatg tttaaggacc ggttcatgga c              51


<210>  61
<211>  50
<212>  DNA
<213>  Artificial Sequence

<220>
<223>  Primer Matrix KSI1220N 2R

<400>  61
agggcggtgt taccacccat gccggatcca gagcagttac cgacctcgga                50


<210>  62
<211>  51
<212>  DNA
<213>  Artificial Sequence

<220>
<223>  Primer Matrix KSI1220K 3F

<400>  62
atgggtggta agaccgccct cagaggtatg tttaaggacc ggttcatgga c              51

<210>  63
<211>  50
<212>  DNA
<213>  Artificial Sequence

<220>
<223>  Primer Matrix KSI1220K 2R

<400>  63
agggcggtct taccacccat gccggatcca gagcagttac cgacctcgga          50


<210>  64
<211>  51
<212>  DNA
<213>  Artificial Sequence

<220>
<223>  Primer Matrix KSI1220D 3F

<400>  64
atgggtggtg acaccgccct cagaggtatg tttaaggacc ggttcatgga c          51


<210>  65
<211>  50
<212>  DNA
<213>  Artificial Sequence

<220>
<223>  Primer Matrix KSI1220D 2R

<400>  65
agggcggtgt caccacccat gccggatcca gagcagttac cgacctcgga          50


<210>  66
<211>  51
<212>  DNA
<213>  Artificial Sequence

<220>
<223>  Primer Matrix KSI1220E 3F

<400>  66
atgggtggtg agaccgccct cagaggtatg tttaaggacc ggttcatgga c          51


<210>  67
<211>  50
<212>  DNA
<213>  Artificial Sequence

<220>
<223>  Primer Matrix KSI1220E 2R

<400>  67
agggcggtct caccacccat gccggatcca gagcagttac cgacctcgga          50


<210>  68

<211>  51
<212>  DNA
<213>  Artificial Sequence

<220>
<223>  Primer Matrix KSI1220C 3F

<400>  68
atgggtggtt gcaccgccct cagaggtatg tttaaggacc ggttcatgga c                51


<210>  69
<211>  50
<212>  DNA
<213>  Artificial Sequence

<220>
<223>  Primer Matrix KSI1220C 2R

<400>  69
agggcggtgc aaccacccat gccggatcca gagcagttac cgacctcgga                50


<210>  70
<211>  51
<212>  DNA
<213>  Artificial Sequence

<220>
<223>  Primer Matrix KSI1220M 3F

<400>  70
atgggtggta tgaccgccct cagaggtatg tttaaggacc ggttcatgga c                51


<210>  71
<211>  50
<212>  DNA
<213>  Artificial Sequence

<220>
<223>  Primer Matrix KSI1220M 2R

<400>  71
agggcggtca taccacccat gccggatcca gagcagttac cgacctcgga                50


<210>  72
<211>  51
<212>  DNA
<213>  Artificial Sequence

<220>
<223>  Primer Matrix KSI1220W 3F

<400>  72
atgggtggtt ggaccgccct cagaggtatg tttaaggacc ggttcatgga c                51


<210>  73
<211>  50
<212>  DNA
<213>  Artificial Sequence

<220>
<223>  Primer Matrix KSI1220W 2R

<400>  73
agggcggtcc aaccacccat gccggatcca gagcagttac cgacctcgga          50


<210>  74
<211>  51
<212>  DNA
<213>  Artificial Sequence

<220>
<223>  Primer matrix KSI1220F 3F

<400>  74
atgggtggtt tcaccgccct cagaggtatg tttaaggacc ggttcatgga c        51


<210>  75
<211>  50
<212>  DNA
<213>  Artificial Sequence

<220>
<223>  Primer matrix KSI1220F 2R

<400>  75
agggcggtga aaccacccat gccggatcca gagcagttac cgacctcgga          50


<210>  76
<211>  51
<212>  DNA
<213>  Artificial Sequence

<220>
<223>  Primer Matrix KSI1220R 3F

<400>  76
atgggtggtc gaaccgccct cagaggtatg tttaaggacc ggttcatgga c        51


<210>  77
<211>  50
<212>  DNA
<213>  Artificial Sequence

<220>
<223>  Primer Matrix KSI1220R 2R

<400>  77
agggcggttc gaccacccat gccggatcca gagcagttac cgacctcgga          50


<210>  78
<211>  51
<212>  DNA
<213>  Artificial Sequence

<220>
<223>  Primer Matrix KSI1220G 3F

<400>  78
atgggtggtg gcaccgccct cagaggtatg tttaaggacc ggttcatgga c                51

<210>  79
<211>  50
<212>  DNA
<213>  Artificial Sequence

<220>
<223>  Primer Matrix KSI1220G 2R

<400>  79
agggcggtgc caccacccat gccggatcca gagcagttac cgacctcgga                  50

<210>  80
<211>  40
<212>  DNA
<213>  Artificial Sequence

<220>
<223>  Primer FAS_S1305V_ F

<400>  80
cgatgatttc caggaggagg tttctcagga gtttgcaaac                             40

<210>  81
<211>  40
<212>  DNA
<213>  Artificial Sequence

<220>
<223>  Primer FAS_ S1305V_ R

<400>  81
gtttgcaaac tcctgagaaa cctcctcctg gaaatcatcg                             40